# EUROPEAN PATENT APPLICATION

(11) **EP 3 330 280 A1**
(43) Date of publication of application: **06.06.2018**
(21) Application number: 16833007.4
(22) Date of filing: 01.08.2016
(51) Int. Cl.: C07K 7/54, A61K 8/64, A61K 9/06, A61K 9/10, A61K 9/107, A61K 9/12, A61K 9/14, A61K 9/20, A61K 38/00, A61P 3/00, A61P 3/04, A61P 7/10, A61P 9/04, A61P 9/10, A61P 9/12, A61P 11/02, A61P 17/00, A61P 17/04, A61P 17/14, A61P 29/00

(54) **CNP CYCLIC PEPTIDE, AND MEDICINE, EXTERNAL PREPARATION AND COSMETIC EACH CONTAINING SAID CYCLIC PEPTIDE**

(30) Priority: 31.07.2015 JP 2015152722
(71) Applicant: Igisu Co., Ltd., Tokyo 105-0004 (JP)
(72) Inventor: ENDO, Kyoko, Sendai-shi Miyagi 980-0011 (JP); ENDO, Yori, Sendai-shi Miyagi 980-0011 (JP)
(74) Representative: Zech, Stefan Markus
(86) International application number: PCT/JP2016/072562
(87) International publication number: WO 2017/022728

(57) **Abstract**

The present invention is aimed at providing a novel peptide with quick expression of drug efficacy/effect and long duration of maintaining amelioration, a medicament or an external preparation comprising the same, in particular a prophylactic or therapeutic for dermatitis, skin roughness, rhinitis, alopecia or thinning hair, or a hair growth stimulant, hair growing agent, an antipruritic or a skin-care product, etc. The present invention has reached said object by providing a cyclic peptide having an amino acid sequence expressed by the formula I or a derivative thereof or a pharmaceutically acceptable salt thereof, wherein the amino acid sequence has no peptide bond other than those which are formed between the amino acids constituting the amino acid sequence.

SEQUENCE LISTING

<110>  IGISU Co., Ltd.

<120>  Cyclic peptide and preparation for external use and cosmetics

<130>  PCT2781EN

<160>  62

<170>  PatentIn version 3.5

<210>  1
<211>  17
<212>  PRT
<213>  Artificial Sequence

<220>
<223>  Inventor : Endo Kyoko; Endo Yori


<220>
<221>  DISULFID
<222>  (1)..(17)

<220>
<221>  MUTAGEN
<222>  (3)..(3)
<223>  Xaa is Gly or Ala

<220>
<221>  MUTAGEN
<222>  (6)..(6)
<223>  Xaa is Leu, Ala or Met

<220>
<221>  MUTAGEN
<222>  (9)..(9)
<223>  Xaa is Ile or Val

<220>
<221>  MUTAGEN
<222>  (11)..(11)
<223>  Xaa is Ser, Thr, Ala, Val, Ile or Leu

<220>
<221>  MUTAGEN
<222>  (13)..(13)
<223>  Xaa is Ser, Thr or Ala

<220>
<221>  MUTAGEN
<222>  (15)..(15)
<223>  Xaa is Leu, Ala, Val, Ile, Met or Phe

<400>  1

Cys Phe Xaa Leu Lys Xaa Asp Arg Xaa Gly Xaa Met Xaa Gly Xaa Gly
1               5                   10                  15


Cys

<210>  2
<211>  17
<212>  PRT
<213>  Artificial Sequence

<220>
<223>  Inventor : Endo Kyoko; Endo Yori


<220>
<221>  DISULFID
<222>  (1)..(17)

<220>
<221>  MUTAGEN
<222>  (6)..(6)
<223>  Xaa is Leu, Ala or Met

<220>
<221>  MUTAGEN
<222>  (11)..(11)
<223>  Xaa is Ser, Thr, Ala, Val, Ile or Leu

<220>
<221>  MUTAGEN
<222>  (13)..(13)
<223>  Xaa is Ser, Thr or Ala

<220>
<221>  MUTAGEN
<222>  (15)..(15)
<223>  Xaa is Leu, Ala, Val, Ile, Met or Phe

<400>  2

Cys Phe Gly Leu Lys Xaa Asp Arg Ile Gly Xaa Met Xaa Gly Xaa Gly
1               5                   10                  15


Cys




<210>  3
<211>  17
<212>  PRT
<213>  Artificial Sequence

<220>
<223>  Inventor : Endo Kyoko; Endo Yori


<220>
<221>  DISULFID
<222>  (1)..(17)

<400>  3

Cys Phe Gly Leu Lys Leu Asp Arg Ile Gly Ser Met Ser Gly Leu Gly
1               5                   10                  15

Cys

<210> 4
<211> 22
<212> PRT
<213> Homo sapiens

<220>
<221> DISULFID
<222> (6)..(22)

<400> 4

Gly Leu Ser Lys Gly Cys Phe Gly Leu Lys Leu Asp Arg Ile Gly Ser
1               5                   10                  15

Met Ser Gly Leu Gly Cys
            20

<210> 5
<211> 17
<212> PRT
<213> Artificial Sequence

<220>
<223> Inventor : Endo Kyoko; Endo Yori

<220>
<221> DISULFID
<222> (1)..(17)

<400> 5

Cys Phe Gly Leu Lys Leu Asp Arg Ile Gly Ser Met Ser Gly Leu Gly
1               5                   10                  15

Cys

<210> 6
<211> 17
<212> PRT
<213> Artificial Sequence

<220>
<223> Inventor : Endo Kyoko; Endo Yori

<220>
<221> DISULFID
<222> (1)..(17)

<400> 6

Cys Phe Ala Leu Lys Leu Asp Arg Ile Gly Ser Met Ser Gly Leu Gly
1               5                   10                  15

Cys

<210>  7
<211>  17
<212>  PRT
<213>  Artificial Sequence

<220>
<223>  Inventor : Endo Kyoko; Endo Yori

<220>
<221>  DISULFID
<222>  (1)..(17)

<400>  7

Cys Phe Gly Leu Lys Ala Asp Arg Ile Gly Ser Met Ser Gly Leu Gly
1               5                   10                  15

Cys

<210>  8
<211>  17
<212>  PRT
<213>  Artificial Sequence

<220>
<223>  Inventor : Endo Kyoko; Endo Yori

<220>
<221>  DISULFID
<222>  (1)..(17)

<400>  8

Cys Phe Gly Leu Lys Met Asp Arg Ile Gly Ser Met Ser Gly Leu Gly
1               5                   10                  15

Cys

<210>  9
<211>  17
<212>  PRT
<213>  Artificial Sequence

<220>
<223>  Inventor : Endo Kyoko; Endo Yori

<220>
<221>  DISULFID
<222>  (1)..(17)

<400>  9

Cys Phe Gly Leu Lys Leu Asp Arg Val Gly Ser Met Ser Gly Leu Gly
1               5                   10                  15

Cys

<210>  10
<211>  17
<212>  PRT
<213>  Artificial Sequence

<220>
<223>  Inventor : Endo Kyoko; Endo Yori

<220>
<221>  DISULFID
<222>  (1)..(17)

<400>  10

Cys Phe Gly Leu Lys Leu Asp Arg Ile Gly Thr Met Ser Gly Leu Gly
1               5                   10                  15

Cys

<210>  11
<211>  17
<212>  PRT
<213>  Artificial Sequence

<220>
<223>  Inventor : Endo Kyoko; Endo Yori

<220>
<221>  DISULFID
<222>  (1)..(17)

<400>  11

Cys Phe Gly Leu Lys Leu Asp Arg Ile Gly Ala Met Ser Gly Leu Gly
1               5                   10                  15

Cys

<210>  12
<211>  17
<212>  PRT

<213>  Artificial Sequence

<220>
<223>  Inventor : Endo Kyoko; Endo Yori


<220>
<221>  DISULFID
<222>  (1)..(17)

<400>  12

Cys Phe Gly Leu Lys Leu Asp Arg Ile Gly Val Met Ser Gly Leu Gly
1               5                   10                  15


Cys




<210>  13
<211>  17
<212>  PRT
<213>  Artificial Sequence

<220>
<223>  Inventor : Endo Kyoko; Endo Yori


<220>
<221>  DISULFID
<222>  (1)..(17)

<400>  13

Cys Phe Gly Leu Lys Leu Asp Arg Ile Gly Ile Met Ser Gly Leu Gly
1               5                   10                  15


Cys




<210>  14
<211>  17
<212>  PRT
<213>  Artificial Sequence

<220>
<223>  Inventor : Endo Kyoko; Endo Yori


<220>
<221>  DISULFID
<222>  (1)..(17)

<400>  14

Cys Phe Gly Leu Lys Leu Asp Arg Ile Gly Leu Met Ser Gly Leu Gly
1               5                   10                  15


Cys

<210>  15
<211>  17
<212>  PRT
<213>  Artificial Sequence

<220>
<223>  Inventor : Endo Kyoko; Endo Yori


<220>
<221>  DISULFID
<222>  (1)..(17)

<400>  15

Cys Phe Gly Leu Lys Leu Asp Arg Ile Gly Ser Met Thr Gly Leu Gly
1               5                   10                  15


Cys




<210>  16
<211>  17
<212>  PRT
<213>  Artificial Sequence

<220>
<223>  Inventor : Endo Kyoko; Endo Yori


<220>
<221>  DISULFID
<222>  (1)..(17)

<400>  16

Cys Phe Gly Leu Lys Leu Asp Arg Ile Gly Ser Met Ala Gly Leu Gly
1               5                   10                  15


Cys




<210>  17
<211>  17
<212>  PRT
<213>  Artificial Sequence

<220>
<223>  Inventor : Endo Kyoko; Endo Yori


<220>
<221>  DISULFID
<222>  (1)..(17)

<400>  17

Cys Phe Gly Leu Lys Leu Asp Arg Ile Gly Ser Met Ser Gly Ala Gly
1               5                   10                  15

Cys

<210>  18
<211>  17
<212>  PRT
<213>  Artificial Sequence

<220>
<223>  Inventor : Endo Kyoko; Endo Yori

<220>
<221>  DISULFID
<222>  (1)..(17)

<400>  18

Cys Phe Gly Leu Lys Leu Asp Arg Ile Gly Ser Met Ser Gly Val Gly
1               5                   10                  15

Cys

<210>  19
<211>  17
<212>  PRT
<213>  Artificial Sequence

<220>
<223>  Inventor : Endo Kyoko; Endo Yori

<220>
<221>  DISULFID
<222>  (1)..(17)

<400>  19

Cys Phe Gly Leu Lys Leu Asp Arg Ile Gly Ser Met Ser Gly Ile Gly
1               5                   10                  15

Cys

<210>  20
<211>  17
<212>  PRT
<213>  Artificial Sequence

<220>
<223>  Inventor : Endo Kyoko; Endo Yori

<220>
<221>  DISULFID
<222>  (1)..(17)

<400>  20

Cys Phe Gly Leu Lys Leu Asp Arg Ile Gly Ser Met Ser Gly Met Gly
1               5                   10                  15

Cys

<210>  21
<211>  17
<212>  PRT
<213>  Artificial Sequence

<220>
<223>  Inventor : Endo Kyoko; Endo Yori

<220>
<221>  DISULFID
<222>  (1)..(17)

<400>  21

Cys Phe Gly Leu Lys Leu Asp Arg Ile Gly Ser Met Ser Gly Phe Gly
1               5                   10                  15

Cys

<210>  22
<211>  17
<212>  PRT
<213>  Artificial Sequence

<220>
<223>  Inventor : Endo Kyoko; Endo Yori

<220>
<221>  DISULFID
<222>  (1)..(17)

<220>
<221>  MUTAGEN
<222>  (3)..(3)
<223>  Xaa is Gly or Ala

<220>
<221>  MUTAGEN
<222>  (6)..(6)
<223>  Xaa is Leu, Ala or Met

<400>  22

Cys Phe Xaa Leu Lys Xaa Asp Arg Ile Gly Ser Met Ser Gly Leu Gly
1               5                   10                  15

Cys

<210>  23
<211>  17
<212>  PRT
<213>  Artificial Sequence

<220>
<223>  Inventor : Endo Kyoko; Endo Yori

<220>
<221>  DISULFID
<222>  (1)..(17)

<220>
<221>  MUTAGEN
<222>  (3)..(3)
<223>  Xaa is Gly or Ala

<220>
<221>  MUTAGEN
<222>  (11)..(11)
<223>  Xaa is Thr or Ala

<400>  23

Cys Phe Xaa Leu Lys Leu Asp Arg Ile Gly Xaa Met Ser Gly Leu Gly
1               5                   10                  15

Cys

<210>  24
<211>  17
<212>  PRT
<213>  Artificial Sequence

<220>
<223>  Inventor : Endo Kyoko; Endo Yori

<220>
<221>  DISULFID
<222>  (1)..(17)

<220>
<221>  MUTAGEN
<222>  (3)..(3)
<223>  Xaa is Gly or Ala

<220>
<221>  MUTAGEN
<222>  (13)..(13)
<223>  Xaa is Ser, Thr or Ala

<400>  24

Cys Phe Xaa Leu Lys Leu Asp Arg Ile Gly Ser Met Xaa Gly Leu Gly
1               5                   10                  15

Cys

<210>  25
<211>  17
<212>  PRT
<213>  Artificial Sequence

<220>
<223>  Inventor : Endo Kyoko; Endo Yori

<220>
<221>  DISULFID
<222>  (1)..(17)

<220>
<221>  MUTAGEN
<222>  (3)..(3)
<223>  Xaa is Gly or Ala

<220>
<221>  MUTAGEN
<222>  (15)..(15)
<223>  Xaa is Ala,Val, Ile, Met or Phe

<400>  25

Cys Phe Xaa Leu Lys Leu Asp Arg Ile Gly Ser Met Ser Gly Xaa Gly
1               5                   10                  15

Cys

<210>  26
<211>  17
<212>  PRT
<213>  Artificial Sequence

<220>
<223>  Inventor : Endo Kyoko; Endo Yori

<220>
<221>  DISULFID
<222>  (1)..(17)

<220>
<221>  MUTAGEN
<222>  (6)..(6)
<223>  Xaa is Leu, Ala or Met

<220>

<221>  MUTAGEN
<222>  (11)..(11)
<223>  Xaa is Thr or Ala

<400>  26

Cys Phe Gly Leu Lys Xaa Asp Arg Ile Gly Xaa Met Ser Gly Leu Gly
1               5                   10                  15

Cys

<210>  27
<211>  17
<212>  PRT
<213>  Artificial Sequence

<220>
<223>  Inventor : Endo Kyoko; Endo Yori

<220>
<221>  DISULFID
<222>  (1)..(17)

<220>
<221>  MUTAGEN
<222>  (6)..(6)
<223>  Xaa is Leu, Ala or Met

<220>
<221>  MUTAGEN
<222>  (13)..(13)
<223>  Xaa is Ser, Thr or Ala

<400>  27

Cys Phe Gly Leu Lys Xaa Asp Arg Ile Gly Ser Met Xaa Gly Leu Gly
1               5                   10                  15

Cys

<210>  28
<211>  17
<212>  PRT
<213>  Artificial Sequence

<220>
<223>  Inventor : Endo Kyoko; Endo Yori

<220>
<221>  DISULFID
<222>  (1)..(17)

<220>
<221>  MUTAGEN
<222>  (6)..(6)

<223>  Xaa is Leu, Ala or Met

<220>
<221>  MUTAGEN
<222>  (15)..(15)
<223>  Xaa is Ala,Val, Ile, Met or Phe

<400>  28

Cys Phe Gly Leu Lys Xaa Asp Arg Ile Gly Ser Met Ser Gly Xaa Gly
1               5                   10                  15

Cys

<210>  29
<211>  17
<212>  PRT
<213>  Artificial Sequence

<220>
<223>  Inventor : Endo Kyoko; Endo Yori

<220>
<221>  DISULFID
<222>  (1)..(17)

<220>
<221>  MUTAGEN
<222>  (11)..(11)
<223>  Xaa is Thr or Ala

<220>
<221>  MUTAGEN
<222>  (13)..(13)
<223>  Xaa is Ser, Thr or Ala

<400>  29

Cys Phe Gly Leu Lys Leu Asp Arg Ile Gly Xaa Met Xaa Gly Leu Gly
1               5                   10                  15

Cys

<210>  30
<211>  17
<212>  PRT
<213>  Artificial Sequence

<220>
<223>  Inventor : Endo Kyoko; Endo Yori

<220>
<221>  DISULFID
<222>  (1)..(17)

<220>
<221>  MUTAGEN
<222>  (11)..(11)
<223>  Xaa is Thr or Ala

<220>
<221>  MUTAGEN
<222>  (15)..(15)
<223>  Xaa is Ala,Val, Ile, Met or Phe

<400>  30

Cys Phe Gly Leu Lys Leu Asp Arg Ile Gly Xaa Met Ser Gly Xaa Gly
1               5                   10                  15

Cys

<210>  31
<211>  17
<212>  PRT
<213>  Artificial Sequence

<220>
<223>  Inventor : Endo Kyoko; Endo Yori

<220>
<221>  DISULFID
<222>  (1)..(17)

<220>
<221>  MUTAGEN
<222>  (13)..(13)
<223>  Xaa is Ser, Thr or Ala

<220>
<221>  MUTAGEN
<222>  (15)..(15)
<223>  Xaa is Ala,Val, Ile, Met or Phe

<400>  31

Cys Phe Gly Leu Lys Leu Asp Arg Ile Gly Ser Met Xaa Gly Xaa Gly
1               5                   10                  15

Cys

<210>  32
<211>  17
<212>  PRT
<213>  Artificial Sequence

<220>
<223>  Inventor : Endo Kyoko; Endo Yori

<220>

<221>  DISULFID
<222>  (1)..(17)

<400>  32

Cys Phe Ala Leu Lys Met Asp Arg Ile Gly Ser Met Ser Gly Leu Gly
1               5                   10                  15

Cys

<210>  33
<211>  17
<212>  PRT
<213>  Artificial Sequence

<220>
<223>  Inventor : Endo Kyoko; Endo Yori

<220>
<221>  DISULFID
<222>  (1)..(17)

<400>  33

Cys Phe Ala Leu Lys Leu Asp Arg Ile Gly Thr Met Ser Gly Leu Gly
1               5                   10                  15

Cys

<210>  34
<211>  17
<212>  PRT
<213>  Artificial Sequence

<220>
<223>  Inventor : Endo Kyoko; Endo Yori

<220>
<221>  DISULFID
<222>  (1)..(17)

<400>  34

Cys Phe Ala Leu Lys Leu Asp Arg Ile Gly Ser Met Thr Gly Leu Gly
1               5                   10                  15

Cys

<210>  35
<211>  17
<212>  PRT
<213>  Artificial Sequence

<220>
<223>  Inventor : Endo Kyoko; Endo Yori


<220>
<221>  DISULFID
<222>  (1)..(17)

<400>  35

Cys Phe Ala Leu Lys Leu Asp Arg Ile Gly Ser Met Ser Gly Ile Gly
1               5                   10                  15


Cys




<210>  36
<211>  17
<212>  PRT
<213>  Artificial Sequence

<220>
<223>  Inventor : Endo Kyoko; Endo Yori


<220>
<221>  DISULFID
<222>  (1)..(17)

<400>  36

Cys Phe Gly Leu Lys Met Asp Arg Ile Gly Thr Met Ser Gly Leu Gly
1               5                   10                  15


Cys




<210>  37
<211>  17
<212>  PRT
<213>  Artificial Sequence

<220>
<223>  Inventor : Endo Kyoko; Endo Yori


<220>
<221>  DISULFID
<222>  (1)..(17)

<400>  37

Cys Phe Gly Leu Lys Met Asp Arg Ile Gly Ser Met Thr Gly Leu Gly
1               5                   10                  15


Cys

<210>  38
<211>  17
<212>  PRT
<213>  Artificial Sequence

<220>
<223>  Inventor : Endo Kyoko; Endo Yori


<220>
<221>  DISULFID
<222>  (1)..(17)

<400>  38

Cys Phe Gly Leu Lys Met Asp Arg Ile Gly Ser Met Ser Gly Ile Gly
1               5                   10                  15


Cys




<210>  39
<211>  17
<212>  PRT
<213>  Artificial Sequence

<220>
<223>  Inventor : Endo Kyoko; Endo Yori


<220>
<221>  DISULFID
<222>  (1)..(17)

<400>  39

Cys Phe Gly Leu Lys Leu Asp Arg Ile Gly Thr Met Thr Gly Leu Gly
1               5                   10                  15


Cys




<210>  40
<211>  17
<212>  PRT
<213>  Artificial Sequence

<220>
<223>  Inventor : Endo Kyoko; Endo Yori


<220>
<221>  DISULFID
<222>  (1)..(17)

<400>  40

Cys Phe Gly Leu Lys Leu Asp Arg Ile Gly Thr Met Ser Gly Ile Gly
1               5                   10                  15

Cys

<210>  41
<211>  17
<212>  PRT
<213>  Artificial Sequence

<220>
<223>  Inventor : Endo Kyoko; Endo Yori

<220>
<221>  DISULFID
<222>  (1)..(17)

<400>  41

Cys Phe Gly Leu Lys Leu Asp Arg Ile Gly Ser Met Thr Gly Ile Gly
1               5                   10                  15

Cys

<210>  42
<211>  17
<212>  PRT
<213>  Artificial Sequence

<220>
<223>  Inventor : Endo Kyoko; Endo Yori

<220>
<221>  DISULFID
<222>  (1)..(17)

<220>
<221>  MUTAGEN
<222>  (3)..(3)
<223>  Xaa is Gly or Ala

<220>
<221>  MUTAGEN
<222>  (6)..(6)
<223>  Xaa is Leu, Ala or Met

<220>
<221>  MUTAGEN
<222>  (11)..(11)
<223>  Xaa is Thr or Ala

<400>  42

Cys Phe Xaa Leu Lys Xaa Asp Arg Ile Gly Xaa Met Ser Gly Leu Gly
1               5                   10                  15

Cys

<210> 43
<211> 17
<212> PRT
<213> Artificial Sequence

<220>
<223> Inventor : Endo Kyoko; Endo Yori

<220>
<221> DISULFID
<222> (1)..(17)

<220>
<221> MUTAGEN
<222> (3)..(3)
<223> Xaa is Gly or Ala

<220>
<221> MUTAGEN
<222> (6)..(6)
<223> Xaa is Leu, Ala or Met

<220>
<221> MUTAGEN
<222> (13)..(13)
<223> Xaa is Ser, Thr or Ala

<400> 43

Cys Phe Xaa Leu Lys Xaa Asp Arg Ile Gly Ser Met Xaa Gly Leu Gly
1               5                   10                  15

Cys

<210> 44
<211> 17
<212> PRT
<213> Artificial Sequence

<220>
<223> Inventor : Endo Kyoko; Endo Yori

<220>
<221> DISULFID
<222> (1)..(17)

<220>
<221> MUTAGEN
<222> (3)..(3)
<223> Xaa is Gly or Ala

<220>

<221>  MUTAGEN
<222>  (6)..(6)
<223>  Xaa is Leu, Ala or Met

<220>
<221>  MUTAGEN
<222>  (15)..(15)
<223>  Xaa is Ala,Val, Ile, Met or Phe

<400>  44

Cys Phe Xaa Leu Lys Xaa Asp Arg Ile Gly Ser Met Ser Gly Xaa Gly
1               5                   10                  15

Cys

<210>  45
<211>  17
<212>  PRT
<213>  Artificial Sequence

<220>
<223>  Inventor : Endo Kyoko; Endo Yori

<220>
<221>  DISULFID
<222>  (1)..(17)

<220>
<221>  MUTAGEN
<222>  (6)..(6)
<223>  Xaa is Leu, Ala or Met

<220>
<221>  MUTAGEN
<222>  (11)..(11)
<223>  Xaa is Thr or Ala

<220>
<221>  MUTAGEN
<222>  (13)..(13)
<223>  Xaa is Ser, Thr or Ala

<400>  45

Cys Phe Gly Leu Lys Xaa Asp Arg Ile Gly Xaa Met Xaa Gly Leu Gly
1               5                   10                  15

Cys

<210>  46
<211>  17
<212>  PRT
<213>  Artificial Sequence

<220>

<223>  Inventor : Endo Kyoko; Endo Yori

<220>
<221>  DISULFID
<222>  (1)..(17)

<220>
<221>  MUTAGEN
<222>  (6)..(6)
<223>  Xaa is Leu, Ala or Met

<220>
<221>  MUTAGEN
<222>  (11)..(11)
<223>  Xaa is Thr or Ala

<220>
<221>  MUTAGEN
<222>  (15)..(15)
<223>  Xaa is Ala,Val, Ile, Met or Phe

<400>  46

Cys Phe Gly Leu Lys Xaa Asp Arg Ile Gly Xaa Met Ser Gly Xaa Gly
1               5                   10                  15

Cys

<210>  47
<211>  17
<212>  PRT
<213>  Artificial Sequence

<220>
<223>  Inventor : Endo Kyoko; Endo Yori

<220>
<221>  DISULFID
<222>  (1)..(17)

<220>
<221>  MUTAGEN
<222>  (6)..(6)
<223>  Xaa is Leu, Ala or Met

<220>
<221>  MUTAGEN
<222>  (13)..(13)
<223>  Xaa is Ser, Thr or Ala

<220>
<221>  MUTAGEN
<222>  (15)..(15)
<223>  Xaa is Ala,Val, Ile, Met or Phe

<400>  47

Cys Phe Gly Leu Lys Xaa Asp Arg Ile Gly Ser Met Xaa Gly Xaa Gly

1               5                   10                  15

Cys

<210>  48
<211>  17
<212>  PRT
<213>  Artificial Sequence

<220>
<223>  Inventor : Endo Kyoko; Endo Yori

<220>
<221>  DISULFID
<222>  (1)..(17)

<400>  48

Cys Phe Ala Leu Lys Met Asp Arg Ile Gly Thr Met Ser Gly Leu Gly
1               5                   10                  15

Cys

<210>  49
<211>  17
<212>  PRT
<213>  Artificial Sequence

<220>
<223>  Inventor : Endo Kyoko; Endo Yori

<220>
<221>  DISULFID
<222>  (1)..(17)

<400>  49

Cys Phe Ala Leu Lys Met Asp Arg Ile Gly Ser Met Thr Gly Leu Gly
1               5                   10                  15

Cys

<210>  50
<211>  17
<212>  PRT
<213>  Artificial Sequence

<220>
<223>  Inventor : Endo Kyoko; Endo Yori

<220>

<221>  DISULFID
<222>  (1)..(17)

<400>  50

Cys Phe Ala Leu Lys Met Asp Arg Ile Gly Ser Met Ser Gly Ile Gly
1               5                   10                  15

Cys

<210>  51
<211>  17
<212>  PRT
<213>  Artificial Sequence

<220>
<223>  Inventor : Endo Kyoko; Endo Yori

<220>
<221>  DISULFID
<222>  (1)..(17)

<400>  51

Cys Phe Gly Leu Lys Met Asp Arg Ile Gly Thr Met Thr Gly Leu Gly
1               5                   10                  15

Cys

<210>  52
<211>  17
<212>  PRT
<213>  Artificial Sequence

<220>
<223>  Inventor : Endo Kyoko; Endo Yori

<220>
<221>  DISULFID
<222>  (1)..(17)

<400>  52

Cys Phe Gly Leu Lys Met Asp Arg Ile Gly Thr Met Ser Gly Ile Gly
1               5                   10                  15

Cys

<210>  53
<211>  17
<212>  PRT
<213>  Artificial Sequence

<220>
<223>  Inventor : Endo Kyoko; Endo Yori


<220>
<221>  DISULFID
<222>  (1)..(17)

<400>  53

Cys Phe Gly Leu Lys Met Asp Arg Ile Gly Ser Met Thr Gly Ile Gly
1               5                   10                  15


Cys




<210>  54
<211>  17
<212>  PRT
<213>  Artificial Sequence

<220>
<223>  Inventor : Endo Kyoko; Endo Yori


<220>
<221>  DISULFID
<222>  (1)..(17)

<220>
<221>  MUTAGEN
<222>  (3)..(3)
<223>  Xaa is Gly or Ala

<220>
<221>  MUTAGEN
<222>  (6)..(6)
<223>  Xaa is Leu, Ala or Met

<220>
<221>  MUTAGEN
<222>  (11)..(11)
<223>  Xaa is Thr or Ala

<220>
<221>  MUTAGEN
<222>  (13)..(13)
<223>  Xaa is Ser, Thr or Ala

<400>  54

Cys Phe Xaa Leu Lys Xaa Asp Arg Ile Gly Xaa Met Xaa Gly Leu Gly
1               5                   10                  15


Cys




<210>  55

<211>  17
<212>  PRT
<213>  Artificial Sequence

<220>
<223>  Inventor : Endo Kyoko; Endo Yori


<220>
<221>  DISULFID
<222>  (1)..(17)

<220>
<221>  MUTAGEN
<222>  (3)..(3)
<223>  Xaa is Gly or Ala

<220>
<221>  MUTAGEN
<222>  (6)..(6)
<223>  Xaa is Leu, Ala or Met

<220>
<221>  MUTAGEN
<222>  (11)..(11)
<223>  Xaa is Thr or Ala

<220>
<221>  MUTAGEN
<222>  (15)..(15)
<223>  Xaa is Ala,Val, Ile, Met or Phe

<400>  55

Cys Phe Xaa Leu Lys Xaa Asp Arg Ile Gly Xaa Met Ser Gly Xaa Gly
1               5                   10                  15


Cys




<210>  56
<211>  17
<212>  PRT
<213>  Artificial Sequence

<220>
<223>  Inventor : Endo Kyoko; Endo Yori


<220>
<221>  DISULFID
<222>  (1)..(17)

<220>
<221>  MUTAGEN
<222>  (3)..(3)
<223>  Xaa is Gly or Ala

<220>
<221>  MUTAGEN
<222>  (6)..(6)

<223>  Xaa is Leu, Ala or Met

<220>
<221>  MUTAGEN
<222>  (13)..(13)
<223>  Xaa is Ser, Thr or Ala

<220>
<221>  MUTAGEN
<222>  (15)..(15)
<223>  Xaa is Ala,Val, Ile, Met or Phe

<400>  56

Cys Phe Xaa Leu Lys Xaa Asp Arg Ile Gly Ser Met Xaa Gly Xaa Gly
1               5                   10                  15

Cys

<210>  57
<211>  17
<212>  PRT
<213>  Artificial Sequence

<220>
<223>  Inventor : Endo Kyoko; Endo Yori

<220>
<221>  DISULFID
<222>  (1)..(17)

<400>  57

Cys Phe Ala Leu Lys Met Asp Arg Ile Gly Thr Met Thr Gly Leu Gly
1               5                   10                  15

Cys

<210>  58
<211>  17
<212>  PRT
<213>  Artificial Sequence

<220>
<223>  Inventor : Endo Kyoko; Endo Yori

<220>
<221>  DISULFID
<222>  (1)..(17)

<400>  58

Cys Phe Ala Leu Lys Met Asp Arg Ile Gly Thr Met Ser Gly Ile Gly
1               5                   10                  15

Cys

<210> 59
<211> 17
<212> PRT
<213> Artificial Sequence

<220>
<223> Inventor : Endo Kyoko; Endo Yori

<220>
<221> DISULFID
<222> (1)..(17)

<400> 59

Cys Phe Ala Leu Lys Met Asp Arg Ile Gly Ser Met Thr Gly Ile Gly
1               5                   10                  15

Cys

<210> 60
<211> 17
<212> PRT
<213> Artificial Sequence

<220>
<223> Inventor : Endo Kyoko; Endo Yori

<220>
<221> DISULFID
<222> (1)..(17)

<220>
<221> MUTAGEN
<222> (3)..(3)
<223> Xaa is Gly or Ala

<220>
<221> MUTAGEN
<222> (6)..(6)
<223> Xaa is Leu, Ala or Met

<220>
<221> MUTAGEN
<222> (9)..(9)
<223> Xaa is Ile or Val

<220>
<221> MUTAGEN
<222> (11)..(11)
<223> Xaa is Thr or Ala

<220>
<221> MUTAGEN

<222>  (13)..(13)
<223>  Xaa is Ser, Thr or Ala

<400>  60

Cys Phe Xaa Leu Lys Xaa Asp Arg Xaa Gly Xaa Met Xaa Gly Leu Gly
1               5                   10                  15

Cys

<210>  61
<211>  17
<212>  PRT
<213>  Artificial Sequence

<220>
<223>  Inventor : Endo Kyoko; Endo Yori

<220>
<221>  DISULFID
<222>  (1)..(17)

<220>
<221>  MUTAGEN
<222>  (3)..(3)
<223>  Xaa is Gly or Ala

<220>
<221>  MUTAGEN
<222>  (6)..(6)
<223>  Xaa is Leu, Ala or Met

<220>
<221>  MUTAGEN
<222>  (9)..(9)
<223>  Xaa is Ile or Val

<220>
<221>  MUTAGEN
<222>  (11)..(11)
<223>  Xaa is Thr or Ala

<220>
<221>  MUTAGEN
<222>  (15)..(15)
<223>  Xaa is Ala,Val, Ile, Met or Phe

<400>  61

Cys Phe Xaa Leu Lys Xaa Asp Arg Xaa Gly Xaa Met Ser Gly Xaa Gly
1               5                   10                  15

Cys

<210>  62
<211>  17

<212>  PRT
<213>  Artificial Sequence

<220>
<223>  Inventor : Endo Kyoko; Endo Yori



<220>
<221>  DISULFID
<222>  (1)..(17)

<220>
<221>  MUTAGEN
<222>  (3)..(3)
<223>  Xaa is Gly or Ala

<220>
<221>  MUTAGEN
<222>  (6)..(6)
<223>  Xaa is Leu, Ala or Met

<220>
<221>  MUTAGEN
<222>  (9)..(9)
<223>  Xaa is Ile or Val

<220>
<221>  MUTAGEN
<222>  (13)..(13)
<223>  Xaa is Ser, Thr or Ala

<220>
<221>  MUTAGEN
<222>  (15)..(15)
<223>  Xaa is Ala,Val, Ile, Met or Phe

<400>  62

Cys Phe Xaa Leu Lys Xaa Asp Arg Xaa Gly Ser Met Xaa Gly Xaa Gly
1               5                   10                  15


Cys

## Description

### [Technical Field]

The present invention relates to a CNP cyclic peptide, and a medicament, external preparation and cosmetic comprising the cyclic peptide.

### [Background Arts]

CNP (C-type natriuretic peptide), which belongs to human NP family, have been known to be specifically expressed in brain, unlike ANP and BNP which are classified to the same family. It has ben also known that CNP has a weaker natriuresis utilization and antihypertensive activity as compared to ANP and BNP, but has a stronger effect of increasing intracellular cyclic guanosine monophosphate (cGMP) in vascular smooth muscle cells, etc. (Non-patent Reference 1). Thus, it has been considered that CNP takes other physiological roles than those in maintaining homeostasis in body fluid volume and blood pressure.

It has been known that CNP is highly conserved in its amino acid sequence consitution with little differences between species, and its structure possesses a cyclic moiety and tail part as common structures. In this context, it has been shown that the smallest active structure for increasing intracellular cGMP in vascular smooth muscle cells is the cyclic moiety (hereinbelow may also simply referred to as C-ring) of CNP, and various CNP mutants and derivatives have been reported (Patent References 1 and 6).

Recently, several therapeutic drugs and treatment method utilizing such CNPs have just become known (Patent References 1 to 5), among which there is a report that CNP cyclic peptide is effective for arthritis (Patent Reference 6).

Nevertheless, for treating dermatitis, steroids, tacrolimus, etc. are still used now as main stream. Although the use of these drugs is hesitated in children and particular subjects because of their strong side effects such as developing skin atrophy by long-term topical application, increasing likelihood of causing adrenal insufficiency in cases of an extensive skin rash, and increasing risk of infections due to it immunosuppressing effect, the actual circumstances are that there is no other choice but to use these drugs in order to suppress the conditions of diseases while enduring such strong side effects. In this context, it has been reported that therapeutics for dermatitis comprising CNP have no such side effect and dramatically suppresses the symptoms of dermatitis (Patent References 2 to 4). Besides, steroids are also often used in rhinitis though their therapeutic effect is short for their side effecr and cases are found now and then in which the obtained effect is not as much as expected. On the other hand, therapeutics for rhinitis comprising CNP have been reported to have a long effective duration with no side effects (Patent Reference 2 to 4). Furthermore, in alopecia, for which none of the various drugs that have been used exhibited an effect that was equal to or more than expected, it has been demonstrated that therapeutics for alopecia comprising CNP exhibit significant hair stimulating or hair growing effect (Patent Reference 2 to 4).

### [Prior Art References]

### [Patent References]

[Patent Reference 1] JP B 2809533
[Patent Reference 2] WO 2010/062459
[Patent Reference 3] WO 2011/064644
[Patent Reference 4] WO 2011/024973
[Patent Reference 5] US Patent No. 7276481 Specification
[Patent Reference 6] Published US Patent Application No. 2007/0197434 Specification

### [Non-patent References]

[Non-Patent Reference 1] Furuya M, Takehisa M, Minamitake Y, Kitajima Y, Hayashi Y, Ohnuma N, Ishihara T, Minamino N, Kangawa K, Matsuo H Biochem Biophys Res Commun. 1990 Jul 16; 170(1):201-8.

### [Summary of the Invention]

### [Problems to be Solved by the Invention]

As mentioned above, CNP and CNP cyclic peptide are considered to be a substance that is useful for treating various diseases such as arthritis. However, it is desired to develop substances having more potent drug efficacy/effect and substances that acts quicker and for longer dutation in order to obtain higher therapeutic/treatment effect as compared to conventional drugs. Also, a less expensive formulation as compared to conventional ones are desired.

It is quite meaningful to further increasing sustainability and immediate effectiveness of drug efficacy/effect from the viewpoint of reducing required number/frequency of treatment, palliating pain of patient or user, increasing quality of life and improving daily spiritual/physical/social satisfaction (QOL:quality of life). For example, in dermatitis, in cases where the affected site is accompanied by an itches, erythema or a hot flash, or in diseases where a systemic erythema with thich scales like psoriasis is observed, flaking scales often severely impair QOL with problem of appearance. Quickly reliefing these symtoms will reduce pain of the patient. In rhinitis, which is often accompanied by symptoms of nasal congestion and rhinorrhea, a quick suppression of these symptoms will lead to a reduction of number of blowing, etc., resulting in a reduction of patient's discomfort. When an agent for improving thinning hair, a therapeutic for alopecia, a hair growing agent or a hair growth stimulant is used, user's QOL can be improved if the effect of treatment, hair stimulation, hair growh or hair-falling inhibition is quickly expressed.

The inventors focused on this point and tried to search for a novel substance and a novel application with reference to the structure of CNP.

Accordingly, the object of the present invention is to provide a nobel substance, and to provide a novel peptide having a stronger drug efficacy/effect as compared to conventional ones, a medicament and external preparation, particularly a prophylactic or therapeutic for dermatitis, rhinitis or alopecia, and a hair growth stimulant, a hair growing agent, an antipruritic, a cosmetic and a skin-care product comprising the same.

### [Means to Solve the Problem]

So far, it has been known that cyclic CNP is the smallest active structure of cGMP in smooth muscle cells, though the physical properties of the cyclic CNP are yet to be clarified. There are therapeutic drugs in which various modifications have been added to CNP. However, the inventors have rather focused on the ring part of CNP alone, which has been considered not to necessarily have a high activity, and made an intensive research on cyclic peptides that can be obtained by deleting the tail part. The inventors have thus created a novel substance based on CNP cycle, and could obtain new findings such as that such cyclic peptide has a high level, quick expression and extended duration of effect and drug efficacy, furrther continued the research to complete the present invention.

Accordingly, the present invention relates:
[1] A cyclic peptide having an amino acid sequence expressed by, the formula I: 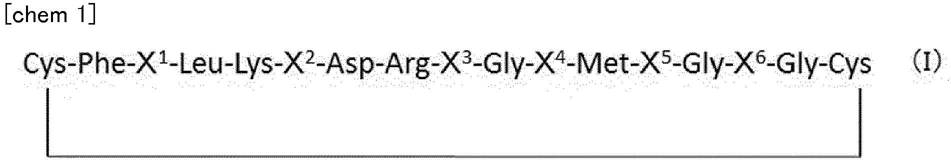 wherein,
   X¹ denotes Gly or Ala,
   X² denotes Leu, Ala or Met,
   X³ denotes Ile or Val,
   X⁴ denotes Ser, Thr, Ala, Val, Ile or Leu,
   X⁵ denotes Ser, Thr or Ala,
   X⁶ denotes Leu, Ala, Val, Ile, Met or Phe,
   the line that links two Cys represents a disulfide bond,
   and wherein the amino acid sequence has no peptide bond other than those which are formed between the amino acids constituting the amino acid sequence,
   or a derivative thereof or a pharmaceutically acceptable salt thereof.
[2] The cyclic peptide according to [1], which is selected from SEQ ID NOs: 60 to 62, or a derivative thereof or a pharmaceutically acceptable salt thereof.
[3] The cyclic peptide according to [1], which is selected from SEQ ID NOs: 22 to 31, 42 to 47 and 54 to 56, or a derivative thereof or a pharmaceutically acceptable salt thereof.
[4] The cyclic peptide according to [1], which is selected from SEQ ID NOs: 6 to 21, 32 to 41, 48 to 53 and 57 to 59, or a derivative thereof or a pharmaceutically acceptable salt thereof.
[5] The cyclic peptide according to any one of [1] to [4], or a derivative thereof or a pharmaceutically acceptable salt thereof, wherein the derivative has been substituted with a substituent that is replaceable for the hydrogen atom, hydroxyl group, carboxyl group, amino group, imino group in the cyclic peptide.
[6] A cyclic peptide or a derivative thereof or a pharmaceutically acceptable salt thereof that is formed by deleting 1 to 6 amino acid(s) in the cyclic peptide according to [4], or by substituting 1 to 6 amino acid(s) in the cyclic peptide according to [4] with other amino acids, or by adding 1 to 6 amino acid(s) to the cyclic peptide according to [4], and that has equal functions to the cyclic peptide expressed by each of said fomulae.
[7] An external preparation comprising one or more cyclic peptides according to any one of [1] to [6], and/or a derivative thereof and/or a pharmaceutically acceptable salt thereof.
[8] The external preparation according to [7], which is an ingredient for a therapeutic for dermatitis, a prophylactic for dermatitis, an antipruritic, an antiphlogistic, an epidermal regeneration accelerating agent or a skin-care product.
[8] The external preparation according to [8], wherein the skin-care product is for moisturization, and/or for preventing/improving skin roughness, and/or for preventing/improving skin sensitivity, and/or for preventing/improving dry skin, and/or for improveming sebum condition/for acne care, and/or for reliefing irritation/for anti-inflammation, and/or for skin lightening, and/or for anti-aging, and/or for preventing/improving wrinkle/flabbiness and/or dullness/dark circles, and/or for preventing/improving photosensitivity, and/or for preventing/reliefing UV damages, and/or for slimming, and/or for skin-cleansing.
[10] The external preparation according to [8], which is a bathing agent, body cleansing agent, hair cleansing agent, hair rinse/treatment, hair tonic, hair oil, hair loation, scalp care agent.
[11] The external preparation according to [7], which is a therapeutic for alopecia, a prophylactic for alopecia, a hair growing agent and/or a hair growth stimulant and/or a prophylactic/inhibitor for hair-falling, a prophylactic cosmetic for hair-falling.
[12] The external preparation according to [7], which is a scalp care agent for preventing/improving dandruff or itch, and/or for improving/preventing hair/scalp dryness, and/or for improving/preventing seborrheic condition of scalp.
[13] The external preparation according to [7],which is a therapeutic for rhinitis and/or a prophylactic for rhinitis and/or a therapeutic for rhinosinusitis and/or a prophylactic for rhinosinusitis.
[14] The external preparation according to [7], which is a cosmetic.
[15] The external preparation according to any one of [7] to [14], wherein the dosage form is a solid, semisolid, powder, liquid, spray, ointment, cream, emulsion, gel or patch.
[16] The external preparation according to any one of [7] to [15] comprising the cyclic peptide according to any one of [1] to [5] and/or a derivative thereof and/or a pharmaceutically acceptable salt thereof at a concentration from 0.0001 to 1000000 *µ*g/mL.
[17] The external preparation according to any one of [7] to [16] for use as a quasi-drug and cosmetic product.
[18] Use of the cyclic peptide according to any one of [1] to [5], and/or a derivative thereof and/or a pharmaceutically acceptable salt thereof for the manufacture of an external preparation.
[19] A method of using the external preparation comprising applying the external preparation according to any one of [7] to [17] to skin and/or mucosa of a subject.
[20] The method of using the external preparation according to [18], wherein the mucosa is a lip, oral cavity, nasal cavity, eye or vagina.
[21] The method of using the external preparation according to [18], which is a method of treating and/or preventing dermatitis, a method of alleviating and/or eliminating dermatitis, a method of treating erosion/ulcer or a skin-care method.
[22] The method of using the external preparation according to [18], which is a method of treating and/or preventing dermatitis selected from a group consisting of eczema, atopic dermatitis, contact dermatitis, photosensitivity, psoriasis, acne, cystic acne, seborrheic eczema, and itches.
[23] The method of using the external preparation according to [18], which is a method of improving and preventing skin roughness, hypersensitivity of skin, dry skin, oily skin, photosensitivity, wrinkles, flabbiness, dullness, dandruffs and itches.
[24] The method of using the external preparation according to [18], which is a method of treating and/or preventing thinning hair/alopecia, and/or a method of stimulating hair growth and/or a method of growing hair, a method of inhibiting and/or preventing hair falling.
[25] The method of using the external preparation according to [18], which is a method of inhibiting and/or preventing dandruffs or itches, and/or a method of improving/preventing hair/scalp dryness, and/or a method of preventing/improving seborrheic condition of scapl.
[26] The method of using the external preparation according to [18], which is a method of treating/preventing rhinitis/rhinosinusitis.
[27] A medicament comprising one or more cyclic peptides according to any one of [1] to [5] or a derivative thereof or a pharmaceutically acceptable salt thereof.
[28] The medicament according to [27], which is a therapeutic for hypertension, unstable angina, acute myocardial infarction, edematous diseases, renal failure, heart failure, immune diseases, autoimmune diseases, allergic diseases, cancer, gastrointestinal diseases, Crohn's disease, ulcerative colitis, obesity, metabolic syndrome.

According to the present invention, a novel cyclic peptide and a composition comprising the same can be provided. Such composition is applied for an external preparation for preventing or treating dermatitis, rhinitis, alopecia, or further for a hair growth stimulant, hair growing agent, hair-falling inhibiting agent, an antipruritic, etc., which are all provided as a medicament, quasi-drug, skin-care product or cosmetic product.

The external preparation of the present invention has a more remarkable effect for dermatitis than conventional steroid external preparations or CNP. Moreover, it generally improves symptoms within 3 minutes, showing an effect that acts quicker, in greater extent and more sustainable with longer dutation of amelioration.

The external preparation of the present invention, when being applied onto skin or mucosa of the subject suffering dermatitis, can also quickly remit or eliminate various perceptible symptoms and conditions which has been caused by or could be caused by dermatitis such as pruritus, sore (pain), heat sensation, tautness, infiltration, erythema, while improving objective symptoms of dermatitis.

Furthermore, the external preparation of the present invention exhibits moisturizing effect on the applied site while it improves skin texture if there is corneum at the applied site, thereby being capable of exhibiting effects such as improving skin texture, dry skin or skin roughness, softening and moisturizing skin, shallowing and making whinkles inconspicuous, preventing photosensitivity, preventing/improving wrinkles and flabbiness, preventing/improving dullness/dark circles, and further improving lip roughness.

The external preparation of the present invention, when being applied onto scapl, also has effects of preventing hair loss and promoting hair-growth stimulation or growing of hair at the applied site, preventing hair-falling, and thickening hair. In this case, stimulated hairs tend to be terminal hairs that are not white hairs. These effects are provided quicker and at a greater level as compared to other ingredients conventionally used in a therapeutic for alopecia, e.g., CNP. Furtherrmore, in any symptoms of M- and O-types in male pattern and female pattern alopecia, provided are remarkable effects of thickening and quickly growing hair and improving thinning of hair.

Furthermore, the external preparation of the present invention also has an immediate effect on rhinitis, and the effect is greater and sustainable for a long duration.

The cyclic peptide of the present invention is a peptide that has a structural common part with CNP, which is an intrinsic hormone by nature, and therefore is considered to have little concern about side effects, and so far no side effect has been found/reported. Also, because it is mainly dermally administrated and the CNP ring has a large molecular weight (>500D) and is non-hydrophobic, the amount of CNP ring absorbed to the body is considered to be very small. Namely, as long as it is used in an appropriate amount, or as long as it is applied externally to skin, etc., it is conjectured that the systemic side effects or the influence on blood circulation dynamics would be small. Therefore, it can be administered for a prolonged period to a patient in need of a long-term administration, e.g., a patient with chronic dermatitis. The external preparation of the present invention also causes little irritation upon external application and thus can be applied to a subject having hypersensitive skin, and to particularly sensitive part such as face and neck. It can also be applied to a pregnant or lactating woman.

The above-mentioned cyclic peptide may also be used as an ingredient for an external preparation such as a dermatitis therapeutic/prophylactic agent, rhinitis therapeutic/prophylactic agent, alopecia therapeutic/prophylactic agent, hair growing agent, hair growth stimulant, therapeutic/prophylactic agent for hair falling, or an antipruritic. It can further be used as an ingredient for a skin-care product, quasi-drug or cosmetic product. It is, of course, also possible to use the above-mentioned cyclic peptide as an alternative for CNP in a medicament utilizing the CNP activity described above, for example a medicament for treating hypertension, unstable angina, acute myocardial infarction, edematous diseases, renal failure, heart failure, immune diseases, autoimmune diseases, allergic diseases, cancer, gastrointestinal diseases, obesity or metabolic syndrome. Moreover, because
the above-mentioned cyclic peptide is smaller than CNP and its production cost is low, a formulation using it can be provided at a lower expence than using CNP.

### [Brief Description of Figures]

[Fig. 1]
   A diagram showing the results of two-sided application in which either C-ring or CNP gel formulation is applied to one of the lower thighs of a patient (P6) who develops psoriasis.
[Fig. 2]
   A diagram showing the results of before and after the application of C-ring gel formulation to a patient who developed male pattern AGA (M- and O-types) (A11).

### [Modes for Carrying out the Invention]

Hereinbelow, the present invention is to be explained in details based on suitable embodiments. CNP cyclic peptide herein (hereinbelow may also be referred to as "CNP cyclic peptide", "C-ring" or "C-ring compound") is, as mentioned above, derived from a wild-type CNP. The wild-type CNPs encompass not only those dervied from human but also those from species that have an identical sequence to human wild-type CNP, for example, without limitation, those derived from monkey, pig, chicken or rat. Accordingly, C-ring peptide encompasses not only those derived from human, but also those derived from, for example, without limitation, monkey, pig, chicken or rat, and it goes without saying that they can naturally act as substitutes to exhibit the effect of the present invention.

Futhermore, the cyclic peptide of the present invention encompasses aforementioned cyclic peptides that have been mutated. Namely, the mutant of the present invention can be obtained by deleting an amino acid(s) in the cyclic peptide, or substitute an amino acid(s) in the cyclic peptide with other amino acid(s), or add other amino acid(s), as long as it maintains CNP activity.
1. Cyclic peptide, derivatives thereof and pharmaceutically acceptable salts thereof First, the cyclic peptide of the present invention, derivatives thereof and a pharmaceutically acceptable salts thereof are explained.

A cyclic peptide having an amino acid sequence expressed by the formula I: 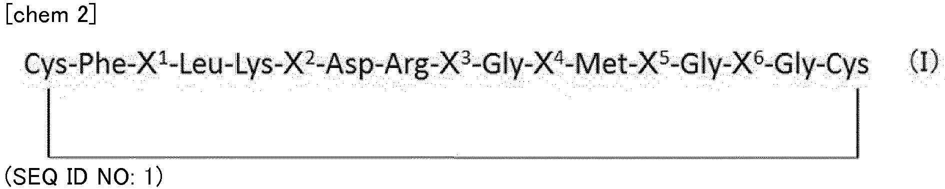 wherein,
X¹ denotes Gly or Ala,
X² denotes Leu, Ala or Met,
X³ denotes Ile or Val,
X⁴ denotes Ser, Thr, Ala, Val, Ile or Leu,
X⁵ denotes Ser, Thr or Ala,
X⁶ denotes Leu, Ala, Val, Ile, Met or Phe,
the line that links two Cys represents a disulfide bond,
and wherein the amino acid sequence has no peptide bond other than those which are formed between the amino acids constituting the amino acid sequence.

Furthermore, in another embodiment of the present invention, the present invention is a cyclic peptide which is amino acid sequence of SEQ ID NO: 1 having CNP activity, and wherein the amino acid sequence has no peptide bond other than those which are formed between the amino acids constituting the amino acid sequence. It is also possible to exclude the amino acid sequence of from the amino acid sequence of SEQ ID NO: 1.

Such cyclic peptide binds to a receptor NPR-B which has a guanylate cyclase domain (also known as GC-B), like CNP, and promotes cGMP production, and thus is conjectured to have actions such as, for example, diuretic action, vasodilation, suppression of renin/aldosterone secretion, suppression of sympathetic nerves, hypertrophy suppression.

Another embodiment of the present invention is cyclic peptide having an amino acid sequence expressed by the formula I: 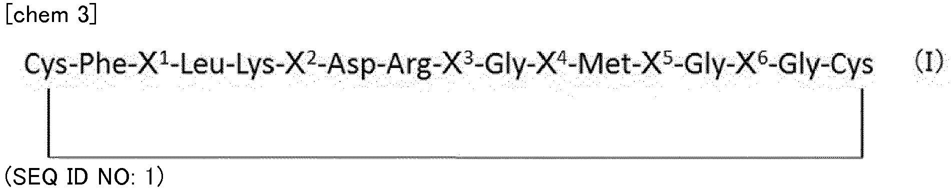 wherein,
X¹ denotes Gly or Ala,
X² denotes Leu, Ala or Met,
X³ denotes Ile or Val,
X⁴ denotes Ser, Thr, Ala, Val, Ile or Leu,
X⁵ denotes Ser, Thr or Ala,
X⁶ denotes Leu, Ala, Val, Ile, Met or Phe,
the line that links two Cys represents a disulfide bond,
provided excluding the cyclic peptide (SEQ ID NO: 3) consisiting of an amino acid sequence expressed by the formula II: 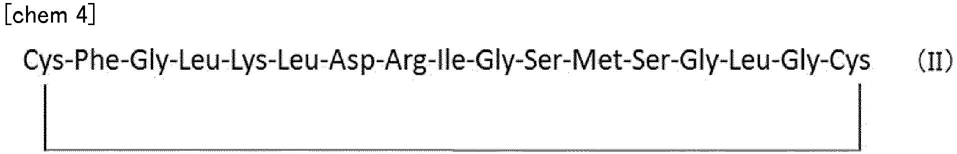 wherein, the line that links two Cys represents a disulfide bond,
   and wherein the amino acid sequence has no peptide bond other than those which are formed between the amino acids constituting the amino acid sequence,
   or a derivative thereof or a pharmaceutically acceptable salt thereof.

It is also prefered that the above-mentioned cyclic peptide is the cyclic peptide of the formula (I) above, wherein
X¹ denotes Gly, and
X³ denotes Ile (SEQ ID NO: 2).

Futhermore, the cyclic peptide of the present invention is preferably a cyclic peptide in which the amino acid sequence expressed by the formula (I) consists of an amino acid sequence expressed by: 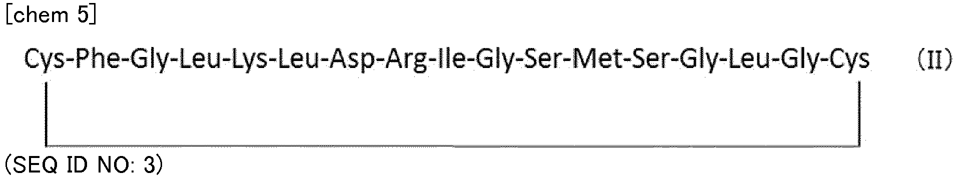 wherein, the line that links two Cys represents a disulfide bond. The amino acid sequence expressed by the formula (II) is the cyclic moiety of CNP (hereinbelow may also be referred to as CNP-17). Namely, the human wild-type CNP (hereinbelow may also be referred to as CNP-22) is Gly-Leu-Ser-Lys-Gly-Cys-Phe-Gly-Leu-Lys-Leu-Asp-Arg-Ile-Gly-Ser-Met-Ser-Gly-Leu-Gly-Cys (SEQ ID NO: 4), in which said peptide correseponds to a peptide between 6th (Cys) and 22nd (Cys) amino acids from the N-terminal in the human wild-type CNP.

In another embodiment of the present invention, it is also possible to exclude the amino acid sequence expressed by the formula (II) (SEQ ID NO: 3) from the peptide consisting of the amino acid sequence expressed by the formula (I) (SEQ ID NO: 1).

Another embodiment of the present invention may be a cyclic peptide or a derivative thereof or a pharmaceutically acceptable salt thereof which has an amino acid sequence selected from SEQ ID NOs: 60 to 62, wherein the amino acid sequence has no peptide bond other than those which are formed between the amino acids constituting the amino acid sequence. Another embodiment of the present invention may be a cyclic peptide or a derivative thereof or a pharmaceutically acceptable salt thereof which has an amino acid sequence selected from SEQ ID NOs: 60 to 62 (provided excluding the amino acid sequence of SEQ ID NO: 3), wherein the amino acid sequence has no peptide bond other than those which are formed between the amino acids constituting the amino acid sequence.

Furthermore, another embodiment of the present invention may be a cyclic peptide or a derivative thereof or a pharmaceutically acceptable salt thereof which has an amino acid sequence selected from SEQ ID NOs: 54 to 56, wherein the amino acid sequence has no peptide bond other than those which are formed between the amino acids constituting the amino acid sequence. Another embodiment of the present invention may be a cyclic peptide or a derivative thereof or a pharmaceutically acceptable salt thereof which has an amino acid sequence selected from SEQ ID NOs: 54 to 56 (provided excluding the amino acid sequence of SEQ ID NO: 3), wherein the amino acid sequence has no peptide bond other than those which are formed between the amino acids constituting the amino acid sequence.

Furthermore, another embodiment of the present invention may be a cyclic peptide or a derivative thereof or a pharmaceutically acceptable salt thereof which has an amino acid sequence selected from SEQ ID NOs: 42 to 47, wherein the amino acid sequence has no peptide bond other than those which are formed between the amino acids constituting the amino acid sequence. Another embodiment of the present invention may be a cyclic peptide or a derivative thereof or a pharmaceutically acceptable salt thereof which has an amino acid sequence selected from SEQ ID NOs: 42 to 47 (provided excluding the amino acid sequence of SEQ ID NO: 3), wherein the amino acid sequence has no peptide bond other than those which are formed between the amino acids constituting the amino acid sequence.

Moreover another embodiment of the present invention may be a cyclic peptide or a derivative thereof or a pharmaceutically acceptable salt thereof which has an amino acid sequence selected from SEQ ID NOs: 22 to 31, wherein the amino acid sequence has no peptide bond other than those which are formed between the amino acids constituting the amino acid sequence. Another embodiment of the present invention may be a cyclic peptide or a derivative thereof or a pharmaceutically acceptable salt thereof which has an amino acid sequence selected from SEQ ID NOs: 22 to 31 (provided excluding the amino acid sequence of SEQ ID NO: 3), wherein the amino acid sequence has no peptide bond other than those which are formed between the amino acids constituting the amino acid sequence.

Furthermore, another embodiment of the present invention may be a cyclic peptide or a derivative thereof or a pharmaceutically acceptable salt thereof which has an amino acid sequence selected from SEQ ID NOs: 6 to 21, 32 to 41, 48 to 53 and 57 to 59, and wherein the amino acid sequence has no peptide bond other than those which are formed between the amino acids constituting the amino acid sequence.

It also goes without saying that the deletion of amino acid(s) in the cyclic peptide of the present invention, substitution by or addition of other amino acid(s) may occur simultaneously, being independent of each other.

The cyclic peptide mutant as above may contain deletion, substitution or addition of more amino acids in the cyclic peptide, if such deletion, substitution or addition would maintain the binding to the CNP receptor or cause a structural change that further enhances the binding, and the number of such deletion, substitution or addition can be, e.g., 1, 2, 3, 4, 5, 6, or more than 6, as long as the CNP activity is maintained.

The cyclic peptide of the amino acid sequence with said deletion, substitution or addition may have 70%, preferably 80%, more preferably 90% or higher homology to the mutant cyclic peptide described above. Furthermore, as long as it maintains the activity, the homology is not limited to this range.

Moreover, any amino acid(s) that constitute(s) the C-ring peptide of the present invention is replaceable and may be replaced with any other amino acid as long as the peptide maintains CNP function (activity). Replaceable amino acids are glycin, leucine, aspartic acid, isoleucine, glycin and serine that constitute the C-ring peptide, which may be replaced with an alanine, methionine, valine, threonine, isoleucine, leucine and/or phenylalanine.

Moreover, the amino acid(s) which can be replaced with other amino acid(s) may be exemplified as in the cyclic peptide (the formula I) in Tables 27 and 28 below, without being limited thereto.

Cyclic peptides in which one amino acid has been substituted include, without being limited, e.g., SEQ ID NOs: 6 to 21. Cyclic peptides in which two amino acids have been substituted include, without being limited, e.g., SEQ ID NOs: 32 to 41. Cyclic peptides in which three amino acids have been substituted include, without being limited, e.g., SEQ ID NOs: 48 to 53. Furthermore, cyclic peptides in which four amino acids have been substituted include, without being limited, e.g., SEQ ID NOs: 57 to 59. Also, five or more amino acids may be substituted by appropriately combining above-described amino acids into a cyclic peptide of SEQ ID NOs: 1 or 60 to 62, etc. It is also possible to appropriately modify other amino acid(s) without being bound by mutable sites of amino acid described in SEQ ID NO: 1, etc.

Furthermore, the present invention may be the mutants described as above and derivatives thereof. Accordingly, any mutants or derivatives thereof are encompassed in the cyclic peptides according to the present invention as long as they have the effect of the present invention. Another embodiment may be a cyclic peptide having at least CNP activity. Here, having CNP activity refers to having 20 % or more biding activity to natriuretic peptide receptor (e.g., GC-A, GC-B). The measurement of above-mentioned CNP activity can be in accordance with a routine procedure.

Moreover, for the purpose of improving the activity of the cyclic peptide of the present invention, retaining the effect of the present invention, and/or increasing storage stability of the cyclic peptide of the present invention, etc., the cyclic peptide of the present invention or the amino acids constituting it may be appropriately modified. For instance, chemically modifying amino acids, etc. of the cyclic peptide of the present invention, deleting a part of the amino acids constituting the cyclic peptide, substituting them with other amino acids, and/or adding new amino acid(s).

The cyclic peptide of the present invention encompasses a said cyclic peptide that has been derivatized, and such derivative can be used directly as an active substance and can also be used as a prodrug.

A derivative of the present invention can be obtained by adding a known substituent (modifying) to a specific group in an amino acid in the cyclic peptide, e.g., hydrogen atom, hydroxyl group, carboxyl group, amino group and imino group, etc., or substituting it with a known replaceable substituent. Modification includes, without Imitation, chemical modification to an amino acid in the cyclic peptide, e.g., glycosylation, acetylation, phosphorylation or lipidation.

The addition (modification) or substitution to the amino acid in the cyclic peptide may occur simultaneously at more than one groups. For such substituents, there is no need to say that any known substituents may be used without limitation as long as they are replaceable for the group as mentioned above, including those mentioned below as well as protective groups such as BOC as mentioned in the followings.

Furthermore, it is also possible, for example, that -COOH of the terminal group of Cys on the one side of the cyclic peptide is substituted with -COOR¹, -CONHR¹ or -CONR¹₂, and/or NH₂ of the N-terminal group of Cys on the other side of the cyclic peptide is substituted with-NHC(O)R¹ or -N(C(O)R¹)₂. Here, each occurence of R¹ is independently a branched or straight-chain hydrocarbon group or alkylene glycol chain or sugar chain having 1 to 20 carbon atoms. The number of carbon atoms of R¹ is preferably 1 to 10, more preferably 1 to 5, further preferably 1 to 2.

Pharmaceutically acceptable salts include, without being particularly limited, as a cyclic peptide of the present invention or a derivative, for example, a salt with an inorganic base, a salt with an organic base, a salt with an inorganic acid, a salt with an organic acid, a salt with a basic, neutral or acidic amino acid. Suitable examples for a salt with an inorganic base include, e.g., alkali metal salts such as a sodium salt and potassium salt; alkali earth metal salts such as a calcium salt and magnesium sail; and an alminium salt and ammonium salt. Suitable examples for a salt with an organic base include, for example, a salt with an alkylamine such as trimethyl amine and triethyl amine; a salt with a heterocyclic amine such as pyridine and picoline; a salt with an alkanolamine such as ethanol amine, diethanol amine and triethanol amine; a salt with a cycloalkylamine such as cyclohexyl amine and dicyclohexyl amine; a salt with an alkylene diamine derivative such as N,N' -dibenzyl ethylene diamine. Suitable examples for an inorganic acid include, e.g., a salt with hydrochloric acid, hydrobromic acid, nitric acid, sulfuric acid and phosphoric acid, etc. Suitable examples for a salt with an organic acid include, e.g., a salt with a monocarboxylic acid such as formic acid, acetic acid, trifluoroacetic acid, piropionic acid, etc.; a salt with a multivalent carboxylic acid such as fumaric acid, oxalic acid, maleic acid, etc.; a salt with an oxycarboxyic acid such as tartaric acid, citric acid, succinic acid, malic acid, etc.; a salt with a sulfonic acid such as methane sulfonic acid, benzen sulfonic acid, p-toluene sulfonic acid, etc.; and a salt with benzoic acid. Suitable examples for a salt with a neutral amino acid include, for example, a salt with glycin, valine, leucine, etc.; suitable examples for a salt with a basic amino acid include, for example, a salt with arginine, lysine, ornithine, etc.; and suitable examples for a salt with an acidic amino acid include, for example, a salt with aspartic acid or glutamic acid.

Among those described above, a cyclic peptide consisting of the amino acid sequence expressed by the formula (I) or its pharmaceutically acceptable salt are preferred. Namely, the amino acid sequence expressed by the formula (I) is preferred not to be substituted.

Method for producing aa cyclic peptide of the present invention, a derivative, and a pharmaceutically acceptable salt thereof is not particularly limited, and for example, known chemical synthetic or genetic engineering synthetic methods can be used.

When the amino acid sequence described above is chemically synthesized, it can be done by any chemical synthetic methods including known peptide synthetic methods such as e.g., a solid- and liquid-phase synthetic methods. For synthesis, a commercially available peptide synthesizer (e.g., Shimadzu Corp.:PSSM-8, etc.) can be used.

Disulfide bonds in the amino acid sequence may be formed by, for example, e.g., DMSO oxidation method, iodine oxidation method, etc., without being particularly limited. In this case, an intracellular disulfide bond may be formed by treating a free sulfhydryl group or a sulfhydryl group protected with a protective group with DMSO or iodine (I₂), the cyclic peptide can be obtained as a result.

Protective groups include, for example, 4-methylbenzyl group (Bzl (4Me)), trityl group (Trt), tert-butyl group, N- (acetyl) amino methyl group (Acm), etc. Deprotection can be carried out by an appropriate treatment that corresponds to these protective group; for instance, 4-methylbenzyl group can be treated with a strong acid, whereas N-(acetyl)amino methyl group can be treated with iodine.

Next, the cyclic peptide is derivatized as needed to obitain a derivative. Derivatization can be done by a known method. A derivative of the cyclic peptide may be produced simultaneously at the time of peptide synthesis by preliminary introducing a substituent in the amino acids that constitute the cyclic peptide.

Next, a pharmaceutically acceptable salt can be produced as needed by carrying out salt formation of the cyclic peptide or its derivative. Salt can be formed, for example, by bringing the cyclic peptide or its derivative into contact with a desired acid or base.

On the other hand, when the peptide is synthesized by genetic engineering, it can be synthesized by a known method, for example, by the methods described in Sambrook J. et al., Molecular Cloning, A Laboratory Manual (4th edition) (Cold Spring Harbor Laboratory Press (2012)). For instance, at first, DNA fragment encoding for the amino acid sequence expressed by the formula (I) is prepared. For instance, in the case of DNA fragment encoding for the amino acid sequence expressed by the formula (I), DNA fragment is prepared by PCR method using a vector,etc. which contains the full length human CNP gene as a tempelate and primers which have been designed to synthesize a given DNA region to amplify the DNA fragment. Alternatively, DNA fragment may be chemically synthesized.

Next, the amplified DNA fragment is annealed to an appropriate vector to obtain a recombinant vector for protein expression, which is then allowed to be taken up by a target-to-be cell before selecting cells that have taken up the recombinant vectors. Finally, the proten produced by the cells (the protein consisting of the amino acid sequence expressed by the formula (I)) is collected.

The formation of disulfide bonds, derivatization, and salt formation in the collected protein can be carried out as mentioned above.

The production of the compound of interest such as the cyclic peptide may be confirmed by a known procedures such as, e.g., reverse phase HPLC and mass spectrometry.

The presence or absence of CNP activity in the obtained compound may easily be confirmed by a known mean; it may be confirmed, e.g., by examining the cGMP producing activity in NPR-B receptor-expressing vells

### 2. External preparation

Next, the external preparation of the present invention is explained. In the present invention, when simply referring to "external preparation", it means an agent that is applied to skin and/or mucosa, such as a medicament, quasi-drug, skin-care product or cosmetic product, without being limited to its application.

The external preparation of the present invention comprises one or more cyclic peptides and/or derivatives thereof and/or pharmaceutically acceptable salts thereof as mentioned above. When two or more cyclic peptides are used, the number of the cyclic peptide to be mixed is not particularly limited, though as many as 2 or 3 is preferred. For instance, SEQ ID NOs: 50, 58, 15 and 9 may be included, though this is not a limitation and these cyclic peptides can appropriately be combined according to the applied subject, etc., as long as the combination would not harm the effect of the present invention.

### 2.1 Uses

The external preparation of the present invention may be used for the uses as described below, for example, without being particularly limited, and in each case, it exhibits a remarkable effect that has not preexisted. The external preparation of the present invention can be used as a medicament, quasi-drug and/or cosmetic product according to the drug efficacy/effect, without particularly being limited in any of the uses.

Hereinbelow, each use is explained in details.

### (1) Therapeutic/prophylactic for dermatitis

The external preparation of the present invention may be a therapeutic for dermatitis and/or a prophylactic for dermatitis.

The external preparation of the present invention, when being applied to the skin or mucosa of the subject who is suffering from dermatitis, quickly remits or eliminates various perceptible symptoms and conditions that were caused or can be caused by dermatitis such as pruritus, sore (pain), heat sensation, tautness, erythema, infiltration, papule, lichenization, crusts, exudation and/or dry skin, while improving objective symptoms of dermatitis. Moreover, the external preparation of the present invention does not cause an irritation at the applied site.

Such effects of the external preparation comprising the cyclic peptide of the present invention are more rapidly expressed as compared to an external preparation comprising CNP that has tail part with longer duration. Although the cause of this favourable effect of the cyclic peptide as compared to CNP is not clear, it is considered that the cyclic peptide of the present invention has a favourable structure for binding to its receptor, NPR-B receptor, as compared to CNP, which allows more rapid binding to NPR-B receptor in the vicinity of the affected site with a prolonged binding time. Also, relatively rapid binding to NPR-B receptor in the vicinity of the affected site prevents the cyclic peptide from diffusing outside of the affected site via bloodstream, allowing the retention of the cyclic peptide for a relatively long time in the vicinity of the affected site.

The external preparation of the present invention not only suppresses or prevents inflammation in dermatitis but also has an effect of recovering or maintaining the barrier function of skin. The barrier function of skin has a function of preventing the entry of the stimulation from external environment or saprophytic bacteria into skin and a function of retaining humidity. By recovering the barrier function by the external preparation, the progress and exacerbation of inflammation can be prevented. The barrier function of skin is greatly influenced by the alignment of the corneocytes in the corneum, i.e., the conditions of skin texture and humidity retention, Because the external preparation of the present invention has effects of improving skin texture and moisturizing skin, it obviously has effects of recovering and maintaining the barrier function of skin.

The external preparation of the present invention is also effective on skin symptoms such as comedo, i.e. so-called acne, red papule, pustule, cyst/nodules.

Moreover, by quickly remitting or eliminating perceptible symptoms and conditions, the burden to the subject (patient) is decreased and QOL of the patient is improved, while preventing the patient from acting so as to damage the affected site such as touching or scratching being bothered by itchness or pain. Such effects of the external preparation of the present invention of remitting or eliminating perceptible symptoms and conditions are generally expressed within 10 minutes, preferably within 5 minutes, more preferably within 3 minutes.

In addition, the external preparation of the present invention is capable of treating dermatitis that has developed dermopathy by a steroid or dermatitis that cannot be treated with general therapeutics for dermatitis such as steroid and tacrolimus, e.g., dermatitis in which a satisfactory therapeutic effect cannot be obtained or dermatitis which has developed a resistance to these formulations or dermatitis for which the use of these formulations is not suitable or desirable. Conventionally used steroid external preparations have a significant problem that, upon ceasing the external application, the severity turns back to the level before starting the external application or is more exacerbated than the level before starting the external application due to the rebound phenomenon. The external preparation of the present invention does not have such problems as rebound phenomenon.

Dermatitis is in general a disease which causes an inflammation onto skin or mucosa. Dermatitis is usually accompanied by one or more symptoms selected from the group consisting of acute eczematous symptoms such as erythema, infiltrative erythema, papule, vesicles, pustules, infiltration, incrustation and desquamation, chronic eczematous symptoms such as lichenization, pigmentation, and scales, crusts (scab) attachment, scratching, scratch scars, prurigo nodularis, erosion, edema, oozing and squamatization.

Dermatitis is not particularly limited as long as it is a disease being accompanied with an inflammation on skin or mucosa, but includes, e.g., eczema such as chronic eczema, dyshidrotic eczema and infantile xerotic eczema; atopic dermatitis; contact dermatitis such as allergic contact dermatitis and primary irritant contact dermatitis; seborrheic dermatitis; asteatotic dermatitis; autosensitization dermatitis; stasis dermatitis; urticaria such as allergic urticaria (e.g., dietary urticaria, drug-inducedurticaria), nonallergic urticaria (e.g., physical urticaria, solar urticaria and cholinergic urticaria); an insect bite; drug eruption; psoriasis such as plaque psoriasis, guttate psoriasis, erythrodermic psoriasis, pustular psoriasis and psoriasis arthropathica; prurigo such as chronic prurigo, acute prurigo, gestational prurigo and prurigo nodularis; rosacea; rosacea-like dermatitis; cutaneous vasculitis such as cutaneous allergic vasculitis; cutaneous pruritus such as systemic cutaneous pruritus, localized cutaneous pruritus, senile cutaneous pruritus and gestational pruritus; solar dermatitis; erythroderma; nummular dermatitis; localized scratch dermatitis; perioral dermatitis; pompholyx; follicular keratosis; lichen planus, dyshidrotic eczema, dyshidrosis, miliaria, acne vulgaris and photodermatosis. The external preparation of the present invention can be applied for the treatment and prevention of any of these diseases.

The external preparation of the present invention exhibits an excellent effect, in particular, against eczema, atopic dermatitis, contact dermatitis, seborrheic dermatitis, an insect bite, allergic or nonallergic urticaria and psoriasis, preferably against eczema, atopic dermatitis, contact dermatitis, an insect bite and photodermatosis, more preferably against eczema and atopic dermatitis. Accordingly, the external preparation of the present invention may be used for the purpose of treating or preventing one or more dermatitis selected from the aforementioned group.

### (2) Ingredients for a cosmetic or skin-care product

The external preparation of the present invention may be an ingredient for a cosmetic or skin-care product.

The external preparation of the present invention, upon being applied to skin and/or mucosa, provides humidity at the applied site, exhibits humidity-retaining effect and prevents desiccation, while preventing roughness and hypersensitivity of skin. It exhibits an effect of improving skin texture at the applied site when there is corneum. Moreover, it provides/retains skin and mucosa an appropriate elasticity and softness, softens skin, provides resilience and give firmness to skin and mucosa. Also, wrinkles and flabbiness including ripples and large wrinkles are improved at the applied site, and further makes dullness and spots inconspicuous. Making dullness and spots inconspicuous finally provide a whitening effect. Moreover, photosensitivity can be prevented.

As a result, the external preparation of the present invention can be, without limitation, used for the purpose of maintaining or improving skin and/or mucosa condition, for example, for the purpose of one or more selected from the group consisting of preventing or improving the development or progression of dry skin, skin roughness, hypersensitivity of skin, rough lips, flabbiness, ripples and large wrinkles, and of maintaining skin or mucosa condition, preventing photosensitivity, anti-aging and whitening, or of obtaining at least one effect selected from the effects as described abive.

In the present specification, skin roughness incudes miliaria, frostbites, cracks, chaps, acne, diaper rash, festering, sore crotch and razor rash.

Specific uses of the external preparation of the present invention as an ingredient for a cosmetic or skin-care product include, without limitation, e.g., a skin loation, emulsion, beauty essence, cream, cold cream, gel, facial mask, facial pack, powders, handcream, body powders, after-shave lotion, pre-shave lotion, perfume, deodorant, as wels as make-up cosmetics such as a foundation cream, face powder, eyeshadow, eyeliner, mascara, eyebrow, blush, make-up base, lipstick, lip cream and nail color, and furthermore hair cosmetics such as a shanpoo, hair rinse, conditioner, hair color, hair tonic, hair-set agent, hair-permanent agent, and body cleansing agents such as facial wash, cleansing, body soap and hand soap, and baths (bathing agent).

The above-mentioned effects of the external preparation of the present invention are rapidly expressed depending on the type of the effect, but generally within 10 minutes, preferably within 5 minutes, more preferably within 3 minutes of the application.

Also, the above-mentioned effects of the external preparation of the present invention continue for a relatively long time depending on the type of the effect, but generally for 4 hours or more, preferably for 8 hours or more, more preferably for 24 hours or more.

### (3) Antipruritics

The external preparation of the present invention may be an antipruritic.

As mentioned above, the external preparation of the present invention is suitable as an antipruritic because it quickly exhibits an excellent antipruritic effect at the applied site upon being applied onto skin or mucosa.

The antipruritic effect of the external preparation of the present invention as mentioned above is generally expressed within 10 minutes, preferably within 5 minutes, more preferably within 3 minutes of the application.

### (4) A therapeutic for alopecia, prophylactic for alopecia, hair growing agent, hair growth stimulant or prophylactic for hair-falling

The external preparation of the present invention also may be a therapeutic for alopecia, prophylactic for alopecia, hair growing agent, hair growth stimulant and/or prophylactic for hair-falling.

The external preparation of the present invention, when being applied to a site of hair loss or site of hair stimulation, has an effect of preventing hair loss/hair falling at the applied site, while promoting hair stimulation or hair growth and thickening hair. It also has an effect of nourishing hair, promoting the emergence of hair and improving/preventing thinning of hair at the applied site. In this case, the stimulated hair tends to be terminal hair which is not white hair. These effects are expressed relatively quicker as compared to other active agents, e.g., CNP, that have been used in conventional therapeutic for alopecia.

The external preparation of the present invention also has an effect of improving and preventing dermatitis as mentioned above. Accordingly, it can improve or prevent skin inflammation which accompanies alopecia. Such effect is particularly advantageous when alopecia has been exacerbated due to the skin condition of the applied site such as scapl.

Moreover, the external preparation of the present invention, when being applied to skin, exhits effects of retaining humidity and improving skin texture at the applied site as mentioned above.

It can remove and suppresses the development of dandruffs or itches, while providing humidity to hair and scalp and improving/preventing desiccation, improving seborrheic condition and keeping scalp and hair healthy. It can also improve seborrheic condition.

The external preparation of the present invention also has an effect of remitting or eliminating perceptible symptoms and conditions as mentioned above. This decreases the burden to the subject (patient) and improves QOL of the patient. It further prevent the patient from acting so as to damage the affected site such as touching or scratching being bothered by itchness or pain, and as a result, can prevent the exacerbation of the skin condition which causes alopecia.

When the external preparation of the present invention is used as a therapeutic or prophylactic for alopecia, applicable alopecia includes, without being particularly limited, e.g., alopecia described below. The external preparation of the present invention can be used for the purpose of treating or preventing one or more of these alopecia.

### (Acquired alopecia)

(i) alopecia without accompanying scarring or skin lesions (alopecia areata, male pattern alopecia, seborrheic alopecia, alopecia pityroides, female pattern alopecia, gestational alopecia, malignant alopecia, senile alopecia, alopecia totalis, alopecia areata multilocularis, ophiasis, drug-induced alopecia, cancer chemotherapic agent-induced alopecia and radiation exprosure-induced alopecia, traumatic/mechanical alopecia, alopecia associated with a nutritional disorder/metabolic disorder, endocrinopathy-associated alopecia, telogen effluvium (postpartum alopecia, post-hyperthermia alopecia)).
(ii) Alopecia observed in skin lesion or pathologic skin (an infection-induced hair loss, tumor-induced hair loss, and inflammation-induced hair loss).
(iii) Cicatricial alopecia (hair loss induced by skin infection, hair loss induced by the infiltration by inflammatory cells).

### (Congenital alopecia)

Diffuse alopenia, congenital atrichia, alopenia in heritable syndromes, localized alopenia, phakomatosis, aplasia cutis, congenital triangular alopecia.

Among those described above, the external preparation of the present invention exhibits an excellent effect against acquired alopecia, in particular, preferably against alopecia without accompanying scarring or skin lesions, more preferably against alopecia areata, male pattern alopecia, seborrheic alopecia, alopecia pityroides, female pattern alopecia, gestational alopecia, malignant alopecia, senile alopecia, alopecia totalis, alopecia areata multilocularis, ophiasis, drug-induced alopecia, cancer chemotherapic agent-induced alopecia and radiation exprosure-induced alopecia, traumatic/mechanical alopecia, alopecia associated with a nutritional disorder/metabolic disorder, endocrinopathy-associated alopecia and telogen effluvium. Accordingly, the external preparation of the present invention can be used for the purpose of treating or preventing at least one of the alopecia selected from the aforementioned group.

### (5) A therapeutic and/or prophylactic for rhinitis

The external preparation of the present invention may also be a therapeutic and/or prophylactic for rhinitis.

Rhinitis is a disease caused by an inflammation in nasal and/or sinonasal mucosa, and induces main symptoms such as nasal congestion, rhinorrhea and sudden and repeated sneezing, as well as symptoms such as pruritus. In the present invention, "rhinitis" include not only rhinitis of narrow sense which is accompanied with an inflammation in nasal mucosa, but also rhinosinusitis being accompanied with an inflammation in sinonasal mucosa.

The external preparation of the present invention can, when being applied to nasal and/or sinonasal mucosa, improve or prevent various symptoms which accompanies rhinitis such as nasal congestion, rhinorrhea, sneezing and pruritus. Particularly, these effects are more quickly expessed for a longer duration as compared to a therapeutic for rhinitis that comprises CNP having the tail part.

Such effects of the external preparation of the present invention on rhinitis are expressed in general within 8 minutes preferably within 5 minutes, more preferably within 3 minutes, further particularly preferably within 1 minute of the application.

Also, the effect of the external preparation of the present invention on rhinitis as mentioned above last in general for 4 hours or more, preferably for 8 hours or more, more preferably for 24 hours or more.

When the external preparation of the present invention is used as a therapeutic and/or prophylactic for rhinitis, applicable rhinitis include, without being particularly limited, e.g., infectious rhinitis including acute rhinitis and chronic rhinitis; hypersensitivity non-infectious rhinitis including combined (flower-hypersensitive) rhinitis, rhinorrhea-type rhinitis, congestive-type rhinitis, edema-type rhinitis and dry nose-type rhinitis; irritant rhinitis including physical rhinitis, chemical rhinitis and radiation rhinitis; as well as atrophic rhinitis and idiopathic granulomatous rhinitis; rhinosinusitis such as acute rhinosinusitis, chronic rhinosinusitis (maxillary empyema), eosinophilic rhinosinusitis, fungal infection in sinonasal cavity. The external preparation of the present invention can be used for the purpose of treating or preventing one or of thse described above.

Combined rhinitis includes, e.g., allergic rhinitis including perennial allergic rhinitis and seasonal allergic rhinitis as well as nonallergic rhinitis including vasomotor (essential) rhinitis and eosinophilic rhinitis.

Rhinorrhea-type rhinitis includes, e.g., gustatory rhinitis, cold air-induced rhinitis and senile rhinitis.

Congestive-type rhinitis includes, e.g., drug-induced rhinitis, psychogenic rhinitis, gestational rhinitis, endocrine rhinitis and cold rhinitis.

Edema-type rhinitis includes, e.g., aspirin-sensitive rhinitis.

Among those described above, the external preparation of the present invention exhibits an excellent effect particularly against hypersensitive non-infectious rhinitis, irritative rhinitis and rhinosinusitis, preferably against hypersensitive non-infectious rhinitis and chronic rhinosinusitis, more preferably against combined (flower-hypersensitive) rhinitis, rhinorrhea-type rhinitis, congestive-type rhinitis, edema-type rhinitis and dry nose-type rhinitis, chronic rhinosinusitis. Accordingly, the external preparation of the present invention can be used for the purpose of treating or preventing one or more rhinitis selected from the aforementioned group.

From the viewpoint of symptoms, the external preparation of the present invention can be used for any of sneezing/rhinorrhea-type rhinitis, nasal congestion-type rhinitis and rhinitis with both symptoms, since it has effects as mentioned above.

Furthermore, the external preparation of the present invention exhibits an effect of improving symptoms against rhinitis that is difficult to be treated by conventionally-used therapeutic for rhinitis such as steroid drugs.

### (6) Other external preparation

The external preparation of the present invention may also be used for uses other than those mentioned above for the purpose of exhibiting above-mentioned effects of the cyclic peptide. In this case, the external preparation of the present invention can be directed at exhibiting an effect other than above-mentioned effects of the cyclic peptide as a principal purpose. In this case, the cyclic peptide of the present invention, a derivative and/or pharmaceutically acceptable salt thereof is used for the purpose of assisting the principal effect of the external preparation or adding an effect other than the principal effect.

Such uses include such as, without limitation, e.g., deodorization, anti-perspiration, depilation, cleansing, promotion of blood circulation/ perfuming, prevention of ultraviolet damage, ultraviolet protection, sterilization, antisepsis, antioxidation, promotion of percutaneous absorption.

### 2.2 Dosage forms

The external preparation of the present invention can express the effect of its active ingredient, i.e., the cyclic peptide, a derivative and/or pharmaceutically acceptable salt thereof, more reliably and quickly in the vicinity of the applied site by being locally administered at the site of interest (e.g., affected site) of skin or mucosa.

Such an external preparation may be, without limitation, e.g., an agent for integument, eye drop, ear drop, nasal drop, buccal or suppository. Among these, when the external preparation of the present invention is either a therapeutic for dermatitis, prophylactic for dermatitis, antipruritic, skin-care product, therapeutic for alopecia, prophylactic for alopecia, hair growing agent or hair growth stimulant, it is preferred to be an agent for integument. On the other hand, when the external preparation of the present invention is a therapeutic for rhinitis and/or prophylactic for rhinitis, it is preferred to be a nasal drop. Moreover, when it is a therapeutic and/or prophylactic for a corneal disease, it is preferred to be an eye drop.

When tthe external preparation of the present invention is an agent for integument, it may be, without being particularly limited, e.g., an external solid agent, external liquid agent, spray agent, ointment, emulsion, cream, gel or patch.

An external solid agent is a solid formulation for being applied or sprayed onto skin, etc. Such external solid agents include, e.g., a powdered external preparation.

An external liquid agent is a liquid formulation for being applied onto skin, etc. Such external liquid agents include, e.g., a lotion and liniment.

A spray agent is a formulation that sprays an effective ingredient onto skin as a mist, powder, foam or paste. Such spray agents include such as, for example, an external aerosol and pumpspray agent.

An ointment is a semi-solid formulation to be applied onto skin, in which the effective ingredient is dissolved or dispersed in a base. An ointment may also be a lip cream to be applied locally such as onto lips.

A cream is a semi-solid formulation to be applied onto skin, which is either an oil-in-water or water-in-oil type emulsion.

A gel is a gel formulation to be applied onto skin. Gels include, e.g., an aqueous gel or oily gel.

A patch is a formulation to be stuck onto skin. Patches include, e.g., a tape and poultice.

A nasal drop is a formulation to be administered to nasal cavity or nasal mucosa. Nasal drop preparations include, for example, powder for nasal application and liquid agent for nasal application. Among these, a liquid agent for nasal application is preferred.

In any dosage forms mentioned above, the external preparation of the present invention comprises at least the cyclic peptide of the present invention and/or a derivative thereof and/or a pharmaceutically acceptable salt thereof as mentioned above.

When the dosage form is an external liquid agent, ointment, cream or gel, the external preparation of the present invention comprises the cyclic peptide and/or a derivative thereof and/or a pharmaceutically acceptable salt thereof at a concentration, for example, from 0.0001 to 1000000 *µ*g/g, preferably from 0.001 to 10000 *µ*g/g, more preferably from 0.01 to 1000 *µ*g/g, further preferably from 0.1 to 100 *µ*g/g. In another embodiment, it may be comprised at a concentration from 0.1 to 800 *µ*g/g, 1 to 500 *µ*g/g.

When the dosage form is a spray agent, the external preparation of the present invention comprises the cyclic peptide and/or a derivative thereof and/or a pharmaceutically acceptable salt thereof in the stock solution of the spray agent at a concentration, for example, from 0.0001 to 1000000 *µ*g/mL, preferably from 0.001 to 10000 *µ*g/mL, from 0.01 to 1000 *µ*g/mL, more preferably from 0.1 to 100 *µ*g/mL, further preferably from 1 to 100 *µ*g/mL. In another embodiment, it may be comprised at a concentration from 0.1 to 800 *µ*g/mL, 1 to 500 *µ*g/mL may be comprised.

When the dosage form is a patch, the external preparation of the present invention comprises the cyclic peptide and/or a derivative thereof and/or a pharmaceutically acceptable salt thereof at a concentration, for example, from 0.0001 to 1000000 *µ*g/mL, preferably from 0.001 to 10000 *µ*g/mL, more preferably from 0.01 to 1000 *µ*g/mL, further preferably from 0.1 to 100 *µ*g/mL, particularly preferably from 1 to 100 *µ*g/mL. In another embodiment, it may be comprised at a concentration from 0.1 to 800 *µ*g/mL, from 1 to 500 *µ*g/mL.

When the dosage form is a nasal drop, a liquid nasal drop, in particular, the external preparation of the present invention comprises the cyclic peptide and/or a derivative thereof and/or a pharmaceutically acceptable salt thereof in the liquid of the nasal drop (nasal drop liquid) at a concentration, for example, from 0.001 to 10000 *µ*g/mL, more preferably from 0.01 to 1000 *µ*g/mL, preferably from 0.1 to 100 *µ*g/mL, further preferably from 1 to 100 *µ*g/mL. In another embodiment, it may be comprised at a concentration from 0.1 to 800 *µ*g/mL, from 1 to 500 *µ*g/mL.

The external preparation of the present invention can be formulated using methods and constituents which are known to those skilled in the art for any of the dosage forms as mentioned above.

Available constituents include, without being particularly limited, such as, e.g, a thickening gelling agent, oil phase ingredient, alcohol, fatty acid, organic acid, ultraviolet absorbent, ultraviolet scattering agent, particulate matter, pigment, surfactant, polyhydric alcohol,/sugar, polymer, physiologically active ingredient, solvent, antioxidant, chelating agent, flavouring agent, antiseptic agent, animal or plant extract.

As thickening gelling agents, various gelling agents of organic and inorganic compounds may be used.

Gelling agents of inorganic compound include such as, for example, hydrous or water-absorbable silicate, e.g., alminium silicate (e.g., bentonite), magnesium silicate-alminium and colloidal silica. As gelling agents of organic compound, natural, semisynthetic or synthetic polymers can be used. Natural and semisynthetic polymers include such as, for example, polysaccharides such as cellulose, starch, tragacanth, gum arabic, xanthan gum, agar-agar, gelatine, alginic acid and salts threof (e.g., sodium alginate and a derivative thereof), lower alkyl cellulose (e.g., methylcellulose or ethylcellulose), carboxy- or hydroxy-lower-alkyl cellulose(e.g., carboxymethylcellulose or hydroxy propyl cellulose). Synthetic polymer include such as, for example, carboxylvinyl polymer, sodium polyacrylate, (vinylmethyl ether/ethyl maleate) copolymer, polymethacrylate, polyvinyl alcohol, polyvinyl pyrrolidone, polyacrylic acid or polymethacrylic acid. It is also possible to use a commercially available gelling agent as the gel, such as, for example, Lubrajel NP, Lubrajel CG, Lubrajel DV, Lubrajel MS, Lubrajel OIL, Lubrajel TW, Lubrajel DS, (Ashland Inc.), etc.

As an oil, various oil phase ingredients of, for example, ester-type, ether-type, hydrocarbon-type, silicone -type and fluorine-type, as well as animal and plant oils and hydrogenated oils thereof, and waxes of natural origins may be used.

Ester-type oil phase ingredients include, for example, glyceril tri 2-ethyl hexanoate, cetyl 2-ethyl hexanoate, isopropyl myristate, butyl myristate, isopropyl palmitate, ethyl stearate, octyl palmitate, isocetyl isostearate, butyl stearate, butyl myristate, ethyl linoleate, isopropyl linoleate, ethyl oleate, isocetyl myristate, isostearyl myristate, isostearyl palmitate, octyldodecyl myristate, isocetyl isostearate, dietylsebacate, diisopropyladipate, isoalkylneopentanoate, glycelyl tri(caprylate/caprate), trimethylolpropane tri 2-ethyl hexanoate, trimethylolpropane triisostearate, pentaerythritol tetra 2-ethyl hexanoate, cetyl caprylate, decyl laurate, hexyl laurate, decyl myristate, myristyl myristate, cetyl myristate, stearyl stearate, decyl oleate, cetyl ricinoleate, isostearyl laurate, isotridecyl myristate, isocetyl myristate, isostearyl myristate, isocetyl palmitate, isostearyl palmitate, octyl stearate, isocetyl stearate, isodecyl oleate, octyldodecyl oleate, octyldodecyl linoleate, isopropyl isostearate, cetostearyl 2-ethyl hexanoate, stearyl 2-ethyl hexanoate, hexyl isostearate, ethyleneglycol dioctanoate, ethyleneglycol dioleate, propyleneglycol dicaprate, propyleneglycol di(caprylate/caprate), propyleneglycol dicaprylate, neopentylglycol dicaprate, neopentylglycol,dioctanoate,
glycelyl tricaprylate, glycelyl triundecylate, glycelyl triisopalmitate, glycelyl triisostearate, octyldodecyl neopentanoate, isostearyl octanoate, octyl isononanate, hexyldecyl neodecanoate, octyldodecyl neodecanoate, isocetyl isostearate, isostearyl isostearate, octyldecyl isostearate, polyglycerin oleate ester, polyglycerin isostearate ester, dipropyl carbonate, dialkyl carbonate (C12-18), triisocetyl citrate, triisoalkyl citrate, triisooctyl citrate, lauryl lactate, myristyl lactate, cetyl lactate, octyldecyl lactate, triethyl citrate, acetyltriethyl citrate, acetyltributyl citrate, trioctyl citrate, diisostearyl malate, 2-ethylhexyl hydroxystearate, di 2-ethylhexyl succinate, diisobutyl adipate, diisopropyl sebacate, dioctyl sebacate, cholesteryl stearate, cholesteryl isostearate, cholesteryl hydroxystearate, cholesteryl oleate, dihydrocholesteryl oleate, phytosteryl isostearate, phytosteryl oleate, isocetyl 12-stearoylhydroxy stearate, stearyl 12-stearoylhydroxy stearate, isostearyl 12-stearoylhydroxy stearate, etc.

Hydrocarbon-type oil phase ingredients include, for example, squalane, liquid paraffin, *α*-olefin oligomer, isoparaffin, ceresin, paraffin, liquid isoparaffin, solid paraffin, polybutene, microcrystalline wax, petroleum jelly, etc.

Silicone -type oil phase ingredients include, for example, dimethyl polysiloxane, methylphenyl polysiloxane, methylcyclopolysiloxane, octamethyl polysiloxane, decamethylpolysiloxane, dodecamethyl cyclosiloxane, methyl hydrogen polysiloxane, polyether denatured organopolysiloxane, dimethyl siloxane/methylcetyloxysiloxane copolymer, dimethyl siloxane/methylstearoxysiloxane copolymer, alkyl denatured organopolysiloxane, terminal denatured organopolysiloxane, amino denatured silicone oil, amino denatured organopolysiloxane, dimethiconol, silicone gel, acryl silicone, trimethyl siloxysilicate, silicone RTV gum, etc. Fluorine-type oil phase ingredients include, for example, perfluoropolyether, fluorine denatured organopolysiloxane, pitch fluoride, fluorocarbon, fluoroalcohol, fluoroalkyl/polyoxy alkylene co-denatured organopolysiloxane, etc.

Animal and plant oils and hydrogenated oils thereof, and waxes of natural origin include, for example, animal and plant oils and hydrogenated oils thereof such as beef tallow, hydrogenated beef tallow, lard, hydrogenated lard, horse oil, hydrogenated horse oil, mink oil, Orange roughy oil, fish oil, hydrogenated fish oil, egg yolk and jojoba oil, plant oils and hydrogenated oils thereof such as avocado oil, almond oil, olive oil, cocoa butter, apricot kernel oil, Kukui nut oil, sesame oil, wheat germ oil, rice germ oil, rice bran oil, safflower oil, shea butter, soybean oil, evening primrose oil, camellia oil, corn oil, rapeseed oil, hydrogenated rapeseed oil, palm kernel oil, hydrogenated palm kernel oil, palm oil, hydrogenated palm oil, peanut oil, hydrogenated peanut oil, castor oil, hydrogenated castor oil, sunflower oil, grape seed oil, jojoba oil, hydrogenated jojoba oil, macadamia nut oil, medhom oil, cotton seed oil, hydrogenated cotton seed oil, coconut oil, hydrogenated coconut oil and rose hip oil, waxes such as bee wax, high acid number bee wax, lanolin, reduced lanolin, hydrogenated lanolin, liquid lanolin, carnauba wax, montan wax.

Higher alcohols include, for example, lauryl alcohol, myristyl alcohol, cetyl alcohol, stearyl alcohol, isostearyl alcohol, oleyl alcohol, behenyl alcohol, 2-ethyl hexanol, hexadecyl alcohol, octyl dodecanol, etc.

Fatty acids include, for example, caprylic acid, capric acid, undecylenic acid, lauric acid, myristic acid, palmitic acid, palmitate, palmitoleic acid, stearate, isostearate, oleate, linoleate, linolenic acid, arachic acid, arachidonic acid, behenic acid, erucic acid, 2-ethyl hexanoic acid, etc.

Ultraviolet absorbents include, for example, p-amino benzoate, p-amino amyl benzoate, p-amino ethyldihydroxypropyl benzoate, p-amino glycelyl benzoate, p-amino ethyl benzoate, p-amino octyl benzoate, p-amino octyldimethyl benzoate, ethylene glycol salicylate, octyl salicylate, triethanol salicylate amine, phenyl salicylate, butylphenyl salicylate, benzyl salicylate, homomenthyl salicylate, benzyl cinnamate, paramethoxy octyl cinnamate, paramethoxy 2-ethylhexyl cinnamate, diparamethoxy cinnamate glycelyl mono 2-ethyl hexanoate, paramethoxy isopropyl cinnamate, paramethoxy hydrocinnamate diethanol amine salt, diisopropyl/diisopropyl cinnamate ester mixture, urocanate, ethyl urocanate, hydroxymethoxy benzophenone, hydroxymethoxy benzophenonesulfonate and salts thereof, dihydroxymethoxy benzophenone, sodium dihydroxymethoxy benzophenone disulfonate, dihydroxy benzophenone, dihydroxydimethoxy benzophenone, hydroxyoctoxy benzophenone, tetrahydroxy benzophenone, butylmethoxy dibenzoyl methane, 2,4,6-trianilino-p-(carbo-2-ethylhexyl-1-oxy)-1,3,5-triazine, 2-(2-hydroxy-5-methylphenyl)benzotriazol, methyl-O-aminobenzoate, 2-ethylhexyl-2-cyano-3,3-diphenylacrylate, phenyl benzimidazole sulfuric acid, 3- (4-methyl benzylidene)camphor, isopropyl dibenzoyl methane, 4-(3,4-dimethoxyphenyl methylene)-2,5-dioxo-1-imidazolidine 2-ethylhexyl propionate, etc., and polymer derivatives and silane derivatives thereof, titanium oxide and zinc oxide and dispersions thereof, etc. Titanium oxide, zinc oxide, etc. may be those which have been surface-treated.

Percutaneous absorption auxiliaries include, for example, acetic acid, sodium acetate, limonene, menthol, salicylic acid, hyaluronic acid, oleic acid, N,N-dietyl-m-toluamide (N,N-dietyl-3-methyl benzamide), n-butyl stearate, benzyl alcohol, isopropyl myristate, isopropyl palmitate, polypropylene glycol, crotamiton, sebacate dietyl, N-methylpyrrolidone, N-ethylpyrrolidone, lauryl alcohol, etc.

Powders and pigments include, for example, pigments such as Red No. 104 (Phloxine B), Red No. 201 (Lithol Rubine B), Yellow No. 4 (Tartrazine), Blue No. 1 (Brilliant Blue), Black No. 401 (Naphthol Blue Black), basic dyes, HC colors, dispersion dyes and direct dyes, lake pigments such as Yellow No. 4 (Tartrazine) AL lake, Yellow No.203 (Quinoline Yellow WS) BA lake; polymers such as nylon powder, silk powder, urethane powder, silicone powder, polymethacrylic acid methyl powder, cellulose powder, starch, silicone elastomer spherical powder, polyethylene powder; colored pigments such as yellow iron oxide, red iron oxide, black iron oxide, chromium oxide, carbon black, ultramarine blue and prussian blue, white pigments such as zinc oxide, titanium oxide and cerium oxide; extender pigments such as talc, mica, sericite, kaolin and planar barium sulfate; pearl pigment such as titanated mica; metal salts such as barium sulfate, calcium carbonate, magnesium carbonate, alminium silicate and magnesium silicate, inorganic powders such as silica and alimina; metal soaps such as alminium stearate, magnesium stearate, zinc palmitate, zinc myristate, magnesium myristate, zinc laurate and zinc undecylenate, bentonite, smectite, boron nitride, etc. The shape (spherical, rod-like, needle-shaped, planar, amorphous, flake-shaped, spindle-shape, etc.) and particle size of these poweders are not particularly limited.

These powders and pigments may preliminarily surface-treated by conventional known surface treatment such as, e.g., fluorine compound treatment, silicone treatment, silicone resin treatment, pendant treatment, silane coupling agent treatment, titanium coupling agent treatment, oil treatment, N-acylate lysine treatment, polyacrylic acid treatment, metallic soap treatment, amino acid treatment, lecithin treatment, inorganic compound treatment, plasma treatment or mechanochemical treatment.

As a surfactant, any of anionic surfactants, cationic surfactants, amphoteric surfactants and nonionic surfactants may appropriately used.

Anionic surfactants include, for example, fatty acid soap, *α*-acyl sulfonate, alkyl sulfonate, alkyl allyl sulfonate, alkyl naphthalene sulfonate, alkyl sulfate, POE alkyl ether sulfate, alkyl amide sulfate, alkyl phosphate salt, POE alkyl phosphate salt, alkyl amide phosphate salt, alkyloyl alkyl taurine salt, N-acylamino acid salt, POE alkylether carboxylate salt, alkyl sulfosuccinate salt, sodium alkylsulfoacetate, acyl isethionate salt, acylated hydrolysed collagen peptide salt, perfluoroalkyl phosphate ester.

Cationic surfactants include, for example, alkyl trimethyl ammonium chloride, stearyl trimethyl ammonium chloride, stearyl trimethyl ammonium bromide, cetostearyl trimethyl ammonium chloride, distearyl dimethyl ammonium chloride, stearyl dimethyl benzyl ammonium chloride, behenyl trimethyl ammonium bromide, benzalkonium chloride, propyldimethylhydroxypropyl ammonium behenate chloride amide, dietylethyl stearate amino amide, dimethylamino propyl stearate amide, lanolin derivative quaternary ammonium salt, etc. Also included are tertiary amine and salts thereof such as fatty acid amide dialkylamine.

Amphoteric surfactants include various amphoteric surfactants of, such as, for example, carboxy betaine type, amide betaine type, sulfobetaine type, hydroxysulfobetaine type, amide sulfobetaine type, phosphobetaine type, amino carbonate salt type, imidazoline derivative type, and amide amine type.

Nonionic surfactants include, for example, propylene glycol fatty acid ester, glycerin fatty acid ester, polyglycerin fatty acid ester, sorbitan fatty acid ester, POE sorbitan fatty acid ester, POE sorbit fatty acid ester, POE glycerin fatty acid ester, POE alkyl ether, POE fatty acid ester, POE hydrogenated castor oil, POE castor oil, POE/POP copolymer, POE/POP alkyl ether, polyether denatured silicone laurate alkanol amide, alkylamine oxide, hydrogenated soy bean phospholipid, hydroxylated soy bean phospholipid, polymeric surfactant, biosurfactant, etc.

Natural surfactants may also be used, and such surfactants include, for example, lecithin, saponin, sugar surfactant, etc.

Polyhydric alcohols and sugers include, for example, ethylene glycol, diethylene glycol, polyethylene glycol, propylene glycol, dipropylene glycol, polypropylene glycol, glycerin, diglycerin, polyglycerin, 3-methyl-1,3-butane diol, 1,3-butylene glycol, sorbitol, mannitol, raffinose, erythritol, glycose, sucrose, fructose, xylitol, lactose, maltose, maltitol, trehalose, alkylated trehalose, mixed isomerized sugar, sulfated trehalose, pullulan, etc. Their chemically modified forms may also be used.

Polymers include, for example, anionic polymer compounds such as acrylate ester /methacrylate ester copolymer, vinyl acetate/crotonate copolymer, vinyl acetate/crotonate/vinyl neodecanoate copolymer, methylvinyl ether maleic acid half ester, T-butyl acrylate/ethyl acrylate/methacrylic acid copolymer, vinyl pyrrolidone/vinyl acetate/vinyl propionate copolymer, vinyl acetate/crotonate copolymer (Luviset CA: BASF), vinylacetate/crotonate/vinylpyrrolidone copolymer, vinylpyrrolidone/acrylate copolymer, acrylate/acrylamide copolymer, vinyl acetate/butyl maleate/isobornyl acrylate copolymer, carboxyvinyl polymer and alkyl acrylate/methacrylate copolymer, or amphoteric acetic acid compounds of aminoethyl dialkyl methacrylate polymer, amphoteric polymer compound such as octyl acrylate acrylamide/hydroxypropyl acrylate/butyl amino ethyl methacrylate copolymer, quaternized compound of vinylpyrrolidone/dimethylamino ethyl methacrylate, cationic polymer compounds such as methylvinyl imidazolium chloride/vinylpyrrolidone copolyme, nonionic polymer compound such as polyvinyl pyrrolidone, vinylpyrrolidone/vinyl acetate copolymer, vinylpyrrolidone/dimethylamino ethyl methacrylate copolymer, and vinyl caprolactam/vinylpyrrolidone/dimethylamino ethyl methacrylate copolymer, etc. Also suitably compounded are polymer compounds of natural origins such as cellulose or derivatives thereof, keratin and collagen or derivatives thereof, calcium alginate, pullulan, agar-agar, gelatine, tamarind seed polysaccharide, xanthan gum, carrageenan, high-methoxyl pectin, low-methoxyl pectin, guar gum, gum arabic, crystalline cellulose, arabinogalactan, karaya gum, tragacanth gum, alginic acid, albumin, casein, curdlan, gellan gum and dextran, and glucooligosaccharide, fucose-containing polysaccharide, and rhamnose-containing polysaccharide.

Physiologically active ingredients include substances which confer skin some physiological activity upon being applied to the skin, e.g., an ingredient having an effect such as whitening, antiinflammatory, antiaging, ultraviolet protection, slimming, tightening, antioxidating, stimulating/growing hair, suppressing hair growth, moisturizing, blood circulation promoting, antibacterial/sterilizing, cool/hot feeling, wound healing promoting, stimulation mitigating, analgesic or cell acivating effects, including plant extract, seaweed extract, vitamins and derivatives thereof, amino acid, various peptides other than the cyclic peptide; biopolymers such as sodium hyaluronate, mucopolysaccharide; intercellular lipid constituents or analogues thereof such as ceramide, phytosphingosine, cholesterol, phytosterol; and enzymatic ingredients. Suitable examples of these combined ingredient include, e.g., *Angelica keiskei* extract, avocado extract, sweet hydrangea leaf;extract, althea extract, arnica extract, aloe extract, apricot extract, apricot kernel extract, isoflavone, *Ginkgo biloba* extract, fennel extract, turmeric extract, oolong tea extract, rose fruit extract, *Echinacea angustifolia* leaf extract, scutellaria root extract, phellodendron bark extract, Coptis japonica extract, 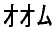 barley extract, *Hypericum erectum* extract, Lamium album extract, Nasturtium officinale extract, orange extract, cacao extract, dried seawater, seaweed extract, hydrolysed elastin, hydrolysed wheat flour, hydrolysed silk, pumpkin seed extract, chamomilla extract, carrot extract, *Artemisia capillaris* extract, licorice extract, *Hibiscus sabdariffa* extract, *Pyracantha fortuneana* extract, kiwi fruit extract, cinchona extract, cucumber extract, guanosine, gardenia extract, *Sasa albo-marginata* extract, *Sophora angustifoliaextract,* cranberry extract, walnut extract, grapefruit extract, clematis extract, chlorella extract, mulberry extract, gentian extract, tea extract, yeast extract, burdock extract, fermented rice bran extract,
rice germ oil, comfrey extract, collagen, bilberry extract, choy sum extract, Bupleurum root extract, umbilical cord extract liquid, salvia extract, *Saponaria officinalis* extract, Sasa-bamboo extract, *Crataegus cuneata* extract, *Zanthoxylum piperitum* extract, *Lentinus edodes* extract, rehmannia root extract, lithospermum root extract, *Perilla frutescens var. crispa* extract, Japanese linden extract, *Filipendula multijuga* extract, *Paeonia lactiflora* extract, calamus root extract, birch extract, *Equisetum arvense* extract, English ivy extract, hawthorn extract, european elder extract, yarrow extract, peppermint extract, sage extract, mallow extract, cnidium rhizome extract, *Swertia japonica* extract, soybean extract, jujube extract, fermented soybean extract, thyme extract, tea extract, clove extract, cogon grass extract, *Citrus unshiu* peel extract, evening primrose extract, *Centella asiatica* extract, *Terminalia sericea* extract, *Angelica acutiloba* extract, *Calendula officinalis* extract, peach kernel extract, spruce tree extract, *Houttuynia cordata* extract, tomate extract, natto (fermented soybean) extract, carrot extract, garlic extract, wild rose extract, hibiscus extract, ophiopogon tuber extract, parsley extract, honey, banana flowe extract, hamamelis extract, *Parietaria judaica* extract, *Isodon japonicus* extract, bisabolol, *Eriobotrya japonica* extract, coltsfoot extract, *Petasites japonicus* extract, *Poria sclerotium* extract, butcher broom extract, grape extract, propolis, sponge gourd extract, safflower extract, peppermint extract, linden extract, *Paeonia suffruticosa* extract, hop extract, pine extract, marronnier extract, *Lysichiton camtschatcense Schot* textract, soapberry extract, melissa extract, peach extract, knapweed extract, eucalyptus extract, saxifrage extract, *Citrus junos* extract, coix seed extract, mugwort extract, lavender extract, apple extract, litchi extract, lettus extract, lemon extract, *Astragalus sinicus* extract, rose extract, rosemary extract, roman chamomile extract, royak jelly extract, etc.

Also included are biopolymers such as deoxyribonucleic acid, mucopolysaccharide, sodium hyaluronate, sodium chondroitin sulfate, collagen, elastin, chitin, chitosan, hydrolysed shell membrane; moisturizing ingredients such as polypeptide including amino acids, hydrolysed peptides, sodium lactate, urea, sodium carbonate pyrrolidone, betaine, whey, trimethyl glycine, lysine/arginine condensate; intercellular lipid constituents or analogues thereof such as sphingolipid, ceramide, phytosphingosine, cholesterol, cholesterol derivative, phytosterol derivative, phospholipid; antiinflammatory agent such as *ε* -amino caproic acid, glycyrrhizic acid, *β*-glycyrrhetic acid, lysozyme chloride, guaiazulene, hydrocortisone, teatree oil; vitamins such as vitamin A and derivatives thereof, vitamin B2 and derivatives thereof, vitamin B6 and derivatives thereof, vitamin C and derivatives thereof, vitamin D and derivatives thereof, vitamin E and derivatives thereof, calcium pantothenate, biotin, nicotinate amide; active ingredients such as allantoin, diisopropylamine dichloroacetate, 4-amino methylcyclohexane carboxylic acid; antioxidant such as tocopherol, carotenoid, flavonoid, tannin, lignan, saponin; cell activating agents such as *α*-hydroxy acid, *β*-hydroxy acid; blood circulation promoting agents such as *γ*-oryzanol, vitamin E derivative; wound healing agents such as retinol, retinol derivative; whitening agents such as arbutin, kojic acid, placenta extract, sulfur, ellagic acid, linoleate, tranexamic acid, glutathione; hair growing agents such as cepharanthine, Glycyrrhiza extract, capsicum tincture, hinokitiol, iodized garlic extract, pyridoxine hydrochloride, DL- *α*-tocopherol, DL- *α*-tocopherol acetate, nicotinic acid, nicotinic acid derivative, calcium pantothenate, D-pantothenyl alcohol, acetylpantothenylethyl ether, biotin, allantoin, isopropylmethyl phenol, estradiol, ethinyl estradiol, carpronium chloride, benzalkonium chloride, diphenhydramine hydrochloride, Takanal, camphor, salicylic acid, nonylic acid vanillyl amide, vanillyl nonanoate amide, piroctone olamine, glycelyl pentadecanoate, L-menthol, mononitroguaiacol, resorcin, *γ-*amino butyric acid, benzethonium chloride, mexiletine hydrochloride, auxin, female hormone, cantharis tincture, cyclosporin, zinc pyrithione, hydrocortisone, minoxidil, polyoxy ethylene sorbitan monostearate, peppermint oil, Sasanishiki extract.

Antioxidants include, for example, sodium bisulfite, sodium sulfite, erythorbic acid, sodium erythorbate, dilauryl thiodipropionate, tocopherol, tolyl biguanide, nordihydroguaiaretic acid, patahydroxy anisole, butylhydroxy anisole, dibutylhydroxy toluene, ascorbyl stearate, ascorbyl palmitate, octyl gallate, propylgallate, carotenoid, flavonoid, tannin, lignan, saponin, and plant extracts that are recognized for antioxidative effect such as apple extract or clove extract.

Solvents include such as physiological saline, purified water, ethanol, lower alcohol, ethers, LPG, fluoro carbon, N-methylpyrrolidone, fluoro alcohol, volatile straight-chain silicone, next generation fluorocarbon.

As mentioned above, the cyclic peptide of the present invention and/or a derivative thereof and/or a pharmaceutically acceptable salt thereof can be used for the manufacture of an external preparation. Therefore, the present invention also relates to use of the cyclic peptide of the present invention and/or a derivative thereof and/or a pharmaceutically acceptable salt thereof for the manufacture of an external preparation.

### 3. Methods of using the external preparation

Next, methods of using the external preparation of the present invention are explained.

A method of using the external preparation of the present invention includes applying above-mentioned external preparation of the present invention to skin and/or mucosa of a subject.

Subjects include, without being particularly limited, e.g., vertebrates such as birds and mammals. Mammals, in particular, include such as, for example, rodents such as mice, rats, gerbils, hamsters and guinea pigs; experimental animals such as rabbits; livestocks such as pigs, cows, horses, sheep and minks; companion animals such as dogs and cats; primates such as human, monkeys, cynomolgus monkeys, rhesus monkeys, marmosets, orangutans and chimpanzees. Among these, human is particularly preferred. On the other hand, it is also possible to exclude human from the subject.

Skin and/or mucosa of the subject to which the external preparation is applied can be skin or mucosa of any sites of the subject. It can be applied to skin or mucosa of, for example, head (scapl), face, neck, arms, torso, arms, hands and feet.

More specific methods of using the external preparation of the present invention are as follows.

### (1) Method of treating and preventing dermatitis

When the external preparation of the present invention is used as a therapeutic or prophylactic for dermatitis, the external preparation can be directly applied to the skin and/or mucosal site of interest (e.g., the affected site where dermatitis has developed).

Application frequency is, without being particularly limited, e.g., from once to 10 times/day, preferably from once to 5 times/day, further preferably from once to 3 times/day. Because the external preparation of the present invention has an effect which lasts for a prolonged duration, it exhibits a sufficient effect even if it was applied in relatively low frequency, for example, once a day. Dose can be, without being particularly limited, e.g., for one applicaiton, from 0.0001 to 1000000 *µ*g/mL, preferably from 0.001 to 10000 *µ*g/mL, more preferably from 0.01 to 1000 *µ*g/mL, further preferably from 0.1 to 100 *µ*g/mL, particularly preferably from to 100 *µ*g/mL as total amount of the cyclic peptide of the present invention and a derivative thereof and a pharmaceutically acceptable salt thereof. In another embodiment, it may be comprised at a concentration from 0.1 to 800 *µ*g/mL, from once to 500 *µ*g/mL.

### (2) Method of alleviating and/or eliminating dermatitis

When the external preparation of the present invention is used as an antipruritic, the external preparation can be directly applied to the skin and/or mucosal site of interest.

Application frequency is, without being particularly limited, e.g., from once to 10 times/day, preferably from once to 5 times/day, further preferably from once to 3 times/day.

Dose can be, without being particularly limited, e.g., for one applicaiton, from 0.0001 to 1000000 *µ*g/mL, preferably from 0.001 to 10000 *µ*g/mL, more preferably from 0.01 to 1000 *µ*g/mL, further preferably from 0.1 to 100 *µ*g/mL, particularly preferably from 1 to 100 *µ*g/mL as total amount of the cyclic peptide of the present invention and a derivative thereof and a pharmaceutically acceptable salt thereof. In another embodiment, it may be comprised at a concentration from 0.1 to 800 *µ*g/mL, from 1 to 500 *µ*g/mL.

### (3) Method of using cosmetics

When the external preparation of the present invention is used as a cosmetic, the external preparation can be directly applied to the skin and/or mucosal site of interest. Application frequency is, without being particularly limited, e.g., from once to 10 times/day, preferably from once to 5 times/day, further preferably from once to 3 times/day.

Dose can be, without being particularly limited, e.g., for one applicaiton, from 0.0001 to 1000000 *µ*g/mL, preferably from 0.001 to 10000 *µ*g/mL, more preferably from 0.01 to 1000 *µ*g/mL, further preferably from 0.1 to 100 *µ*g/mL, particularly preferably from 1 to 100 *µ*g/mL as total amount of the cyclic peptide of the present invention and a derivative thereof and a pharmaceutically acceptable salt thereof. In another embodiment, it may be comprised at a concentration from 0.1 to 800 *µ*g/mL, from 1 to 500 *µ*g/mL.

### (4) Methods of treating and preventing alopecia, stimulating hair growth and growing hair

When the external preparation of the present invention is used as a therapeutic for alopecia, prophylactic for alopecia, hair growth stimulant or hair growing agent and/or a method of inhibiting hair-falling, the external preparation can be directly applied to the site of interest (e.g., scapl, skin site of hair loss).

Application frequency is, without being particularly limited, e.g., from once to 10 times/day, preferably from once to 5 times/day, further preferably from once to 3 times/day.

Dose can be, without being particularly limited, e.g., for one applicaiton, from 0.0001 to 1000000 *µ*g/mL, preferably from 0.001 to 10000 *µ*g/mL, more preferably from 0.01 to 1000 *µ*g/mL, further preferably from 0.1 to 100 *µ*g/mL, particularly preferably from 1 to 100 *µ*g/mL as total amount of the cyclic peptide of the present invention and a derivative thereof and a pharmaceutically acceptable salt thereof. In another embodiment, it may be comprised at a concentration from 0.1 to 800 *µ*g/mL, from 1 to 500 *µ*g/mL.

### (5) Method of treating and preventing rhinitis

When the external preparation of the present invention is used as a therapeutic or prophylactic for rhinitis, the external preparation can be directly applied to the site of interest (e.g., nasal mucosa). Application frequency is, without being particularly limited, e.g., from once to 10 times/day, preferably from once to 5 times/day, further preferably from once to 3 times/day. Dose can be, without being particularly limited, e.g., for one applicaiton for each nasal cavity, preferably from 0.001 to 10000 *µ*g/mL, more preferably from 0.01 to 1000 *µ*g/mL, further preferably from 0.1 to 100 *µ*g/mL, particularly preferably from 1 to 100 *µ*g/mL as total amount of the cyclic peptide of the present invention and a derivative thereof and a pharmaceutically acceptable salt thereof. In another embodiment, it may be comprised at a concentration from 0.1 to 800 *µ*g/mL, from 1 to 500 *µ*g/mL.

### 4. Medicaments

Next, medicaments of the present invention are explained.

A medicament of the present invention comprises one or more cyclic peptides of the present invention or a derivative thereof or a pharmaceutically acceptable salt thereof.

As mentioned above, it is considered that the cyclic peptide of the present invention or a derivative thereof or a pharmaceutically acceptable salt thereof binds, just like CNP, to the receptor NPR-B that has a guanylate cyclase domain and promote cGMP production, thereby having effects such as, for example, diuretic action, vasodilation, suppression of renin/aldosterone secretion, suppression of sympathetic nerves and hypertrophy suppression. These compounds of the invention are considered to have a better drug efficacy/effect, a better immediate effectivity, in particular, as compared to CNP.

Accordingly, a medicament of the present invention can be used for a similar purpose as a medicament comprising conventional CNP as an effective ingredient.

Diseases to which the medicament of the present invention is applicable include, without being particularly limited, such as, e.g, apart from those diseases which are mentioned above, hypertension, unstable angina, acute myocardial infarction, edematous diseases, renal failure, heart failure, immune diseases, obesity and metabolic syndrome.

Dosage forms for the medicament of the present invention are not particularly limited, and can be, apart from those external preparation as mentioned above, such as, e.g., a formulation for oral administration such as tablets, capsules, granules, powders, an oral liquid agent, syrup and oral jelly; formulation for oral application such as a oral tablet, oral spray agent, oral semisolid agent and gargarism; a formulation for administration via injection such as an injection and transfusion; a formulation for dialysis such as dialysis preparation; an inhalant, eye drop, eye ointment, ear drop, vaginal tablet and pessary.

### [Working Examples]

Hereinbelow, the present invention is explained in further details by means of the working examples. It should be appreciated that the present invention is not limited to these working examples.

### 1. Production of cyclic peptides

First, a cyclic peptide consisting of the amino acid sequence expressed by the formula I was synthesized.

In specific, by a method of solid synthesis of peptide using a peptide synthesizer, amino acids were bound in order to form a straight-chain peptide consisting of 17 amino acids. Then the protective groups on the Cys residues in 1st and 17th residues from the N-terminal were detached. Subsequent iodine (I₂) treatment led to the formation of cysteine bond between oxidatively same amino acid residues, resulting in the cyclic peptide.

The composition comprising the obtained cyclic peptide was purified by reverse-phase high performance liquid chromatography (reverse-phase HPLC), and then lyophilized to give the purified product of the cyclic peptide as white powder.

The obtained cyclic peptide was also analyzed by mass spectrometry.

Conditions for HPLC are indicated below.

| | |
|---|---|
| Apparatus: | Agilent 1100 |
| Flow rate: | 1.0 ml/min |
| Eluent A: | 0.1% trifluoroacetic acid/water |
| Eluent B: | 0.1% trifluoroacetic acid/acetonitrile |
| Gradient: | 80% Eluent B (isocratic) |

Conditions for mass spectrometry (MS) conditions are indicated below.

| | |
|---|---|
| Apparatus: | Thermo Finnigan LCQ Advantage |
| Ionization method: | Electrospray ionization |
| Analysis method: | Ion trap method |

The observed results, m/z = 878.41 ([M+2H]²⁺) and m/z = 878.41 ([M+H]⁺), confirmed that the peptide described above was in agreement with theoretical values of the molecular weight (1755.10) and the mass number (1753.8314) calculated from the composition formula of the cyclic peptide of interest (C₇₄H₁₂₃N₂₁O₂₂S₃).

Furthermore, In order to measure the purity of the peptide described above, HPLC measuremetn was carried out under following conditions.

| | |
|---|---|
| Column: | Discovery C18, 4.6mm × 250mm, particle size 5 micrometer |
| Column temperature: | RT |
| Eluent A: | 0.1% trifluoroacetic acid/water |
| Eluent B: | 0.1% trifluoroacetic acid/ acetonitrile |
| Gradient: | 20 to 40% Eluent B/20 min |
| Flow volume: | 1.2ml/min |
| Temperature: | RT |
| Injection volume: | 20 *µ*l |
| Detector: | UV detector (detection at wavelength 215nm) |

The result of the measurement confirmed that the purity of the obtained protein was 99.2%.

### 2. Production of formulations

### 2.1 Production of gel formulations

A gel-based preparation that comprises a cyclic peptide consisting of the amino acid sequence expressed by the formula I (in the following working examples, "C-ring" means the cyclic peptide expressed by the formula I) (C-ring gel formulation, working examples) and a gel-based preparation (CNP gel formulation, comparative examples) that comprises human CNP are produced as follows.

0.1g of parahydroxy benzoate methyl ester (trade name: Mekkins-M, Ueno Fine Chemicals Industry, Ltd.), 0.2 g of phenoxyethanol, and 3.0 g of 1,2-pentanediol were weighed into one same vessel, heated to 60 to 70°C to make a homogenous solution, and this solution was poured into a mixer.

Next, added into the mixer 6.0 g of concentrated glycerin, and then the mixture of 0.44 g of carboxyvinyl polymer (trade name: Carbopol® 940, Lubrizol Advanced Materials Corporation) and 0.08 g of xanthane gum (trade name: KELTROL® T, CP Kelco, Inc.), and the mixuter was stirred with a paddle until they dispersed sufficiently.

Then 83.95 g of purified water was gradually added with stirring with a paddle. The mixer was heated to 70 to 80°C while stirring with a paddle or disper until the dispersed contents were dissolved to give a solution. Subsequently, the disper was stopped and the solution was cooled immediately after confirming that the contents in the solution were dissolved. When the temperature of the mixer reached approximately 40°C, 6.0 g of Lubrajel® NP from Ashland Inc. (glycerin 2.7 g, carboxyvinyl polymer 0.06 g, sodium polyacrylate 0.018 g, water 3.222 g) was added to the solution, mixed uniformly with a paddle. Subsequently, 0.230 g of potassium hydroxide was further added to neutralize the solution, then the rotation of the paddle was stopped when the temperature of the mixer reached 25°C to prepare a gel base.

Next, 1mg of a cyclic peptide (C-ring) consisting of the amino acid sequence expressed by the formula II was dissolved in 1mL of purified water to obtain solution. 17 *µ*l of this solution was admixed with 10 g of the gel base obtained as described above, and the mixture was stirred uniformly to prepare a gel-based formulation (C-ring gel formulation) containing the above-described C-ring at a concentration of about 1 *µ*M (about 1.7 *µ*g/g). Similarly, gel-based formulations containing C-ring at concentrations of about 2.0 *µ*M (about 3.4 *µ*g/g) and about 5.0 *µ*M (about 8.5 *µ*g/g) were prepared. In the working examples below, as long as it is specifically described otherwise, "C-ring gel formulation" used contained C-ring at a concentration of about 1 *µ*M.

Next, 1mg of human CNP-22 (American Peptide Company) was dissolved in 1mL1mL of purified water to obtain CNP solution. 22 *µ*l of this solution was admixed with 10 g of the gel base obtained as described above, and the mixure was stirred uniformly to prepare a gel-based formulation (CNP gel formulation) containing CNP-22 at a concentration of about 1 *µ*M. Similarly, gel-based formulations containing CNP-22 at concentrations of about 2.0 *µ*M and 5.0 *µ*M were prepared. In the working examples below, as long as being specifically described otherwise, "CNP gel formulation" used contained CNP at a concentration of about 1 *µ*M.

### 2.2 Preparation of a nasal drop

A nasal drop comprising a cyclic peptide (C-ring) consisting of the amino acid sequence expressed by the formula II (C-ring nasal drop, working example) and a nasal drop comprising human CNP (CNP nasal drop, comparative example) were prepared as folllows.

First, a cyclic peptide (C-ring) consisting of the amino acid sequence expressed by the formula II is dissolved in physiological saline, the concentration was adjusted to prepare C-ring nasal drop containing C-ring at a concentration of about 1 *µ*mol/l (about 1.7 *µ*g/g).

Next, the C-ring nasal drop was filled in a quantitative nasal spray container (AS ONE Corporation), and the amount ro be sprayed for each administration was adjusted to100 *µ*l (0.1 ml) to provide the C-ring nasal drop.

Similarly, a CNP nasal drop comprising human CNP (American Peptide Company) at a concentration of 1 *µ*mol/l was obtained, which was filled in a quantitative nasal spray container (AS ONE Corporation), and the amount ro be sprayed for each administration was adjusted to 100 *µ*l (0.1 ml), to provide the CNP nasal drop.

### 3. Confirming the therapeutic effect on dermatitis

### 3.1 Confirming the effect of C-ring gel formulation

For subjects suffering various dermatitis, C ring gel formulation as described was applied onto the affected site, and changes in symptoms before and after the application were observed. When possible, as a comparative example, a CNP gel formulation as described was applied onto another affected site of the same subject where the C-ring gel formulation had not been applied, and changes in symptoms before and after the application were observed. For pruritus, VAS (Visual Analogue Scale) was used for evaluating each affected site on 10 stages.

Examination results are given in tables below with age, sexuality and symptoms of the subject and formulation given to the subject.

**[Table 1]**

| Case | Treatment | Disease Name | Pre-application Symptoms (Severity) | Post-application Symptoms (Severity) | Pre-/Post-application Pruritus (VAS) | Notes |
|---|---|---|---|---|---|---|
| D1 | 1 µM | Chronic eczema | Limbs: developed erythema, lichenification, skin desiccation, scratch scars (severe). | Right forearm: No itches or stinging after 2 min on C-ring applied side. Lichenification was remitted after 4 min, no more stiffening. Skin desiccation was improved, skin was moisturized and soft (moderate). | Right arm 10 -> 0 | |
| | C-ring gel formulation | | | | | |
| Age: 30's | 1 Application | | | | | |
| Sex: Male | 1 µM | | | Left forearm: After 4 min, no improvement in itches and pains. Skin felt desiccated and stiff due to no improvement in desiccation, lichenification and infiltration (severe). | Left arm 10-> 10 | |
| | CNP get formulation | | | | | |
| | 1 Application | | | | | |
| D2 | 1 µM | Atopic dermatitis | Developed infiltrative erythema with strong itch, scales, many scratch scars with exudation (severe) shown on both forearms and trunk papules (severe). | Right forearm: Itches were eased immediately after application. No itches after 2 min. Erythema and infiltration were remitted (moderate). Quick wound healing with epithelized scratch scars. Remarkable and immediate remission of erythema as compared to CNP. | 10 -> 0 | |
| | C-ring gel formulation | | | | | |
| Age: 10's | 1 Application | | | | | |
| Sex: Female | 1 µM | | | Left forearm: After 4 min, itches still remain, erythema and infiltration were yet to be remitted (severe). | 10 -> 5 | |
| | CNP gel formulation | | | | | |
| | 1 Application | | | | | |
| D3 | 1µM | Atopic dermatitis | Infiltrative erythema, edema, crust with hot feeling (severe) observed on face. | Right face: Hot feeling was eased immediately after application, infiltration and erythema were remitted and the subject felt ease. After 2 min, anti-inflammation effect was observed that erased hot flash, skin felt cool and soft (moderate). | | |
| | C-ring gel formulation | | | | | |
| Age: 30's | 1 Application | | | | | |
| Sex: Male | 1µM | | | Left face: After 2 min, still felt hot, strong erythema and infiltration being observed (severe). | | |
| | CNP gel formulation | | | | | |
| | 1 Application | | | | | |

**[Table 2]**

| Case | Treatment | Disease Name | Pre-application Symptoms (Severity) | Post-application Symptoms (Severity) | Pre-/Post-application Pruritus (VAS) | Notes |
|---|---|---|---|---|---|---|
| D4 | 1 µM C-ring gel formulation 1 application | Atopic dermatitis | Developed infiltrative erythema, papules, exudation, many scratch scars (severe). | No itches after 3 min. Erythema and infiltration were remitted, scratch scars were epithelized, crust were remarkably improved, with mild erythema being observed (mild). | 10 -> 0 | Intractable Steroid-resistant |
| Age 40's | | | | | | |
| Sex: Male | | | | | | |
| D5 | C-ring gel formulation 1 application | Atopic dermatitis | Scales, many scratch scars, infiltration, erythema and papules with stinging strong itch (VAS5) (severe) developed on face. | Itches vanished in 1 min (VAS 0), erythema and exudation were improved. After 5 min skin rash severity was improved to moderate. One more application at night on the same day kept the subject without itches until 3 days thereafter. Scratch scars were epithelized. | 5 -> 0 | |
| Age: 20's | | | | | | |
| Sex: Male | | | | | | |
| D6 | 1 µM C-ring gel formulation 1 application | Atopic dermatitis | Infiltrative erythema with strong itch, scales, papules, many scratch scars (severe) developed on face. | Right face: Immediately after application, itches and stinging feeling became weak and clear. No itches after 1 min. Erythema and infiltration were remitted (moderate). | 5 -> 0 | |
| Age: 20's | 1 µM CNP gel formulation 1 application | | | Left face: After 3 min, itches still remain, erythema and infiltration were yet to be remitted (severe). | 5 -> 3 | |
| Sex: Male | | | | | | |
| D7 | 1 µM C-ring gel formulation 1 application | Atopic dermatitis | Infiltrative erythema and scales with strong itch developed on face. | Right face: Immediately after application, itches were removed. After 3 min, erythema, infiltration and desiccation were remitted (moderate). | 10 -> 0 | |
| Age: 30's | 1 µM | | | Left face: After 3 min, itches still remain, with erythema, infiltration and scales being observed (severe). | 10 -> 4 | |
| Sex: Male | CNP gel formulation 1 application | | | | | |
| D8 | 1 µM C-ring gel formulation 1 application | Atopic dermatitis | Lichenification, prurigo nodularis, infiltrative erythema and scales with strong itch being observed (severe). | Right forearm: After 3 min, itches were eliminated and the subject felt ease. Lichenification, prurigo nodularis, infiltration erythema, scales and scratch scars were all improved as compared to the left side. Skin was soft and moisturized (moderate). | | |
| Age: 30's | 1 µM CNP gel formulation 1 application | | | Left forearm: After 5 min, strong itch remained, none of lichenification, prurigo nodularis, infiltration erythema, scales and scratch scars was improved (severe). No improvement in lichenification, infiltrative erythema, desiccation or scales. | 10 -> 10 | |
| Sex: Male | | | | | | |

**[Table 3]**

| Case | Treatment | Disease Name | Pre-application Symptoms (Severity) | Post-application Symptoms (Severity) | Pre-/Post-application Pruritus (VAS) | Notes |
|---|---|---|---|---|---|---|
| D9 | Right face: 1 µM C-ring gel formulation 1 application/ day, for 3 days | Atopic dermatitis | Strong itch, infiltrative erythema, scales, exudation, crust (severe). | Right face: Immediately after application, itches were remitted. Edema, erythema and infiltration started to be remitted. After 2 min, anti-inflammation effect was observed that stopped exudation and substantially remitted edema, erythema and infiltration. Opening eyelids became easier. Continuous application for 3 days thereafter kept condition well, causing no itches. | 6 -> 0 | |
| Age: 20's | | | | | | |
| Sex:Male | Left face: 1 µM CNP gel formulation 1 application | | | Left face: Exudation, erythema and infiltration still remained after 3 min. | 6 -> 4 | |
| D10 | Right face: 1 µM C-ring gel formulation 1 application | Atopic dermatitis | Infiltration, erythema and papules all over face, with strong itch and hot flash (severe). | Right face: After 2 min, erythema and infiltration started to be remitted and itches were eliminated. Anti-inflammation effect was observed that erased hot flash (moderate). | 10 -> 0 | |
| Age: 50's | Left face: 1 µM CNP gel formulation 1 application | | | Left face: Itches still remained after 5 min. | 10 -> 3 | |
| Sex: Female | | | | | | |
| D11 | Right face: 1 µM C-ring gel formulation 1 application | Contact dermatitis Atopic dermatitis | Facial edema, erythema and many scratch scars including contact dermatitis with very strong itch as main symptom (severe) (VAS 10). | Right face: Itches eliminated 3 min after application (VAS 0). Scratch scars were epithelized, erythema and infiltration were remitted (moderate). | 10 -> 0 | |
| Age: 40's | | | | | | |
| Sex: Female | Left face: 1 µM CNP gel formulation 1 application | | | Left face: 3 min after application, strong itch remains. No improvement in erythema (severe). | 10 -> 6 | |
| D12 | 1 µM C-ring gel formulation 1 application | Wheals, insect bites | Multiple wheals, erythema and very strong itch developed on both lower limbs. | No itches after 3 min. Wheals were substantially remitted. | 10 -> 0 | |
| Age: 10's | | | | | | |
| Sex: Female | | | | | | |

**[Table 4]**

| Case | Treatment | Disease Name | Pre-application Symptoms (Severity) | Post-application Symptoms (Severity) | Pre-/Post-application Pruritus (VAS) | Notes |
|---|---|---|---|---|---|---|
| D13 | Right forehead: 2 µM C-ring gel formulation 1 application | Contact dermatitis | Skin-eruption from from hair dye, with erythema, infiltration, scratch scars being presented (moderate). Very strong itch being felt (VAS 10). | Right forehead: After 2 min, itches were no longer felt. | 10 -> 0 | |
| | | | | After 3 min, itches were eliminated (VAS 0), the subject felt ease as compared to the left. Erythema and infiltration were both remitted, scratch scars were epithelized (mild). | | |
| Age: 40's | | | | | | |
| Sex: Female | Left forehead: 1 µM CNP gel formulation 1 application | | | Left forehead: 2 min after application, strong itch was present as compared to the right side. 3 min after application, itches remained (moderate). | 10 -> 6 | |
| D14 | Right forearm: 1 µM C-ring gel formulation 1 application | Chronic eczema | Infiltrative erythema, scales, lichenification, crust, papules and skin desiccation with hot feeling being observed (severe). | Right forearm: After 3 min, skin became soft, infiltrative erythema and lichenification were remitted, scales were removed, dry skin was improved and moisturized. Anti-inflammation effect was observed that removed heat and made skin cool as compared to the left side (moderate) | | |
| Age: 30's | | | | | | |
| Sex: Male | Left forearm: 1 µM CNP gel formulation 1 application | | | Left forearm: After 3 min, none of desiccation, lichenification, scales, erythema and infiltration was remitted (severe). | | |
| D15 | Right face: 1 µM C-ring gel formulation 1 application | Eczema | Strong itch, erythema, infiltration, scales and edema being observed around eyes (moderate). | Right face: Immediately after application, edema, erythema were remitted, itches were eliminated (VAS 0) (mild). | 10 -> 0 | |
| Age: 30's | | | | | | |
| Sex: Female | Left face: CNP gel formulation 1 application | | | Left face: Immediately after application, scales, erythema and infiltration remained. Skin texture was rough leaving itches (moderate). | 10 -> 3 | |

**[Table 5]**

| Case | Treatment | Disease Name | Pre-application Symptoms (Severity) | Post-application Symptoms (Severity) | Pre-/Post-application Pruritus (VAS) | Notes |
|---|---|---|---|---|---|---|
| D16 | 2 µM C-ring gel formulation 1 application | Miliaria | Miliaria rubra and flare were presented on forehead with pruritus being felt. | After 2 min, red small papules were remitted, flare became inconspicuous leaving no pruritus. | | |
| Age: 30's | | | | | | |
| Sex: Female | | | | | | |
| D17 | 1 µM C-ring gel formulation 1 application | Miliaria | Miliaria rubra and flare being observed on anterior neck. | After 3 min, red small papules were remitted, flare became inconspicuous. | | |
| Age: 50's | | | | | | |
| Sex: Female | | | | | | |
| D18 | 1 µM C-ring gel formulation 1 application | Chronic urticaria | Multiple wheals with strong pruritus. | Right lower thigh: Immediately after application, pruritus was eliminated. 2 min after application, wheals were remitted. | 10 -> 0 | |
| Age: 30's | 1 µM CNP gel formulation 1 application | | | Left lower thigh: 2 min after application, wheals were yet to be remitted. | | |
| Sex: Female | | | | | | |
| D19 | 1 µM C-ring gel formulation 1 application | Rosacea | Telangiectasia, diffuse flare, follicular papules and pustules developed on cheeks. | Diffuse flare, follicular papules and pustules were remitted. | N/A | |
| Age: 40's Female | | | | Subsequent 2 applications/day for 1 week remarkably improved diffuse flare, follicular papules and pustules changed skin quality which no longer present flare. | | |
| Sex:Female | | | | Anti-inflammation effect was observed that eliminated hot flash/hot feeling. | | |
| D20 | Right perioral: 1 µM C-ring gel formulation 1 application followed by 2 applications/ day continued for 3 | days. Perioral dermatitis | Diffuse flare, follicular papules, red papules developed around mouth. Subjective symptoms include hot feeling. | Right perioral: 2 min after application, diffuse flare and follicular papules were remitted. After 3 days of continuous 2 applications/day further improved reddishness and red papules. | N/A | |
| Age: 40's | | | | | | |
| Sex:Female | Left perioral: 1 µM CNP gel formulation 1 application | | | Left perioral: 2 min after application, no changes in diffuse flare and follicular papules. | | |

**[Table 6]**

| Case | Treatment | Disease Name | Pre-application Symptoms (Severity) | Post-application Symptoms (Severity) | Pre-/Post-appiication Pruritus (VAS) | Notes |
|---|---|---|---|---|---|---|
| D21 | Right cheek: 1 µM C-ring gel formulation 1 application | Rosacea | Telangiectasia, diffuse flare and follicular papules being observed on cheeks. Subjective symptom include hot flash and itches. | Right cheek: C-ring application: After 1 min, hot flash, itch was cooled down. After 2 min, diffuse flare and follicular papules were remarkably remitted. | | |
| Age: 30's | | | | | | |
| Sex: Male | Left cheek: 1 µM CNP gel formulation 1 application | | | Left cheek: CNP application: Even after 3 min, hot flash, itches and flare still remained. | | |
| D22 | Right cheek: 1 µM C-ring gel formulation 1 application | Rosacea | After 1 month application of steroid ointment, hot flash, itch, telangiectasia, diffuse flare and follicular papules emerged on cheeks. | Right cheek: C-ring application: After 2 min, hot flash, itch were cooled down. After 2 min, diffuse flare and follicular papules were remarkably remitted. | | |
| Age: 30's | | | | | | |
| Sex: Female | Left cheek: 1 µM CNP gel formulation 1 application | | | Left cheek: CNP application: Even after 4 min, hot flash, itches and flare still remained. | | |
| D23 | Right cheek: 1 µM C-ring gel formulation once/day for 1 week | Acne vulgaris | Multiple pinpoint follicular papules and pustules on both cheeks. Oily skin. | 2 min after application, anti-inflammation effect was observed, pustules and papules were reduced, and reddishness started to disappear. After 1 week application, both pustules and papules were substantially remitted, reddishness was further remitted. | | |
| Age: 30's | | | | | | |
| Sex: Female | Left cheek: 1 µM CNP gel agent single application | | | 3 min after application, no changes in symptoms. | | |
| D24 | 1 µM C-ring gel formulation 1 application | Acne vulgaris | Multiple comedos, pinpoint follicular papules/pustules on forehead. | 3 min after application, comedos became inconspicuous, oily skin was improved and skin texture was improved. Red papules/pustules were reduced. | | |
| Age: 30's | | | | | | |
| Age: Female | | | | | | |
| D25 | 5 µM C-ring gel formulation 1 application | Acne vulgaris | Multiple comedos, pinpoint follicular red papules/pustules on forehead and jaw. | 3 min after application, comedos became inconspicuous, oily skin was improved and skin texture was improved. Red papules/pustules were reduced and dried. | | |
| Age: 20's | | | | | | |
| Age: Female | | | | | | |

As shown in Tables above, in all subjects, and in all dermatitis of any symptoms, remission or elimination of various symptoms of dermatitis was observedat the site where the C-ring gel formulation was applied.

Specifically, when the C-ring gel formulation was applied, remission or elimination of pruritus was observed immediately after the application. Generally in dermatitis, patients tend to scratch where pruritus was felt, potentially causing severer dermatitis. Such a remarkable remission or elimination of pruritus in this manner can consequently prevent aggravation of dermatitis.

In all cases, the effects of the C-ring gel formulation were superior to those with CNP gel formulation. Specifically, the C-ring gel formulation had faster-acting and longer-lasting effects as well as more potent antipruritic effect as compared to CNP gel formulation. It was confirmed that these effect cannot be obtained by the gel base.

### 3.2 Comparing the C-ring gel formulation to gel base by two-sided application

For subjects suffering various dermatitis, either the C-ring gel formulation or the gel base was applied to the affected site, and changes in symptoms before and after the application were observed. The results are summarized in the tables below.

**[Table 7]**

| Case | Applied site | Treatment | Disease Name | Pre-application Symptoms | Post-application Symptoms |
|---|---|---|---|---|---|
| E1 | Right face | C-ring | Atopic dermatitis | Multiple infiltrative erythema, scales and scratch scars on face with desiccation. | 3 min after application, infiltrative erythema, scales and desiccation were improved, scratch scars were epithelized and became inconspicuous, with no itches left. |
| Age: 20's | | | | | |
| Sex: Male | Left face | Gel base | | | After 3 min, none of infiltration erythema, scales and desiccation was remitted, with itches left over. |
| E2 | Right | Right forearm: 1 µM C-ring | Chronic eczema | Infiltrative erythema, scales, desiccation and scratch scars being observed. | Right forearm: 2 min after application, itches were removed. 4 min after application, infiltration, erythema and scales were remitted, and islets of normal skin were observed. Scratch scars were epithelized and dried. |
| Age: 30's | | | | | |
| Sex: Male | Left | Left forearm: Gel base | | | Left forearm: 4 min after application, stinging itch remained with no improvement. |
| E3 | Right face | C-ring | Atopic dermatitis | Erythema, infiltration and edema being observed. | Immediately after application, erythema and edema started to be improved. 3 min after application, erythema, infiltration and edema were remarkably remitted. |
| Age: 20's | | | | | |
| Sex: Female | Left face | Gel base | | | 3 min after application, none of edema, erythema and infiltration was remitted. |
| E4 | Right | C-ring | Chronic eczema | Lichenification, infiltrative erythema, scales, hot feeling being observed. | Immediately after application, reddishness was remitted. 3 min after application, reddishness was remarkably remitted, lichenification and infiltration was improved, and skin was softened, Anti-inflammation effect was observed that removed hot feeling and made skin cool. |
| Age: 30's | | | | | |
| Sex: Male | Left | Gel base | | | 3 min after application, there still was infiltration, skin was hard showing unimproved reddishness and felt hot. |

### 3.3 Confirming the effect of the C-ring gel formulation on psoriasis

Followings are the summary of the therapeutic effects for psoriasis cases. As described above, the C-ring gel formulation was applied to the affected site of psoriasis, and changes in symptoms before and after the application were observed. When possible, as a comparative example, the CNP gel formulation described above was applied onto another affected site of the same subject where the C-ring gel formulation had not been applied, and changes in symptoms before and after the application were observed. Examination result is given in tables below with subjects' age, sex and symptoms, and prescription for the subjects.

**[Table 8]**

| Case | Treatment | Disease Name | Pre-application Symptoms | Post-application Symptoms | Notes |
|---|---|---|---|---|---|
| | Right lower thigh: | Parapsoriasis guttata | Multiple small erythema with small white scales almost all over the body. | Right lower thigh: After 3 min, no changes in scales and infiltration. Even after subsequent 1 application/day for 1 week, scales and infiltration still remained. | |
| | 1 µM CNP gel formulation | | | | |
| | 1 application | | | | |
| P1 | followed by 1 application/day for 1 week | | | | |
| 50's Age: | Left lower thigh: | | | Left lower thigh: After 3 min, scales and infiltration were remitted. Subsequent 1 application/day for 1 week further remitted scales and infiltration. | |
| Sex: Female | 1 µM C-ring gel formulation | | | | |
| | 1 application | | | | |
| | followed by 1 application/day for 1 week | | | | |
| P2 | 1 µM | Psoriasis vulgaris | Multiple red aspect with attached scales having clear edges on both lower limbs. | Upon observation at 3 min after application, scales, infiltration and erythema were remarkably improved, softly flattened, 6 min after application, scales were completely eliminated. | Activated Vitamin D₃ ointment application remitted none of scales, infiltration and erythema after 3 min. |
| Age: 40's | C-ring gel formulation | | | | |
| Sex: Male | twice/day for 1 week | | | | |
| P3 | 1 µM | Psoriasis vulgaris | Multiple red aspect with attached thick scales having clear edges on lower thighs. | Scales once being thick before application were almost eliminated 3 min after application. After 6 min, infiltration and scales were both remarkably improved. | |
| Age: 40's | C-ring gel formulation | | | | |
| Sex: Male | 1 application | | | | |
| | 1 µM | Psoriasis vulgaris | Multiple red aspect with attached scales having clear edges on both lower limbs. | Upon observation at 3 min after C-ring application, scales, infiltration and erythema were remarkably improved, softly flattened. 6 min after application, scales were almost eliminated. 3 days thereafter, erythema was also remitted. | |
| P4 | C-ring gel formulation | | | | |
| Age: 60's | 1 application | | | | |
| Sex: Female | followed by 2 applications/day for 1 week | | | | |
| P5 | 1 µM | Psoriasis vulgaris | Multiple red aspect with attached thick scales having clear edges on lower thighs. | Even at 9 min after application, infiltration and scales were not improved. | |
| Age: 20's | CNP gel formulation | | | | |
| Sex: Male | 1 application | | | | |

**[Table 9]**

| Case | Treatment | Disease Name | Pre-application Symptoms | Post-application Symptoms | Notes |
|---|---|---|---|---|---|
| | Right lower thigh: | Psoriasis vulgaris | Multiple red aspect accompanied by scales with clear edge on both lower limbs. | Right lower thigh: Scales once being thick before application were almost eliminated at 3 min after application. After 6 min, infiltration and scales were both remarkably improved. Subsequent continuous 2 applications/day for 1 week almost eliminated scales after 3 days. Infiltration and erythema not relapse | |
| | 1 µM C-ring gel formulation | | | | |
| P6 | 1 application | | | | |
| Age: 60's | followed by 2 applications/ day for 1 week | | | | |
| Sex: Female | Left lower thigh: 1 µM | | | Left lower thigh: At CNP applied side, scales, infiltration and erythema were not improved upon observation after 3 min. Continuous 2 applications/day for 1 week made scales inconspicuous, but erythema and infiltration were improved only a little. | |
| | CNP gel formulation | | | | |
| | 1 application | | | | |
| | followed by 2 applications/ day for 1 week | | | | |
| P7 | 1 µM | Psoriasis vulgaris | Multiple red aspect with attached thick scales having clear edges on lower thighs. | Even at 9 min after application, neither infiltration nor scales was improved. | |
| Age: 20's | CNP gel formulation | | | | |
| Sex: Male | 1 application | | | | |
| | Right olecranon, | Psoriasis vulgaris | Red aspect with attached thick scales having clear edges accumulated on olecranon and patella. | Right olecranon and right patella: At C-ring applied side, scales once being thick before application were almost eliminated at 3 min after application. After 6 min, infiltration and scales were both remarkably improved. | |
| | Right patella: 5 µM | | | | |
| P8 | C-ring gel formulation | | | | |
| Age: 40's | 1 application | | | | |
| Sex: Female | Left olecranon, | | | Left olecranonand left patella: At CNP applied side, scales, neither infiltration nor erythema was improved upon observation after 3 min. Continuous 2 applications/ day for 1 week allowed scales to start becoming inconspicuous, though erythema and infiltration were yet to be improved. | |
| | Left patella: 5 µM | | | | |
| | CNP gel formulation | | | | |

**[Table 10]**

| Case | Treatment | Disease Name | Pre-application Symptoms (Severity) | Post-application Symptoms (Severity) | Notes |
|---|---|---|---|---|---|
| | Right forearm: | Parapsoriasis guttata | Multiple small erythema with tumefactive white scales having clear edges on forearms. Left forearm presents severer symptom than right forearm. | Right forearm: At CNP applied side, upon observation after 3 min, scales, infiltration and erythema were not improved. | Thick scales, erythema being observed on scalp along hairline. C-ring application almost eliminated both scales and erythema and achieved normalization after 3 min. |
| | 5 µM CNP gel formulation | | | | |
| | 1 application | | | | |
| P9 | followed by 2 applications/ day for 1 week | | | | |
| Age: 20's | Left forearm: | | | Left forearm: At C-ring applied side, flattening was observed after 3 min, reddishness was remitted and scales were eliminated. | |
| Sex: Male | 5 µM C-ring gel formulation | | | | |
| | 1 application | | | | |
| | followed by 2 applications/ day for 1 week | | | | |

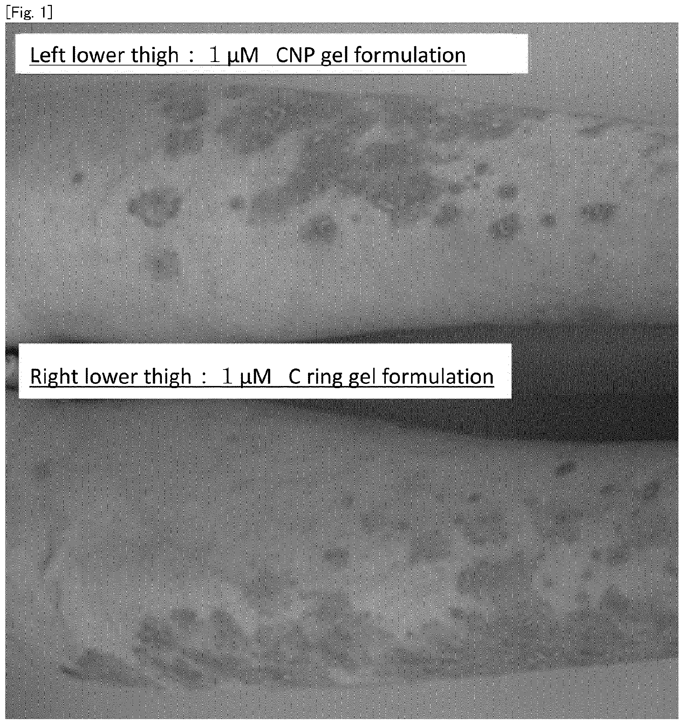

Also, on each of the lower thighs of the patient who has developed psoriasis (P6),either C-ring or CNP gel formulation was applied (two-sided application) and the results after 3 days from the application are shown in Fig. 1. It was observed that scales were almost eliminated and reddish appearance was suppressed on the right lower thigh to which C-ring was applied. However, on the left lower thigh to which CNP was applied, scales became inconspicuous but still remains, and erythema and infiltration were improved only a little, and the obtained effects were not as much those of C-ring.

It is recognized that C-ring has a quicker effect on psoriasis than conventional steroid topical drugs or vitamin D₃ topical drugs, needing less effort for enforcement as compared to biological formulations, while providing results of improving therapeutic satisfaction and QOL. It is also recognized that C-ring has a more remarkable therapeutic effect and a drug efficacy at a lower concentration as compared to CNP. Surprisingly it has a high therapeutic effect on a thick scales which it is difficult to be sufficiently treated with CNP, showing an immediate effects to give remission within several minutes and an amelioration maintaining effect thereafter. In psoriasis, systemic and multiple erythema is accompanied by thick scales, which fall off to cause problems in appearance and impairs QOL. Topical therapy with C-ring may improve QOL of psoriasis patients early. Psoriasis is one of chronic imflammatory dermal diseases and intractable. Currently, choices of therapeutic drugs for psoriasis include topical therapies with steroid topical drugs and vitamin D₃ topical drugs, and therapies with ultraviolet irradiation, cyclosporine and etretinate, and in recent years biological formulations which have been used for cases of moderate diseases or worse. However, while a biological formulation has been evaluated for its drug efficacy, its expensive cost burden, concerns for side effects during use for a prolongled period and necessary effort for enforcement by subcutaneous injection have also been pointed out. There are few cases which is ameliorated solely by a topical therapy with vitamin D₃, often being difficult to avoid continuing combined steroid use. However, a long-term topical application of steroid often causes skin atrophy and adrenal insufficiency. Moreover, the risk of hypercalcemia needs to be noted when vitamin D₃ is used in a subject who shows enhanced absorption of a topical drug becaused of thinned skin due to a long-term topical application of steroid and a use of etretinate, or in a case with renal impairment. For these, the C-ring of the present invention not only exhibit a better effect as compared to conventional therapeutic methods as described, but also resolves the concern as described above.

### 4. Confirming the effect as a cosmetic

The effects of a cosmetic comprising the C-ring is evaluated for gel, bathing agent and hair cosmetic product (shanpoo, hair rinse) or body soap and comprising C-ring were evaluated and the results are confirmed.

### 4.1.1 Confirming the effect of improving skin quality (comparison of the C-ring gel formulation to the CNP gel formulation)

The C-ring gel formulation and CNP gel formulation as described above were prescribed to the subjects. For each subject, the C-ring gel formulation was applied to the right cheek and around right eye, the CNP gel formulation to the left, and changes in skin conditions were observed and compared. Cases and their evaluations are summarized in the following tables. Changes in skin condition were judged based on cenesthesic evaluation by the subject and objective observation by the inventor as a physician.

**[Table 11]**

| Case | Method/site of application | Pre-application Symptoms | Post-application Symptoms |
|---|---|---|---|
| | Right: | Crow's feet, flabbiness, pigmentation of outer corners of eyes, and dullness. | 3 min after application, wrinkles on outer and inner corners of eyes were shallowed, skin became full, flabbiness and skin texture were improved, pigmentation of outer corners of eyes and dullness were improved and became inconspicuous. |
| F1 | C-ring gel formulation 1 µM | | |
| Age: 40's | 1 application | | |
| Sex: Female | Left: | | |
| | CNP gel formulation 1 µM1 | | |
| | application | | 3 min after application, no improvement in wrinkles. |
| | Right: | Ripples, flabbiness and desiccation being observed. | 3 min after application, skin became full and wrinkles became inconspicuous, skin texture was improved, moisturized and softened. |
| F2 | C-ring gel formulation 1 µM | | |
| Age: 40's | 1 application | | |
| Sex: Female | Left: | | |
| | CNP gel formulation 1 µM | | 3 min after application, no improvement in wrinkles. |
| | 1 application | | |
| | Right: | Desiccation, mild erythema, stiffness and roughness being observed with stinging feeling. | Right: At C-ring applied side, 2 min after application, dry skin was improved, moisturized and softened, with no more stinging feeling. |
| | C-ring gel formulation 1 µM | | |
| F3 | 1 application | | |
| Age: 40's | | | |
| Sex: Female | Left: CNP gel formulation being | | Left: At CNP gel formulation applied side, even at 4 min after application, no improvement observed in reddishness and stinging feeling. |
| | applied | | |
| | Right: | Dry and stinging. | 1 min after application, no more stinging. Dry skin was improved and moisturized, skin texture was improved. Skin was flexible, soft and moisturized. |
| | C-ring gel formulation 0.5 µM | | |
| F4 | 1 application | | |
| Age: 30's | Left: | | |
| Sex: Female | CNP gel formulation 0.5 µM1 | | 3 min after application, stinging with no improvement in desiccation and reddishness being observed. |
| | application | | |
| | Right: | Crow's feet, flabbiness, and desiccation. | 2 min after application, crow's feet became inconspicuous, dry skin was improved, given resilience and moisture and became flexible. |
| | C-ring gel formulation 1 µM | | |
| F5 | 1 application | | |
| Age: 30's | | | |
| Sex: Female | Left: CNP gel formulation 1 µM | | Left side: At CNP gel applied site, 5 min after application, no improvement in desiccation, wrinkles and flabbiness being observed. |
| | 1 application | | |

**[Table 12]**

| Case | Method/site of application | Pre-application Symptoms | Post-application Symptoms |
|---|---|---|---|
| | Right: C-ring gel formulation2 µM | Skin desiccation, oily skin and red papules around nose, and mild erythema being observed, with rough skin texture. | 1 min after application, itches were remarkably remitted, After 3 min, skin desiccation and reddishness were improved, and skin was moisturized and made flexible, and skin texture was improved. Red papules around nose were almost eliminated, mixed condition of dry and oily skin was improved, and skin was kept clean. |
| F6 | 1 application | | |
| Age: 30 | Left: CNP gel formulation | | |
| Sex: Female | 2 µM | | |
| | | | 3 min after application, desiccation, reddishness and itches remained, and seborrhea and red papules around nose were not remitted. |
| | 1 application | | |
| F7 | Right: C-ring gel formulation 1 | Desiccation and mild erythema being observed, with stinging feeling. | Immediately after application, no more stinging feeling. 2 min after application, erythema was remitted. |
| Age: 20 | 1 application | | |
| Sex: Female | Left: CNP gel formulation ormuaion 1 µM | | 2 min after application, stinging feeling were left, erythema did not disappear. |
| | 1 application | | |
| F8 | Right: C-ring gel formulation 1 µM | Oily skin and mild erythema around nose being observed, with open pores, rough skin texture, and stinging feeling. | 3 min after application, erythema was remitted, oily skin was improved, with no more stinging feeling. Skin texture was refined and pores became inconspicuous. |
| Age: 60 | 1 application | | |
| Sex: Female | Left: CNP gel formulation 1 µM | | 3 min after application, stinging feeling, skin was left oily, and erythema did not disappear. |
| | 1 application | | |
| F9 | Right: C-ring gel formulation 1 µM | Skin desiccation, dullness, wrinkles, ripples and flabbiness being observed. | Immediately after application, skin was provided with resilience, flabbiness was improved, dullness became inconspicuous. Next day, large wrinkles and ripples were shallowed showing resilience. |
| Age: 60 | 1 application/day | | |
| Sex: Female | Left: CNP gel formulation 1 µM | | Immediately after application, neither desiccation nor flabbiness was improved. Next day, large wrinkles and ripples were less deeply improved than C-ring applied site. |
| | 1 application | | |

Examples in which the C-ring gel formulation was applied without discriminating between right and left sides are summarized in the followings. Changes in skin conditions were by a similar method to the above.

**[Table 13]**

| Case | Method of application | Pre-application Symptoms | Post-application Symptoms |
|---|---|---|---|
| G1 | C-ring gel formulation | Open pores, desiccation, rough skin texture, and hard skin. Felt stiff. | 2 min after C-ring application, stiffness was suppressed, skin texture was improved, and skin was moisturized and softened. |
| Age: 40 | 1 µmol | | |
| Sex: Female | 1 application | | |
| G2 | C-ring gel formulation | Wrinkles and desiccation on forehead and outer corners of eyes being observed. | At C-ring applied site, 3 min after application, wrinkles on forehead and outer corners of eyes were shallowed and became inconspicuous, dry skin was improved, moisturized and made flexible. |
| Age: 20's | 2 µmol | | |
| Sex: Female | 1 application | | |
| | C-ring gel formulation | Desiccation and many small scars due to razor rash on jaw. | 2 min after application, desiccation was remitted, scars vanished , itches were eliminated. 3 min after application, reddishness and desiccation were remitted. No more razor rash after 1 application/day for 3 days. |
| G3 | 2 µmol | | |
| Age: 20's | 1 application | | |
| Sex: Male | followed by 1 application/ day for 3 days | | |
| G4 | C-ring gel formulation | Miliaria, reddishness and pruritus being observed. | At C-ring applied site, 3 min after application, miliaria was prevented, reddishness and itches were remitted. |
| Age: 50's | 1 µmol | | |
| Sex: Female | 1 application | | |
| G5 | C-ring gel formulation | Multiple miliaria rubra on forehead. Oily skin. | 3 min after application, miliaria, reddishness, oily skin, itches were remitted. |
| Age: 30's | 1 µmol | | |
| Sex: Female | 1 application | | |

As above, it was shown that the C-ring of the present invention has a moisturizing effect on skin while improving skin texture, and is further effective in improvement of skin quality such as whitening, spots, dullness, antiaging (flabbiness, resilience, wrinkles) as well in improving mucosal conditions such as rough lips.

In all subjects, a significant effect of improving and maintaining skin quality were recognized at the site to which the C-ring gel formulation has been applied as compared to the site to which the CNP gel formulation was applied. In specific, remarkable effects were recognized at the site to which the C-ring gel formulation has been applied as compared to the site to which the CNP gel formulation has been applied, such as fixing skin, moisturizing skin, improving skintexture, providing skin with resilience and firmness, softening skin, suppressing skin desiccation, making ripples inconspicuous, supplying and retaining water and oil to skin, shallowing crow's feet and making them inconspicuous, eliminating skin rawness, removing and suppressing itches, relieving skin roughness, maintaining skin in a healthy condition

As a result of including subjects of varying ages from 20s to 60s, it was shown that the effects such as improving/erasing wrinkles, improving cheek flabbiness and supplying and retaining moisture, water and oil were exhibited, irrespective of age. Also exhibited were effects of giving firmness and tightness to skin, lifting cheeks, eliminating skin rawness and relieving skin roughness. These effects were recognized more remarkably in the site to which the C-ring gel formulation has been applied as compared to the CNP gel formulation, and the effect was greater as compared to the site to which the CNP gel has been applied.

Furthermore, in the subjects in their 40s to 60s, remarkable effects were observed such as that flabbiness, wrinkles and desiccation were improved quickly after the application of the C-ring gel formulation, the skin was given firmness and dullness, spots, flabbiness and dark circles were made inconspicuous. These effects were greater than those by the CNP gel application.

The effects expressed remarkably quicker by the C-ring gel formulation as compared to the CNP gel formulation. In most cases, effects were confirmed such as that wrinkles started to dissapear and became inconspicuous approximately 2 minutes after the application of the C-ring gel formulation, skin became full with resilience. These effects lasted at least several hours thereafter. No irritating symptoms accompanied the application of the C-ring gel formulation.

### 4.1.2 Confirming the effect of improving skin quality (comparison of the C-ring gel formulation to the gel base)

In order to examine whether the gel base contributes to the effect by the C-ring gel formulation and the CNP gel formulation, in one subject, the C-ring gel formulation was applied to the right face and the gel base to the left, then changes in skin conditions on the face of the subject were observed.

**[Table 14]**

| Case | Method/site of application | Pre-application Symptoms | Post-application Symptoms |
|---|---|---|---|
| | Right: C-ring gel | Rough skin texture, desiccation with stinging feeling, crow's feet. | At C-ring gel formulation applied site, 2 min after application, skin was soft and moisturized, with no stinging feeling. Skin texture was improved, and crow's feet became inconspicuous. |
| | formulation | | |
| H1 | 1 µmol | | |
| Age: 30 | 1 application | | |
| Sex: Female | Left: Gel base | | At gel base applied site, 2 min after application, still felt stinging, with no improvement in crow's feet. |
| | 1 µmol | | |
| | 1 application | | |

At the gel base-applied site, feelings of skin desiccation and rawness were not improved. On the other hand, at the site to which the C-ring gel formulation has been applied, skin was soft and moisturized 2 minutes after the application, with improved feeling of rawness. Skin texture was also improved and ripples became inconspicuous.

### 4.2 Observation of mucosal condition to which the C-ring gel formulation has been applied

To normal subjects who have symptoms of lip roughness, the C-ring gel formulation was applied and changes in lip mucosal conditions were observed and summarized in the following table. Changes in the mucosal condition were judged based on based on cenesthesic evaluation by the subject and objective observation by the inventor as a physician.

**[Table 15]**

| Case | Method/site of application | Pre-application Symptoms | Post-application Symptoms |
|---|---|---|---|
| I1 | C-ring 1 µM | Cracks on corner of mouth, dry lips, with deepened wrinkles. | After 2 min, cracks on corner of mouth were epithelized, shallowed and remitted. Lips were softened and made full, and wrinkles became inconspicuous. |
| Age: 30's Sex: Female | 1 application | | |
| | Right lips: | Cracks on corner of mouth, dry lips, with stinging feeling. | Right lips: |
| | C-ring | | 2 min after application, cracks on corner of mouth were shallowed, desiccation was improved, lips were softened and no longer stinging. |
| I2 | 1 µM | | |
| Age: 40's | 1 application | | |
| Sex: Female | Left lips: | | Left lips: |
| | CNP | | 2 min after application, cracks and stinging feeling remained on corner of mouth. |
| | 1 µM | | |
| | 1 application | | |
| I3 | C-ring 1 µM | Desiccation of lips being concerned. | After 2 min, lips were full and soft, wrinkles became inconspicuous and moistened. 1 application kept normal condition for 1 week without desiccation. Scales became inconspicuous. |
| Age: 60's | 1 application | | |
| Sex: Female | | | |
| I4Age: 30 | C-ring 1 µM | Desiccation, wrinkles and scales on lips. | After 3 min, lips were full and soft, wrinkles became inconspicuous and moistened. |
| Sex: Female | 1 application | | |
| I5Age: 20's | C-ring 1 µM | Desiccation, deep wrinkles and itches on lips. | 3 min after application, itches were removed, lips were full, and wrinkles were stretched. |
| Sex: Male | 1 application | | |

As above, it was confirmed that the cyclic peptide of the present invention has an effect of improving mucosal condition and immediate effectiveness in terms of the action as described above.

### 4.3 Observation of the condition of scalp/hair to which C-ring gel formulation has been applied

To normal subjects who have symptoms of scalp/hair, the C-ring gel formulation was applied was applied, and changes in conditions of scalp and hair were observed and summarized in the following table¥. Changes in conditions of hair of head/hair were judged based on based on cenesthesic evaluation by the subject and objective observation by the inventor as a physician.

**[Table 16]**

| Case | Method of application | Pre-application Symptoms | Post-application Symptoms |
|---|---|---|---|
| J1 | | Scalp erythema, dandruffs and itches being observed. | Immediately after application, itches were removed, scalp were relieved from roughness/ desiccation, and moistened. |
| Age: 50's | C-ring 1 µM | | |
| Sex: Male | 1 application | | |
| J2 | C-ring 1 µM | Scalp erythema, itches, thinning and falling of hair being observed. | Falling hairs were reduced, itches and erythema were improved, growing, stimulating and nourishing of hair were recognized. Hair body was increased, improving thinning of hair. Hair became flexible and glossy. |
| Age: 60's | 1 application/day for 1 week | | |
| Sex: Female | | | |
| J3 | C-ring 1 µM | Large deal of hair thinning and falling in frontal part, parietal part. Hair was thin and glossless. | Falling hairs were reduced, promoting, growing, stimulating and nourishing of hair were recognized. Hair body was increased, starting to improve thinning of hair. Hair became moist, elastic and glossy. |
| Age: 40's | 1 application/day for 7 days | | |
| Sex: Female | | | |
| J4 | C-ring 1 µM | Dandruffs, itches and seborrheic scalp condition. | Immediately after application, itches were terminated, scalp seborrhea and dandruffs were improved. |
| Age: 60's | | | |
| Sex: Male | 1 application | | |
| J5 | C-ring 1 µM | Desiccation, itches, dandruffs, seborrhea, being observed. | Right C-ring applied side: After 1 min, itches were terminated. After 3 min, scalp desiccation, dandruffs and seborrhea started to be improved and the subject felt refreshing. |
| Age: 30's | 1 application | | |
| Sex: Male | CNP gel formulation 1 application | | Left CNP gel formulation applied side: 3 min after application, itches remained. |
| J6 | C-ring 2 µM | Dandruffs, itches and desiccation. | Immediately after application, itch were terminated, and scalp desiccation was improved after 2 min. Then dandruffs were removed, scalp became healthy and clean. |
| Age: 30's | | | |
| Sex: Female | 1 application | | |

As above, the C-ring of the present invention provides and retain humidity to scalp and hair, while being capable of supplying and retaining adequate water and oil, improving and preventing desiccation, improving seborrhea and cleaning scalp and hair. Moreover, it has been shown that it is effective in suppressing itchiness of and dandruffs, and also effective in preventing thinning and loss of hair, growing hair, inhibiting and preventing hair falling, promoting, stimulating and forcing hair growth, as well as in improving hair loss and hair growth after illuness or childbirth.

### 4.4 Observation of skin condition upon using bathing agent comprising the C-ring

Subjects were given bath in 37 to 41 °C hot water comprising C-ring dissolved at 0.01 *µ* M (100 ml of 20 *µ* M C-ring solution dissolved in 200 L hot water) once a day for 14 days, and skin condition of the subjects was compared to the case of hot water alone. Cases and evaluations are summarized in the following table. The changes in skin conditions were judged based on based on cenesthesic evaluation by the subject and objective observation by the inventor as a physician.

**[Table 17]**

| Case | Treatment | Symptoms before use (miliaria, cracks, chaps, eczema) | After use | Subjective changes in symptoms |
|---|---|---|---|---|
| K1 Age: 60's Sex: Male | C-ring addition | Eczema, skin desiccation | Eczema and skin desiccation were gradually remitted. | Itches after bathing were remitted. Skin was moisturized, |
| K2 Age: 60's Sex: Male | hot water | Eczema, skin desiccation | Reddishness and desiccation exacerbated. | Itches exacerbates and skin becomes desiccated, especially after bathing. |
| K3 Age: 60's Sex:Female | CNP addition | Eczema, skin desiccation | No remission in skin desiccation and erythema being observed. | No remission in skin desiccation was recognized at this concentration, |
| K4 Age: 30's Sex: Male | C-ring addition | Chaps, rough hands, pains and itches. | Desiccation was remitted. Chaps were improved. | Itches were relieved. Skin was moisturized. |
| K5 Age: 30's Sex: Female | hot water | Chaps, rough hands, pains and itches. | Desiccation exacerbated. | After bathing, pains and itches exacerbated. |
| K6 Age: 20's Sex: Male | C-ring addition | Miliaria. Itches. | Reddishness, miliaria were remitted. | Itches and reddishness were remitted. |
| K7 Age: 20's Sex: Male | hot water | Miliaria. Itches. | No improvement in miliaria. | No improvement in itches. |

As above, the bathing agent comprising the C-ring of the present invention has not only effects of remitting miliaria, cracked or chapped skin but also effects of improving eczema. Furthermore, it was shown to improve desiccation, pain and itch of the skin, indicating effectiveness for improving skin condition.

### 4.5 Observation of scalp and hair conditions or skin condition upon using a hair cosmetic (shanpoo, hair rinse) or body soap comprising the C-ring

### 4.5.1 A shanpoo comprising the C-ring was mixed and prepared in composition as follows.

**[Table 18]**

| | Names of ingredients (tradenames) | Content (%) |
|---|---|---|
| A | Sodium cocoylmethyltaurine solution (NIKKOL CMT-30) | 10.0 |
| | Sodium laureth lactate solution (NIKKOL SBL-2N-27) | 20.0 |
| | Lauryl betaine solution (NIKKOL AM-301) | 10.0 |
| | Cocamide DEA | 4.0 |
| | Antiseptic agent | q.s. |
| B | Citric acid | 0.1 |
| | Propylene glycol | 2.0 |
| | Guar hydroxypropyl trimonium chloride | 0.5 |
| | Fill up with water | 100.0 |
| C | Water, C-ring (C-ring concentration 1 µM) | 1 mL |

A and B were dissolved with heat at 70 °C. To A, B was added, stirred and mixed. C was added at 40 to 35 °C while being further stirred, and allowed to cool to room temperature as kept being stirred.

### 4.5.2 A treatment comprising the C-ring was mixed and prepared in composition as follows.

**[Table 19]**

| | Names of ingredients (tradenames) | Content (%) |
|---|---|---|
| A | pentylene glycol | 1.50 |
| | BG | 3.50 |
| | Glycerin | 1.00 |
| | Methyl paraben | 0.20 |
| | Carbomers | 0.20 |
| | EDTA-2Na | 0.10 |
| | Fill up with water | 100.00 |
| B | Composite emulsifier (NIKKOL NIKKOMULESE LC) | 4.00 |
| | Methylheptyl laurate | 3.50 |
| | Squalane | 0.50 |
| | Cetearyl alcohol | 1.50 |
| | Macadamia nut oil | 0.50 |
| | Avocado oil | 0.50 |
| | Shea fat | 0.50 |
| | Propyl paraben | 0.10 |
| C | Water, C-ring (C-ring concentration 1 µM) | 1 mL |

A and B were dissolved with heat at 80 °C and then A and B were kept at 80 ° C and A was stirred by a homomixer while B was gradually added thereto, and the mixture was emulsified. C was further added to the mixture, and the mixture was allowed to cool to 35 °C as kept being stirred.

### 4.5.3 A body soap comprising the C-ring was mixed and prepared in composition as follows.

**[Table 20]**

| | Names of ingredients (tradenames) | Content (%) |
|---|---|---|
| A | Cocoil glutamic acid TEA solution | 30.0 |
| | Sodium trideceth-4-carboxylate (NIKKOL ECTD-3 NEX) | 5.0 |
| | Sodium cocoamphoacetate solution (NIKKOL AM-101) | 10.0 |
| | PEG-50 hydrogenated castor oil (NIKKOL HCO-50) | 0.5 |
| | 1,3-butylene glycol | 5.0 |
| | Antiseptic agent | q.s. |
| B | EDTA-2Na | q.s. |
| | Fill up with water | 100.0 |
| C | Water, C-ring (C-ring concentration 1 µM) | 1 mL |

A and B were dissolved with heat at 80 °C and then, keep stirring, B was gradually added to A. Keeping urther stirirng, C was added at from 40 to 35 ° C, and the mixture was allowed to cool to RT as kept being stirred.

The shanpoo, hair rinse or body soap prepared as descrbied above was used once a day for 14 days, and conditions of scalp and hair or skin condition were evaluated. Cases and evaluations are summarized in the forllowing table. Changes in skin condition were evaluated by visual observation by the subject of such as dandruffs and erythema on scalp, whereas, scalp itchiness, moist feeling, hair combability, complexion and resilience/elasticity were evaluated by cenesthesic evaluation by the subject.

**[Table 21]**

| Case | Treatment | Symptoms before use | Symptoms during/after use | Subjective changes |
|---|---|---|---|---|
| L1 Age: 60's Sex: Male | Shampoo Hair treatment | Erythema and itches on scalp being observed. Thinning and falling of hair being observed. | Improved scalp erythema and reduced falling of hair. | Itches was improved. Hair became moistened and provided with resilience, radiance and elasticity. |
| L2 Age: 20's Sex: Female | Shampoo | Scalp desiccation and dandruffs being observed with itches, | Dandruff and desiccation of scalp was improved. | Itches was improved. Hair became moistened and provided with radiance, resilience, elasticity, and good combability. |
| L3 Age: 60's Sex: Female | Body soap | Dry and sensitive skin. | Dry skin was improved, itches after bathing was relieved. | Itches after bathing was improved. |
| L4 Age: 60's Sex: Female | Body soap | Dry and sensitive skin. | Dry skin was improved, itches after bathing was relieved. | Itches after bathing was improved. |
| L5 Age: 20's Sex: Male | Body soap | Dry skin. | Dry skin was improved. | Skin was moistened after bathing. |

As above, the use of the hair agent comprising the C-ring of the present invention showed an immediate effect of improving itches, erythema and desiccation of scalp. For thinning or falling hair, it was shown that it is effective for improving the symptoms by the continuous use for at least two weeks Moreover, it was shown that the use of the body soap comprising the C-ring of the present invention has an effect of improving dry skin and hypersensitivity of skin.

### 5. Confirming therapeutic effect for alopecia

The C-ring gel formulation and CNP gel formulation were applied and their effects on various symptoms associated with hair loss were observed. Cases and evaluation are summarized in the tables below. Changes in symptoms were judged based on based on cenesthesic evaluation by the subject and objective observation by the inventor as a physician.

**[Table 22]**

| Case | Treatment | Disease Name | Post-application Symptoms | Notes |
|---|---|---|---|---|
| A1 Sex: Female Age: 30's | Right side of temporal ophiasis site: C-ring gel formulation applied once/day continuously, | Ophiasis, multiple alopecia. | On right side of temporal ophiasis site, stimulation and growth of hair were observed at 7 days after starting applying C-ring gel formulation, | |
| A2 Sex: Female Age: 30's | C-ring gel formulation applied once/day continuously. | Multiple alopecia areata | After 1 week of twice/day applications, remarkable stimulation and growth of terminal hair were observed. | |
| A3 Sex: Male Age: 30's | Left side of head decalvant site: CNP gel formulation applied once/day continuously, | Alopecia universalis | As compared to C-ring applied right side, stimulated area did not extend, and hair elongation speed was slow. | |
| | Right side of head decalvant site: C-ring gel formulation applied once/day continuously. | | After 2 weeks of C-ring application, stimulated area extended to hairline, where there once observed no hair stimulation. As compared to CNP applied left side, hair stimulation and elongation speed was fast. | |
| A4 Sex: Female Age: 20's | Head decalvant site: C-ring gel formulation applied once/day continuously. | Alopecia areata | After 1 week of application, remarkable stimulation and growth of terminal hair were observed. | |

**[Table 23]**

| Case | Treatment | Disease Name | Post-application Symptoms | Notes |
|---|---|---|---|---|
| A5 S ex: Male Age: 30's | Head decalvant site of the subject: C-ring gel formulation applied once/day continuously. | Alopecia areata | After 1 week of application, remarkable stimulation and growth of^{,} terminal hair were observed. | |
| A6 Sex: Female Age: 30's | C-ring gel formulation applied once/day continuously, | Multiple alopecia areata | After 1 week of twice/day applications, remarkable stimulation and growth of terminal hair were observed. | |
| A7 Sex: Male Age: 40's | Right side: C-ring gel formulation applied once/day continuously. | Multiple alopecia areata | On right side of decalvant site, at 7 days after starting applying C-ring gel formulation, there were clearly more stimulated hairs than left side, indicating hair nourishing effect. Hairs were thick terminal hairs, and stimulated area continued to extend. | |
| | Left side: CNP gel formulation applied once/day continuously, | | On left side of CNP gel formulation applied site, few downy hairs were observed, but no stimulation of terminal hair. Elongation speed was slower and stimulated area was clearly smaller. | |

### 5.1 Confirming therapeutic effect for AGA

The C-ring gel formulation and CNP gel formulation were applied and their effects on the symptoms in AGA were observed. Cases and evaluation are summarized in the tables below. Changes in symptoms were judged based on based on cenesthesic evaluation by the subject and objective observation by the inventor as a physician.

**[Table 24]**

| Case | Treatment | Disease Name | Post-application Symptoms | Notes |
|---|---|---|---|---|
| A8 Sex: Male Age: 50's | Right side: C-ring 1 µmol gel formulation applied once/day continuously. | Male-type alopecia | 7 days after starting application, hair became elastic and thick, dramatically reducing the number of falling hairs. As a result, hair density was increased, making hair thinning inconspicuous Gel formulation comprising C-ring exhibited more remarkable hair-stimulating/growing effect than CNP. | |
| | Left side: CNP gel formulation applied once/day continuously, | | | |
| A9 Sex: Male Age: 50's | Parietal hair thinning site: C-ring gel formulation applied once/day continuously. | Male-type alopecia and alopecia pityroides with dandruff complication. | 1 week after starting applying C-ring gel formulation, downy hairs in parietal part became thick, long and dark. Hair was provided with resilience and elasticity. Newly stimulated terminal hairs were observed, and the area of hair thinning was remarkably reduced. Generation of Dandruffs was suppressed, and pruritus was eliminated. | |
| A10 Sex: Female Age: 40's | Parietal head decalvant site and frontal hairline hair thinning site: C-ring gel formulation applied once/day continuously, | Female-type alopecia, scalp seborrhea | 5 days after starting application, hair started to be stimulated from parietal part and frontal hairline part. 2 weeks after starting application, thinning of hair was remarkably improved, making base skin not show through, indicating hair stimulating and growing effect. Scalp seborrhea was also improved, Pruritus was removed, Furthermore, scalp was cleaned and hair became elastic. Non-white hairs started to be increased, In third week, more hairs continued to be stimulated and grown all over the frontal part and hairline, thinning of hair was remarkably improved. | |
| A11 Sex: Male Age: 40's | Hairline M-type decalvant part and parietal O-type decalvant part: C-ring gel formulation applied once/day continuously, | Male-type alopecia consisting of Hairline M-type decalvant part and parietal O-type decalvant part. | 1 week after starting application, hair stimulation was observed, hair body became enhanced. 2 weeks after starting application, remarkable hair restoration was observed in both hairline M-type decalvant part and parietal O-type decalvant part, hair gained resilience and elasticity, became dark and thick, and falling hairs were also decreased. | |

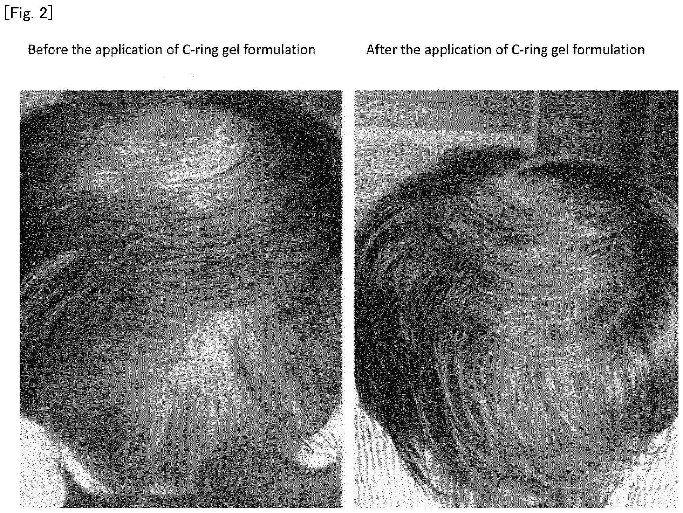

Fig. 2 is a diagrm showing the results of before and after the application of C-ring gel formulation to a patient who developed male pattern AGA (M- and O-types) (A11). At 7 weeks after starting the application, hair gained strong elasticity and thickness, and hair falling decreased drastically.. As a result, hair became denser so that thinning of hair became inconspicuous. These phenomena of stimulating hair growth and growing hair are more prominent with the gel formulation comprising the C-ring than with CNP. Particularly, in a case of M-type thinning hair, once-receded hairline advanced forward, comfirming the effects of stimulating hair growth, restoring hair and growing hair which was extremely more remarkable as compared to CNP.

### 5.2 Summary of cases

In cases of the subject having above-mentioned female pattern alopecia, male pattern alopecia, diffuse alopecia, ophiasis or alopecia areata multilocularis, when the C-ring gel formulation according to the working examples were applied, hair falling were remarkably decreased, the area of stimulated terminal hairs was enlarged and their growth was rapid. Moreover, the existing hairs became resilient and more elastic. In any case, improvement in symptoms were more remarkably expressed as compared to the case in which such CNP gel formulation was applied to a comparative example. That is, the C-gel formulation has superior effects on the symptoms described above as compared to the CNP gel formulation.

Furthermore, the C-ring gel formulation was capable of improving seborrheic conditions, cleaning scalp and suppressing dandruff. It also prevents hair from turning white, grows black hairs, and resulting in an improvement in thinning hair. In addition, it is capable of suppressing itches in 10 minutes, demonstrating an excellent immediate effect.

### 6. Confirming therapeutic effect on rhinitis

### 6.1 Cases

C-ring nasal drop and CNP gel nasal drop were administered via spraying and its effects on rhinitis in the subject, etc. were observed. Cases and their evalueations are summarized in the tables below. Changes in symptoms were judged based on based on cenesthesic evaluation by the subject and objective observation by the inventor as a physician.

**[Table 25]**

| Case | Treatment | Disease Name | Symptoms after nasal drop application |
|---|---|---|---|
| R1 Sex: Female Age: 50's | 1 spray of 0.1 ml C-ring nasal drop agent. | Perennial chronic rhinitis involving rhinorrhea as main symptom, causing 20 or more blowing/day. | C-ring nasal drop treated side: Immediately after nasal drop application, the subject felt she could breathe through the right nasal cavity. After 2 min, nasal respiration became easy. After 3 min, no nasal drip was discharged upon blowing. The effect of 1 nasal drop treatment lasted from the next day for 1 month, leaving little nasal discharge. |
| R2 Sex: Male Age: 10's | Right nasal cavity: 1 spray of 0.1 ml C-ring nasal drop agent. | Rhinitis with severe symptoms of nasal congestion. | Right nasal cavity: 1 min after nasal drop treatment, in right nasal cavity, distress in nasal respiration was dissolved, enabling the breath go through. On the next day of nasal drop treatment, nasal congestion and rhinorrhea were still being resolved. There was no irritation caused by the treatment. |
| | Left nasal cavity: 1 spray of 0.1 ml CNP nasal drop agent. | | Left nasal cavity: 5 min after nasal drop treatment, in left nasal cavity, nasal congestion was somewhat improved, though the subject did not feel he could breathe through left nasal cavity as he did so in right nasal cavity. |
| R3 Sex: Female Age: 40's | Right nasal cavity: 1 spray of 0.1 ml C-ring nasal drop agent. | Perennial allergic rhinitis involving rhinorrhea as main symptom, causing 20 or more blowing/day. | Right nasal cavity: Immediately after nasal drop treatment, nose became clear. After 3 min, the subject was capable of nasal respiration and no nasal drip was discharged upon blowing. |
| | Left nasal cavity: 1 spray of 0.1 ml CNP nasal drop agent. | | Left nasal cavity: 3 min after nasal drop treatment, in left nasal cavity, nasal congestion was somewhat improved, though the subject did not feel he could breathe through left nasal cavity as he did so in right nasal cavity. 3 min after nasal drop, small amount of nasal drip was discharged upon blowing. |
| R4 Sex: Female Age: 50's | 1 spray of 0.1 ml C-ring nasal drop agent. | Chronic rhinitis. Severe rhinorrhea and nasal congestion. | 2 min after nasal drop treatment, nasal congestion was improved, and the subject was capable of normal nasal respiration and rhinorrhea was terminated. 3 min after nasal drop treatment, no nasal drip was discharged upon blowing. 8 min after nasal drop treatment, nasal drip was discharged upon blowing. |
| R5 Sex: Male Age: 40's | Right nasal cavity: 1 spray of 0.1 ml C-ring nasal drop agent. | Allergic rhinitis. sneezing involving rhinorrhea as main symptom, complicated with mild nasal congestion. | Right nasal cavity: 2 min after nasal drop treatment, the subject was easily capable of nasal respiration. 3 min after nasal drop treatment, no nasal drip was discharged from right nasal cavity upon blowing. Nasal respiration was easier in right nasal cavity as compared to left nasal cavity. |
| | Left nasal cavity: 1 spray of 0.1 ml CNP nasal drop agent. | | Left nasal cavity: 3 min after nasal drop treatment, the patient still have difficulty in nasal respiration, where symptoms of nasal congestion were still observed, 9 min after nasal drop treatment, small amount of nasal drip was discharged upon blowing. |

**[Table 26]**

| Case | Treatment | Disease Name | Symptoms after nasal drop application |
|---|---|---|---|
| R6 Sex: Female Age: 30's | Right nasal cavity: 1 spray of 0.1 ml C-ring nasal drop agent. | Rhinitis with both rhinorrhea and congestion, both symptoms being severe, causing 20 or more blowinglday. Neither nasal congestion nor rhinorrhea can sufficiently be improved with oral anti-allergic agent. | Right nasal cavity: 2 min after nasal drop treatment, nasal congestion was remarkably improved, 3 min after nasal drop treatment, the subject was easily capable of nasal respiration. 4 min after nasal drop treatment, no nasal drip was discharged from right nasal cavity upon blowing. Nasal congestion was also dissolved, |
| | | | For 3 days thereafter, neither nasal discharge nor nasal congestion was observed at all, |
| | Left nasal cavity: 1 spray of 0.1 ml CNP nasal drop agent. | | Left nasal cavity: 3 min after nasal drop treatment, no improvement in symptoms of nasal congestion. |
| R7 Sex: Female Age: 60's | 1 spray of 0.1 ml of 1 µM C-ring nasal drop agent. | Rhinitis with rhinorrhea. Continuous symptoms of rhinorrhea and sneezing being observed. Heavy nasal discharge that makes conversation difficult. | C-ring nasal drop treatment: 2 min after nasal drop treatment, the subject was easily capable of nasal respiration. 3 min after nasal drop, no nasal drip was discharged upon blowing. |
| R8 Sex: Female Age: 30's | Right nasal cavity: 1 spray of 0.1 mL of 1 µM C-ring nasal drop agent. | Allergic rhinitis (rhinorrhea-type). The subject has rhinorrhea which cannot be dissolved all day, usually causing 20 or more blowing/day. | Right nasal cavity: 2 min after nasal drop treatment, the subject was capable of nasal respiration. Nasal drop treatment caused no irritation on right nasal cavity side. 3 min after nasal drop treatment, no nasal drip was discharged. |
| | Left nasal cavity: 1 spray of 0.1 mL of 1 µM CNP nasal drop agent, respectively. | | Left nasal cavity: 3 min after nasal drop treatment, the subject was not capable of nasal respiration yet. 4 min after nasal drop treatment, in left nasal cavity, the subject discharged small amount of nasal drip upon blowing. |
| R9 Sex: Female Age: 40's | 1 spray of 0.1 ml of 2 µM C-ring nasal drop agent. | Allergic rhinitis (rhinorrhea-type). The subject cannot be free from rhinorrhea even with nasal steroid application. | C-ring nasal drop treatment: 2 min after nasal application, the subject was easily capable of nasal respiration. 3 min after nasal application, no nasal drip was discharged upon blowing. For 2 days thereafter, no rhinorrhea was observed, and then 2 more weeks, nasal discharge was little. |

### 6.2 Summary of Cases

In any of the cases described above, both rhinorrhea and nasal congestion were more quickly improved or eliminated when the C-ring nasal drop accoding to the working examples was applied, as compared to the cases in which such CNP nasal drop was applied to the comparative examples. That is, the C-ring nasal drop had a superior immediate effect as compared to the CNP nasal drop. Moreover, the effects of the C-ring nasal drop is equal to or more than the effect of the CNP nasal drop, lasted for a prolonged time period, and were capable of suppressing the reccurence of the symptoms.

### 7. Analyzing the state of binding to Type-B receptor of human CNP

As described above, it has been explained that the C-ring compound of the present invention has superior physiological effects on various diseases as compared to conventional CNPs by presenting th. data based on clinical examples. Such physiological effects are also supported by the result of the conformational analysis of compounds carried out the inventors, as explained by the following experimental report for reference.

In order to examine the binding state of human CNP (CNP-22) and its receptor, Type-B receptor (NPR-B), an in silico analysis using conformational homology modeling was carried out. For modeling, Swiss-Pdb viewer and SWISS-MODEL were used.

### 7.1 Template structure

First, prior to the analysis as described above, the template structure for examination of the binding state between human CNP and the Type-B receptor was selected. As this template structure, the conformation of the complex of rat NPR-A and rat ANP peptide (PDB ID:1T34) was used. The rat NPR-A is a homodimer consisting of a A-chain and a B-chain. Conformation of such rat NPR-A has been determined by X-ray crystal structure analysis in which 21 residues from rat ANP Cys7 to Arg27 were bound. The conformation of rat NPR-A was obtained from Protein Data Bank, which is a database for protein conformation. The amino acid consistency between human NPR-B and rat NPR-A is 45 %.

Herein, the number depicted along with a symbol of an amino acid residue refers to the order of that amino acid residue within the peptide from its N-terminal. Accordingly, for example, in human CNP, a notation of "Cys6" means the cysteine in the sixth from N-terminal in the amino acid sequence of human CNP. Notations for amino acid residues at other sites are understood in a similar manner.

### 7.2 CNP peptide model

Here, as CNP peptide model, the amino acid sequence of the resion from Cys6 to Cys22 in human CNP (CNP-22), Cys-Phe-Gly-Leu-Lys-Leu-Asp-Arg-Ile-Gly-Ser-Met-Ser-Gly-Leu-Gly-Cys (SEQ ID NO: :5) was used. Naturally, that resion of the CNP peptide model is consistent with the C-ring of the present invention.

### 7.3 Homology modeling

In order to infer which amino acid residue is involved in the binding of human NPR-B with human CNP, a model of the complex in which the CNP peptide bound to human NPR-B was constructed by homology modeling. In specific, the structures of human CNP peptide and human NPR-B were modeled based on the template structures of the rat ANP peptide and rat NPR-A, respectively.

Next, residues that are directly involved in interaction were speculated among amino acid residues detected between the human CNP peptide model and human NPR-B.

Consequently, it was shown that the CNP peptide model is bound to the human NPR-B dimer being sandwiched in between A- and B-chains.

In thus constructed complex model, the presence of a hydrophobic bond formed by the side chain of Phe2 and Leu4 between the CNP peptide model and A-chain of NPR-B, and a hydrophobic bond formed by the Cys1 principal chain and the side chain of Lys5 and Arg8 were speculated. Also, between the CNP peptide model and the B-chain of NPR-B, the presence of a hydrophobic bond formed by the side chain of Met12, a hydrogen bond formed by the principal chain of Gly16 and the side chain of Arg8 were speculated. Each of Cys1, Phe2, Leu4, Lys5, Arg8, Met12 and Gly16 of the CNP peptide model corresponds to Cys6, Phe7, Leu9, Lys10, Arg13, Met17 and Gly21 of the human CNP (CNP-22), respectively. Among the amino acid residues of human CNP these are considered to the residues that contriubte to the activation of NPR-B.

### 7.4 Considerations

As above, by in silico conformational analysis, it was speculated that the residues that contribute to NPR-B activation of human CNP is present in its cyclic moiety, i.e., C-ring.

Furthermore, from NMR analysis, it was elucidated that ANP does not take any particular conformation in solution, the conformation greatly wobbles, and CNP is considered to be similar. In this point, if the amino acid residues of the cyclic moiety of human CNP contribute to its binding to human NPR-B, it is speculated that the amino acid residues of the tail part make human CNP difficult to enter the CNP binding site between A- and B-chains of human NPR-B because of that large wobbling. Therefore, it is considered that the cyclic moiety of human CNP is smaller than CNP itself and is capable of more readily and quickly entering the CNP binding site of the NPR-B.

Accordingly, it is speculated that the cyclic moiety of human CNP has a higher affinity to human NPR-B than CNP. Molecularbiological mechanism by which the cyclic peptide of the present invention has a better drug efficacy/effect as compared to CNP has not necessarily been clear. However, it is suggested that the cyclic peptide of the present invention higher binding ability and affinity to NPR-B receptor (GC-B) than CNP, and the results of in silico analysis that the CNP cyclic structure contributes to NPR-B activation and binds to NPR-B more easily and quickely than CNP support the aforementioned clinical examples that the C-ring compound of the present invention has a better physiological effect on diseases than conventional CNP.

If C-ring is originated from other species, it is speculated that it exhibits a similar effect to the C-ring of human origin because it has a similar structure as long as it has an analogous amino acid sequence to that of human.

### 8. Speculating replaceable amino acid residues in CNP cyclic moiety

Using the constructed model structure of the complex, we examined the replaceablity of the amino acid residues other than those for which an interaction was speculated.

In specific, in order to examine whether the substituted peptide is capable of binding to NPR-B when the amino acid residues other than those for which an interaction was speculated were substituted with other amino acids, we constructed a CNP mutant model and analyzed about the interaction. Swiss-Pdb viewer was used for modeling.

In specific, among the amino acid residues of human CNP, it was examined whether it is capable of substituting Gly8, Leu11, Asp12, Ile14, Gly15, Ser16, Ser18, Gly19 and Leu20 with other amino acids from the following point.
- Causing no steric hindrance upon binding to NPR-B; showing no interatomic impact in NPR-B and CNP mutant model structure;
- Confiring no influence on surface electrostatic potential;
- Causing no large increase in the value of intramolecular energy (no unreasonable angle and torsion caused in interatomic binding);
- Forming no hydrogen bond which is not originally observed between the chains of NPR-B and CNP and within the chain of CNP.
- Causing no cavity (hollow, gap).

Among those listed above, the value of intramolecular energy was calculated by Compute Energy command of Swiss-Pdb viewe. The intramolecular energy was calculated in unit kilojoule/mol (Kj/mol) based on the sum of the length of interatomic bond, bond angle, torsion and binding energy, etc.

The result of the analysis indicated amino acid residues which are replaceable in Gly8, Leu11, Ile14, Ser16, Ser18 and Leu20. The results are summarized in the tables below. [Table 27]

As above, even when substitution of an amino acid residue corresponding to the substitution described in the table above was carried out in the cyclic moiety of human CNP, i.e., the peptide expressed by the formula II, it was suggested that a peptide in which such substitution was performed exhibits a similar effect to the peptide consisting of the amino acid sequence expressed by the formula II.

### 8. Confirming the therapeutic effect of the cyclic peptide in which amino acid was substituted

Next, for cyclic peptides in which a part of amino acids was substituted were subjected to the following examination in order to confirm their effect. Method of producing the above-mentioned cyclic peptide and method of confirming the amino acid sequence of the produced peptide were similar to the above-described method of producting a cyclic peptide and method of mass spectropy. The mutant cyclic peptides used in the examination were those described in the table below. The table below listed examples of the cyclic peptides of the present invention which are not limited to these. In the table below, the description of the number of the mutation, e.g., "0 to 2", indicates that 0, 1 or 2 amino acids in the sequence can be optionally mutated. In any of the cases of SEQ ID NOs: 22 to 31, 42 to 47, 54 to 56, 60 to 62, it is possible to exclude the case of SEQ ID NO: 3 (i.e., 0 amino acid mutation).

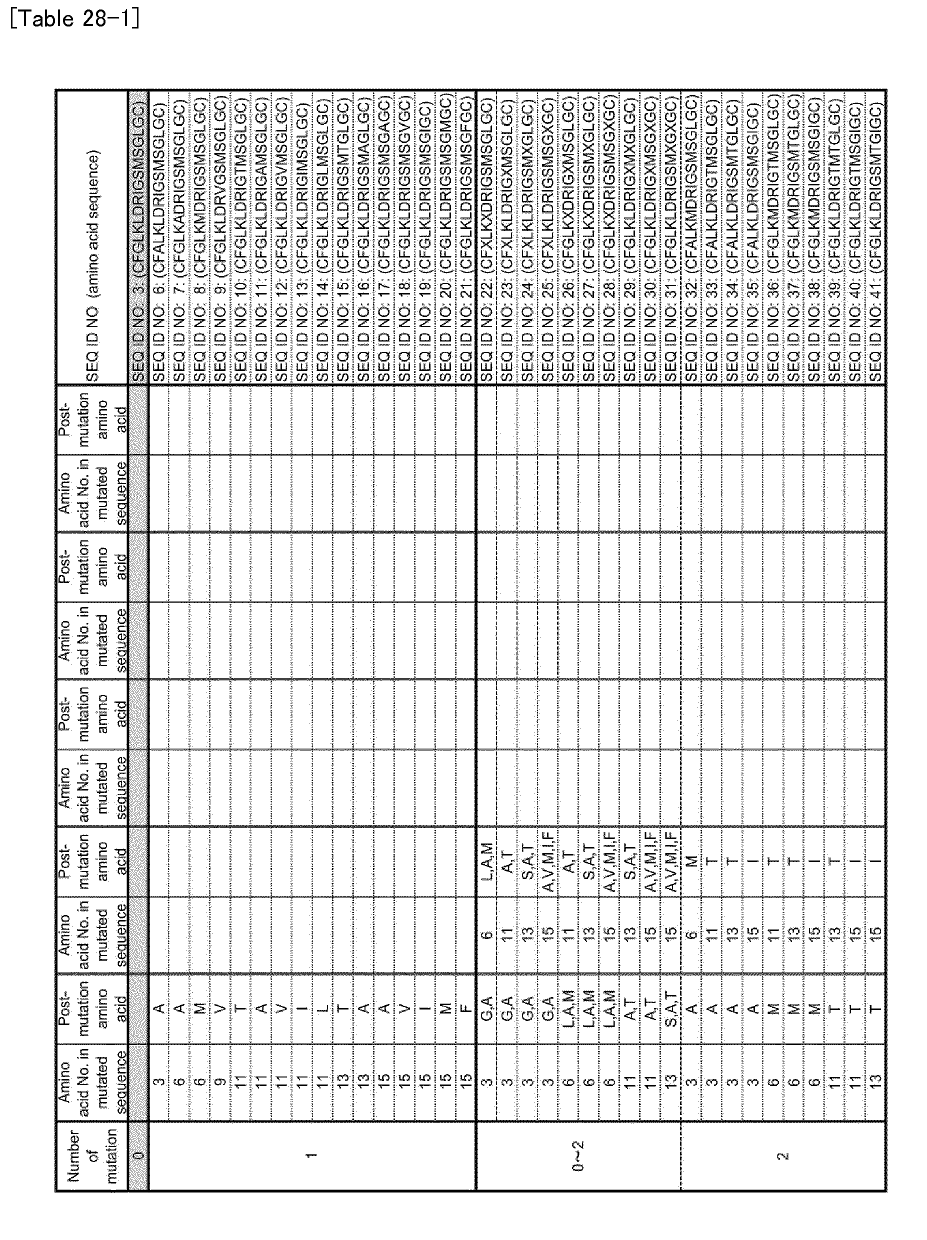

**[Table 28-2]**

| | | | | | | | | | | | |
|---|---|---|---|---|---|---|---|---|---|---|---|
| 0∼3 | 3 | G,A | 6 | L,A,M | 11 | A,T | | | | | SEQ ID NO: 42: (CFXLKXDRICXMSGLGC) |
| | 3 | G,A | 6 | L,A,M | 13 | S,A,T | | | | | SEQ ID NO: 43: (CFXLKXDRIGSMXGLGC) |
| | 3 | G,A | 11 | L,A,M | 15 | A,V,M,I,F | | | | | SEQ ID NO: 44: (CFXLKXDRIGSMSGXGC) |
| | 6 | L,A,M | 11 | A,T | 13 | S,A,T | | | | | SEQ ID NO: 45: (CFGLKXDRIGXMXGLGC) |
| | 6 | L,A,M | 11 | A,T | 15 | A,V,M,I,F | | | | | SEQ ID NO: 46: (CFGLKXDRIGXMSGXGC) |
| | 6 | L,A,M | 13 | S,A,T | 15 | A,V,M,I,F | | | | | SEQ ID NO: 47: (CFGLKXDRIGSMXGXGC) |
| 3 | 3 | A | 6 | M | 11 | T | | | | | SEQ ID NO: 48: (CFALKMDRIGTMSGLGC) |
| | 3 | A | 6 | M | 13 | T | | | | | SEQ ID NO: 49: (CFALKMDRIGSMTGLGC) |
| | 3 | A | 6 | M | 15 | I | | | | | SEQ ID NO: 50: (CFALMKDRIGSMSGIGC) |
| | 6 | M | 11 | T | 13 | T | | | | | SEQ ID NO: 51: (CTGLKMDRIGTMTGLGC) |
| | 6 | M | M | T | 15 | I | | | | | SEQ ID NO: 52: (CFGLKMDRIGTMSGIGC) |
| | 6 | M | 13 | T | 15 | I | | | | | SEQ ID NO: 53 (CFGLKMDRIGSMTGIGC) |
| 0∼4 | 3 | G,A | 6 | L,A,M | 11 | A,T | 13 | S,A,T | | | SEQ ID NO: 54: (CFXLKXDRIGXMXGLGC) |
| | 3 | G,A | 6 | L,A,M | 11 | A,T | 15 | A,V,M,I,F | | | SEQ ID NO: 55: (CFXLKXDRIGXMSGXGC) |
| | 3 | G,A | 6 | L,A,M | 13 | S,A,T | 15 | A,V,M,I,F | | | SEQ ID NO: 56: (CFXLKXDRIGSMXGXGC) |
| 4 | 3 | A | 6 | M | 11 | T | 13 | T | 13 | T | SEQ ID NO: 57: (CFALKMDRIGTMTGLGC) |
| | 3 | A | 6 | M | 11 | T | 15 | I | 15 | I | SEQ ID NO: 58: (CFALKMDRIGTMSGICC) |
| | 3 | A | 6 | M | 13 | T | 15 | I | 15 | I | SEQ ID NO: 59: (CFALKMORIGSMTGIGC) |
| 0∼5 | 3 | G,A | 6 | L,A,M | 9 | I,V | 11 | A,T | 13 | S,A,T | SEQ ID NO: 60: (CFXLKXDRXGXMXGLGC) |
| | 3 | G,A | 6 | L,A,M | 9 | I,V | 11 | A,T | 15 | A,V,M,I,F | SEQ ID NO: 61: (CFXLKKDRXGXMSGXGC) |
| | 3 | G,A | 6 | L,A,M | 9 | I,V | 13 | S,A,T | 15 | A,V,M,I,F | SEQ ID NO: 62: (CFXLKXDRXGSMXGXGC) |

| | | | | | | | | | | | |
|---|---|---|---|---|---|---|---|---|---|---|---|
| In SEQ ID NO: 3, 6 to 62, 1st and 17 th Cys from left form a disulfide bond. | | | | | | | | | | | |

### 8.1 Confirming the effect on skin

The obtained cyclic peptide was combined with purified water at 1, 5, 30, 100, 500 *µ*g/ml and applied to the affected site. Formulations A to E refers to cyclic peptide concentration of 1, 5, 30, 100, 500 *µ*g/ml, respectively. No effect was observed without the peptide or by treating with purified water, comfirming that it was not placebo effect.

**[Table 29-1]**

| SEQ ID No. (amino acid sequence) of the cyclic peptide used | No. | Sex | Age | Diagnostic impression | Formulatio n | Treatment method | Post-treatment diagnostic skin impression |
|---|---|---|---|---|---|---|---|
| SEQ ID NO: 6: (CFALKLDRIGSMSGLGC) | S1 | F | 80's | eczema large winkles | B | once/day | Immediately after application, itches were eliminated. |
| | | | | | | | After 3 min, erythema and edema were remitted, large winkles were shallowed. |
| SEQ ID NO: 6: (CFALKLDRIGSMSGLGC) | S2 | F | 40's | rough skin | C | once/day | Immediately after application, itches were eliminated, skin texture was improved. |
| | | | | | | | After 2 min, wrinkles were shallowed, skin was full and given resilience. |
| SEQ ID NO: 15: (CFGLKLDRIGSMTGLGC) | S3 | M | 40's | psoriasis | B | once/day | After 3 min, scales were remitted. |
| | | | | | | | 3 min after further application, infiltration showed a tendency of getting reduced. |
| SEQ ID NO: 15: (CFGLKLDRIGSMTGLGC) | S4 | F | 60's | seborrheic dermatitis | C | once/day | Immediately after application, itches were remitted. |
| | | | | | | | After 3 min, seborrhea, dandruffs and erythema were remitted. |
| SEQ ID NO: 15: (CFGLKLDRIGSMTGLGC) | S5 | F | 20's | rough skin wrinkles | A | once/day, for 7 days | After 1 min, itches were eliminated, desiccation was remitted. |
| | | | | | | | After 7 days, wrinkles were shallowed, skin was kept moistened, facial erythema and scales were normalized. |
| SEQ ID NO: 16: (CFGLKLDRIGSMAGLGC) | S6 | F | 20's | atopic dermatitis | B | once/day | Immediately after application, itches were eliminated, erythema and scales were remarkably improved. |
| SEQ ID NO: 16: (CFGLKLDRIGSMAGLGC) | S7 | F | 10's | psoriasis vulgaris | C | once/day | After 3 min, crusts, scales, erythema and infiltration was remitted. |
| | | | | | | | After 10 min, erythema was further remitted. |

**[Table 29-2]**

| | | | | | | | |
|---|---|---|---|---|---|---|---|
| SEQ ID NO: 17: (CFGLKLDRIGSMSGAGC) | S8 | M | 40's | psoriasis vulgaris | B | once/day | Immediately after application, crusts, scales, erythema and infiltration were remitted. |
| SEQ ID NO: 17: (CFGLKLDRIGSMSGAGC) | S9 | M | 10's | atopic dermatitis | C | once/day | Immediately after application, itches were eliminated. |
| | | | | | | | After 1 min, infiltration, erythema and scales were remitted. |
| SEQ ID NO: 18: (CFGLKLDRIGSMSGVGC) | S10 | F | 50's | chapped lips | B | twice/day, for 3 days | After 3 days, lip cracks were shallowed, desiccation was improved, lips were soft and full, and kept moistened. |
| SEQ ID NO: 19: (CFGLKLDRIGSMSGIGC) | S11 | F | 50's | atopic dermatitis | B | twice/day, for 3 days | Immediately after application, itches were eliminated. |
| | | | | | | | After 3 days, erythema was eliminated, skin was given resilience, wrinkles were shallowed, |
| SEQ ID NO: 20: (CFGLKLDRIGSMSGMGC) | S12 | F | 50's | chapped lips | B | once/day | After 3 min, cracks at the corner of mouth was epithelized, desiccation was improved. |
| SEQ ID NO: 21: (CFGLKLDRIGSMSGFGC) | S13 | F | 50's | rough skin wrinkles seborrhea | B | once/day | After 1 min, itches were eliminated. |
| | | | | | | | After 3 min, desiccation was improved, skin was given resilience and better texture, kept moistened and made full, wrinkles were shallowed. |
| SEQ ID NO: 32: (CFALKMDRIGSMSGLGC) | S14 | F | 20's | rough skin acne (comedo) | C | once/day | Immediately after application, itches were eliminated. |
| | | | | | | | After 1 min, red papules was eliminated, erythema was remitted, skin texture was improved. |
| | | | | | | | After 3 min, acne (comedo) was reduced. |
| SEQ ID NO: 32: (CFALKMDRIGSMSGLGC) | S15 | F | 10's | psoriasis vulgaris | C | once/day | After 3 min, crusts, scales, erythema and infiltration were remitted. |
| | | | | | | | After 10 min, erythema was further remitted. |

**[Table 29-3]**

| | | | | | | | |
|---|---|---|---|---|---|---|---|
| SEQ ID NO: 33: (CFALKLDRIGTMSGLGC) | S16 | M | 40's | psoriasis | C | once/day | After 3 min, scales were remitted. |
| | | | | | | | 3 min after further application, infiltration showed a tendency of getting reduced. |
| SEQ ID NO: 33: (CFALKLDRIGTMSGLGC) | S17 | F | 20's | atopic dermatitis | B | once/day | After 1 min, itches and scales and erythema were remitted. |
| SEQ ID NO: 33: (CFALKLDRIGTMSGLGC) | S18 | F | 50's | atopic dermatitis | C | once/day, for 3 days | Immediately after application, itches were eliminated. |
| | | | | | | | After 1 min, erythema, scales and infiltration were remarkably remitted. |
| | | | | | | | After 3 days of 1 application/day, erythema, scales and infiltration were further remitted, with no itches left. |
| SEQ ID NO: 33: (CFALKLDRIGTMSGLGC) | S19 | F | 50's | rosacea | A | once/day | After 3 min, itches were eliminated, skin texture was improved, erythema was further remitted. |
| SEQ ID NO: 34: (CFALKLDRIGSMTGLGC) | S20 | F | 50's | atopic dermatitis | C | once/day | Immediately after application, itches were eliminated. |
| | | | | | | | After 1 min, erythema, scales, infiltration were remarkably remitted. |
| | | | | | | | After 3 days of 1 application/day, erythema, scales, infiltration were further remitted, with no itches left. |
| SEQ ID NO: 34: (CFALKLDRIGSMTGLGC) | S21 | F | 80's | atopic dermatitis | C | once/day | After 3 min, itches were eliminated, exudation stopped, red papules and infiltration were remitted. |
| SEQ ID NO: 34: (CFALKLDRIGSMTGLGC) | S22 | F | 50's | atopic dermatitis | C | once/day, for 3 days | Immediately after application, itches were remitted, erythema, infiltration and scales were remarkably improved. |
| | | | | | | | After 3 days of application, the effects described above lasted for 1 week. |
| SEQ ID NO: 34: (CFALKLDRIGSMTGLGC) | S23 | M | 30's | atopic dermatitis | C | once/day | After 3 min, itches were eliminated, papules and erythema were remitted. |

**[Table 29-4]**

| | | | | | | | |
|---|---|---|---|---|---|---|---|
| SEQ ID NO: 34: (CFALKLDRIGSMTGLGC) | S24 | F | 20's | rough skin acne (comedo) | A | once/day | After 3 min, skin texture was improved, skin was given resilience, ruddy face and oily skin were improved, pores were reduced. |
| SEQ ID NO: 34: (CFALKLDRIGSMTGLGC) | S25 | F | 40's | psoriasis vulgaris | C | once/day | After 3 min, scales, crusts, infiltration were remitted. |
| SEQ ID NO: 34: (CFALKLDRIGSMTGLGC) | S26 | F | 20's | atopic dermatitis | C | once/day, for 7 days | After 1 min, itches were eliminated. |
| | | | | | | | After 3 min, erythema, infiltration, lichenification were remarkably improved. |
| | | | | | | | 7 days after application, skin texture was improved, skin was kept resilient and moisturized, erythema were remarkably improved. |
| SEQ ID NO: 34: (CFALKLDRIGSMTGLGC) | S27 | F | 10's | chapped lips | C | once/day | After 3 min, desiccation and erythema were remarkably improved. |
| SEQ ID NO: 34: (CFALKLDRIGSMTGLGC) | S28 | F | 10's | rough skin | C | once/day | After 3 min, skin resilience and texture were improved, desiccation was improved. |
| SEQ ID NO: 7: (CFGLKADRIGSMSGLGC) | S29 | F | 80's | eczema large winkles | B | once/day | Immediately after application, itches were eliminated. |
| | | | | | | | After 3 min, erythema and edema were remarkably remitted. large winkleswere shallowed, given resilience. |
| SEQ ID NO: 7: (CFGLKADRIGSMSGLGC) | S30 | F | 20's | atopic dermatitis | C | once/day | After 3 min, lichenification, scales and desiccation were remitted, skin was softened. |
| SEQ ID NO: 35: (CFALKLDRIGSMSGIGC) | S31 | M | 10's | atopic dermatitis | C | once/day | After 1 min, itches were eliminated. |
| | | | | | | | After 2 min, scales and erythema were remarkably improved. |
| | | | | | | | After 3 min, skin was almost normalized and moisturized. |

**[Table 29-5]**

| | | | | | | | |
|---|---|---|---|---|---|---|---|
| SEQ ID NO: 35: (CFALKLDRIGSMSGIGC) | S32 | F | 20's | atopic dermatitis | B | once/day | Immediately after application, itches were eliminated, erythema and scales were remarkably improved. |
| SEQ ID NO: 36: (CFGLKMDRIGTMSGLGC) | S33 | F | 60's | psoriasis vulgaris | C | once/day, for 2 days | After 3 min, scales and infiltration were remitted. |
| | | | | | | | 2 days after application, infiltration and erythema were remitted. |
| SEQ ID NO: 37: (CFGLKMDRIGSMTGLGC) | S34 | F | 50's | atopic dermatitis | C | once/day, for 3 days | Immediately after application, itches were remitted, erythema, infiltration and scales were remarkably improved. |
| | | | | | | | After 3 days of application, the effects described above lasted for 1 week. |
| SEQ ID NO: 37: (CFGLKMDRIGSMTGLGC) | S35 | M | 20's | atopic dermatitis | A | once/day | After 2 min, itches were eliminated. |
| | | | | | | | After 3 min, infiltration, erythema and scratch scars were remitted. |
| SEQ ID NO: 38: (CFGLKMDRIGSMSGIGC) | S36 | M | 10's | atopic dermatitis | C | once/day | After 1 min, itches were eliminated. |
| | | | | | | | After 2 min, scales and erythema were both remarkably improved. |
| | | | | | | | After 3 min, skin was almost normalized and moisturized. |
| SEQ ID NO: 38: (CFGLKMDRIGSMSGIGC) | S37 | M | 20's | atopic dermatitis | C | once/day | After 1 min, itches were eliminated, scales and erythema were remitted. |
| SEQ ID NO: 38: (CFGLKMDRIGSMSGIGC) | S38 | F | 30's | contact dermatitis | C | once/day | After 1 min, itches was remitted. |
| | | | | | | | After 3 min, swelling lessened, erythema was remitted. |
| SEQ ID NO: 38: (CFGLKMDRIGSMSGIGC) | S39 | F | 30's | rough skin wrinkles | B | once/day | After 2 min, desiccation was remitted, wrinkles were shallowed, skin was full and given resilience. |

**[Table 29-6]**

| | | | | | | | |
|---|---|---|---|---|---|---|---|
| SEQ ID NO: 38: (CFGLKMDRIGSMSGIGC) | S40 | F | 20's | atopic dermatitis | C | once/day | After 3 min, itches were eliminated, scales and erythema were improved. |
| SEQ ID NO: 38: (CFGLKMDRIGSMSGIGC) | S41 | F | 60's | psoriasis vulgaris | C | once/day, for 2 days | After 3 min, scales, infiltration were remitted. |
| | | | | | | | 2 days after application, infiltration and erythema were remitted. |
| SEQ ID NO: 38: (CFGLKMDRIGSMSGIGC) | S42 | F | 40's | rough skin | A | once/day | After 2 min, skin was given resilience, skin was full and kept moistened. |
| SEQ ID NO: 39: (CFGLKLDRIGTMTGLGC) | S43 | M | 20's | atopic dermatitis | C | once/day | After 1 min, itches were eliminated, scales and erythema were remitted. |
| SEQ ID NO: 39: (CFGLKLDRIGTMTGLGC) | S44 | F | 20's | rough skin wrinkles | B | once/day | After 1 min, itches were eliminated. |
| SEQ ID NO: 40: (CFGLKLDRIGTMSGIGC) | S45 | F | 30's | contact dermatitis | B | once/day | After 1 min, itches were eliminated, erythema was remitted. |
| | | | | | | | After 3 min, wrinkles became soft and shallowed. |
| SEQ ID NO: 40: (CFGLKLDRIGTMSGIGC) | S46 | F | 20's | atopic dermatitis | C | once/day | After 3 min, itches were eliminated, scales and erythema were improved. |
| SEQ ID NO: 40: (CFGLKLDRIGTMSGIGC) | S47 | F | 80's | atopic dermatitis | C | once/day | After 3 min, itches were eliminated, exudation stopped, red papules and infiltration were remitted, |

**[Table 29-7]**

| | | | | | | | |
|---|---|---|---|---|---|---|---|
| SEQ ID NO: 40: (CFGLKLDRIGTMSGIGC) | S48 | F | 20's | rough skin | C | once/day | After 2 min, skin was given resilience and complexion, skin texture was improved. |
| SEQ ID NO: 40: (CFGLKLDRIGTMSGIGC) | S49 | M | 10's | atopic dermatitis | C | once/day | Immediately after application, itches were eliminated. |
| | | | | | | | After 1 min, infiltration, erythema and scales were remitted. |
| SEQ ID NO: 40: (CFGLKLDRIGTMSGIGC) | S50 | F | 50's | chapped lips | A | twice/day, for 3 days | After 3 days of application, lip cracks were shallowed, desiccation was improved, lips were soft and full, and kept moistened. |
| SEQ ID NO: 40: (CFGLKLDRIGTMSGIGC) | S51 | F | 50's | atopic dermatitis | B | twice/day, for 3 days | Immediately after application, itches were eliminated. |
| | | | | | | | After 3 days, erythema was eliminated, skin was given resilience, wrinkles were shallowed. |
| SEQ ID NO: 40: (CFGLKLDRIGTMSGIGC) | S52 | F | 40's | contact dermatitis | C | once/day | After 2 min, itches were eliminated, erythema, scales and infiltration were remitted. |
| SEQ ID NO: 40: (CFGLKLDRIGTMSGIGC) | S53 | F | 20' s | atopic dermatitis | C | once/day | After 3 min, lichenification, scales, desiccation was remitted, skin was softened. |
| SEQ ID NO: 40: (CFGLKLDRIGTMSGIGC) | S54 | F | 30's | chapped lips | C | once/day | After 1 min, cracks at the corner of mouth were shallowed, desiccation was improved. |
| | | | | | | | After 3 min, cracks at the corner of mouth 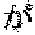 epithelized. |
| SEQ ID NO: 41: (CFGLKLDRIGSMTGIGC) | S55 | F | 10's | psoriasis vulgaris | C | once/day | After 3 min, crusts, scales, erythema and infiltration were remitted. |
| | | | | | | | After 10 min, erythema was further remitted. |

**[Table 29-8]**

| | | | | | | | |
|---|---|---|---|---|---|---|---|
| SEQ ID NO: 41: (CFGLKLDRIGSMTGIGC) | S56 | F | 60's | rough skin wrinkles | B | once/day | Immediately after application, itches were remitted, |
| | | | | | | | After 1 min, skin desiccation were remitted, skin redness was improved, wrinkles were shallowed, skin was given resilience. |
| SEQ ID NO: 48: (CFALKMDRIGTMSGLGC) | S57 | M | 40's | psoriasis | B | once/day | After 3 min, scales were remitted. |
| | | | | | | | 3 min after further application, infiltrative showed a tendency of getting reduced. |
| SEQ ID NO: 48: (CFALKMDRIGTMSGLGC) | S58 | F | 50's | atopic dermatitis | C | once/day | Immediately after application, itches were eliminated. |
| | | | | | | | After 1 min, erythema, scales and infiltration were remarkably remitted. |
| | | | | | | | After 3 days of 1 application/day, erythema, scales and infiltration were further remitted, with no itches left. |
| SEQ ID NO: 48: (CFALKMDRIGTMSGLGC) | S59 | M | 80's | contact dermatitis | C | once/day | After 1 min, itches and pains were remitted. |
| | | | | | | | After 3 min, erythema and infiltration were remitted. |
| SEQ ID NO: 48: (CFALKMDRIGTMSGLGC) | S60 | F | 10's | psoriasis vulgaris | C | once/day | After 3 min, crusts, scales, erythema and infiltration were remitted. |
| | | | | | | | After 10 min, erythema was further remitted. |
| SEQ ID NO: 48: (CFALKMDRIGTMSGLGC) | S61 | F | 80's | eczema | C | once/day | Immediately after application, itches were eliminated. |
| | | | | | | | After 3 min, erythema and infiltration were remarkably remitted. The effects described above lasted for 7 days. |
| SEQ ID NO: 48: (CFALKMDRIGTMSGLGC) | S62 | F | 60's | rough skin wrinkles | A | once/day | Immediately after application, itches were remitted. |
| | | | | | | | After 1 min, skin desiccation was remitted, skin redness was improved, wrinkles were shallowed, and skin was further given resilience. |
| SEQ ID NO: 48: (CFALKMDRIGTMSGLGC) | S63 | F | 50's | atopic dermatitis | C | once/day, for 3 days | Immediately after application, itches were remitted, erythema, infiltration and scales were remarkably improved. |
| | | | | | | | After 3 days of application, the effects described above lasted for 1 week. |

**[Table 29-9]**

| | | | | | | | |
|---|---|---|---|---|---|---|---|
| SEQ ID NO: 48: (CFALKMDRIGTMSGLGC) | S64 | F | 50's | rosacea | C | once/day | After 3 min, erythema, infiltration and papules were remitted. |
| SEQ ID NO: 48: (CFALKMDRIGTMSGLGC) | S65 | F | 50's | rosacea | C | once/day | Immediately after application, erythema were remitted. |
| | | | | | | | After 3 min, acne (comedo) was desiccated and reduced. |
| SEQ ID NO: 48: (CFALKMDRIGTMSGLGC) | S66 | F | 50's | chapped lips | C | once/day | After 3 min, cracks at the corner of mouth was epithelized, desiccation was improved. |
| SEQ ID NO: 48: (CFALKMDRIGTMSGLGC) | S67 | F | 50's | rough skin wrinkles seborrhea | C | once/day | After 1 min, itches were eliminated. |
| | | | | | | | After 3 min, desiccation was improved, skin was given resilience, and skin texture was improved, skin was kept moisturized, skin was full, wrinkles were shallowed. |
| SEQ ID NO: 49: (CFALKMDRIGSMTGLGC) | S68 | F | 50's | seborrheic dermatitis acne (comedo) | C | once/day | Immediately after application, saborrheaand erythema were remitted. |
| | | | | | | | After 3 min, acne (comedo) was reduced. |
| SEQ ID NO: 49: (CFALKMDRIGSMTGLGC) | S69 | M | 60's | psoriasis vulgaris | A | once/day | Immediately after application, scales and infiltrative erythema were remitted. |
| SEQ ID NO: 50: (CFALKMDRIGSMSGIGC) | S70 | F | 80's | eczema | A | once/day | Immediately after application, itches were eliminated. |
| | | | | | | | After 3 min, erythema and infiltration were remitted. |

**[Table 29-10]**

| | | | | | | | |
|---|---|---|---|---|---|---|---|
| SEQ ID NO: 50: (CFALKMDRIGSMSGIGC) | S71 | F | 50's | atopic dermatitis | C | once/day | Immediately after application, itches were eliminated. |
| | | | | | | | After 1 min, erythema, scales and infiltration were remarkably remitted. |
| | | | | | | | After 3 min, lichenification was remitted. |
| | | | | | | | After 3 days of 1 application/day, erythema and infiltration were remitted, itches and lichenification were futher removed. |
| SEQ ID NO: 50: (CFALKMDRIGSMSGIGC) | S72 | F | 10's | psoriasis vulgaris | C | once/day | After 3 min, crusts, scales, erythema and infiltration was remitted. |
| | | | | | | | After 10 min, erythema was further remitted. |
| SEQ ID NO: 50: (CFALKMDRIGSMSGIGC) | S73 | F | 80's | eczema | C | once/day | Immediately after application, itches were eliminated. |
| | | | | | | | After 3 min, erythema and infiltration were remarkably remitted. The effects lasted for 7 days. |
| SEQ ID NO: 50: (CFALKMDRIGSMSGIGC) | S74 | F | 30's | rough skin wrinkles | C | once/day | Immediately after application, desiccation were improved, skin texture was improved, given resilience, wrinkles were shallowed. Crows feet, dark circles beneath eyes were made inconspicuous. The effects lasted for 2 weeks, eye whole was kept resilient and moisturized. |
| SEQ ID NO: 50: (CFALKMDRIGSMSGIGC) | S75 | F | 50's | atopic dermatitis | C | once/day, for 3 days | Immediately after application, itches were remitted, erythema, infiltration and scales were improved, lichenificated skin was softened. |
| | | | | | | | After 3 days of application, erythema was remarkably improved, the effects lasted for 1 week. |
| SEQ ID NO: 50: (CFALKMDRIGSMSGIGC) | S76 | M | 30's | atopic dermatitis | C | once/day | After 3 min, itches were eliminated, papules and erythema were remitted. |
| SEQ ID NO: 8: (CFGLKMDRIGSMSGLGC) | S77 | F | 30's | seborrheic dermatitis acne (comedo) | C | once/day | After 1 min, itches were eliminated. |
| | | | | | | | After 3 min, seborrheaand erythema were remitted, pore opening became inconspicuous, acne (comedo) was reduced. |

**[Table 29-11]**

| | | | | | | | |
|---|---|---|---|---|---|---|---|
| SEQ ID NO: 8: (CFGLKMDRIGSMSGLGC) | S78 | F | 30's | rough skin | A | once/day | Immediately after application, itches were eliminated. After 3 min, skin texture was improved finely, pore opening was improved. |
| SEQ ID NO: 8: (CFGLKMDRIGSMSGLGC) | S79 | M | 20's | atopic dermatitis | B | once/day | Immediately after application, itches were eliminated, erythema and scales were remitted. |
| SEQ ID NO: 8: (CFGLKMDRIGSMSGLGC) | S80 | F | 50's | atopic dermatitis | C | once/day, for 3 days | Immediately after application, itches stopped, and by the day next, erythema was remarkably improved. |
| | | | | | | | After stopping 3 days of application, the effects lasted for 1 week, and never relasped. |
| SEQ ID NO: 51: (CFGLKMDRIGTMTGLGC) | S81 | M | 20's | atopic dermatitis | C | once/day | Immediately after application, itches were eliminated. |
| | | | | | | | After 3 min, erythema, papules, infiltration, crusts and scratch scars were remitted. |
| SEQ ID NO: 51: (CFGLKMDRIGTMTGLGC) | S82 | F | 80's | eczema | B | once/day | Immediately after application, itches were eliminated. |
| | | | | | | | After 3 min, erythema and infiltration were remitted. |
| SEQ ID NO: 52: (CFGLKMDRIGTMSGIGC) | S83 | M | 20's | atopic dermatitis | C | once/day | After 1 min, itches were eliminated, erythema and infiltration were remitted. |
| SEQ ID NO: 52: (CFGLKMDRIGTMSGIGC) | S84 | F | 20's | chronic eczema | C | once/day | After 3 min, itches were eliminated, erythema and lichenification were remitted, scratch scars were further epithelized. |
| SEQ ID NO: 52: (CFGLKMDRIGTMSGIGC) | S85 | M | 80's | contact dermatitis | C | once/day | After 1 min, itches and pains were remitted. |
| | | | | | | | After 3 min, erythema and infiltration were remitted. |

**[Table 29-12]**

| | | | | | | | |
|---|---|---|---|---|---|---|---|
| SEQ ID NO: 52: (CFGLKMDRIGTMSGIGC) | S86 | F | 50's | eczema-secondary erythroderma | C | once/day | After 1 min, itches were eliminated, stinging pain was calmed down. |
| | | | | | | | After 3 min, exudation was almost stopped, erythema and infiltration were remitted, and swelling lessened. |
| SEQ ID NO: 52: (CFGLKMDRIGTMSGIGC) | S87 | F | 20's | atopic dermatitis | C | once/day | After 3 min, itches were eliminated, lichenification, erythema and scales were improved |
| SEQ ID NO: 52: (CFGLKMDRIGTMSGIGC) | S88 | M | 40's | psoriasis vulgaris | B | once/day | After 3 min, crusts, erythema and infiltration were remitted. |
| SEQ ID NO: 52: (CFGLKMDRIGTMSGIGC) | S89 | F | 60's | psoriasis vulgaris | B | once/day | Immediately after application, infiltration were remarkably remitted. After 3 min, erythema was improved. |
| SEQ ID NO: 52: (CFGLKMDRIGTMSGIGC) | S90 | F | 30's | rough skin wrinkles | C | once/day | Immediately after application, desiccation were improved, skin texture was improved, given resilience, wrinkles were shallowed. Crows feet, dark circles beneath eyes were made inconspicuous. |
| | | | | | | | The effects lasted for 2 weeks, eye wholes were kept resilient and moisturized. |
| SEQ ID NO: 52: (CFGLKMDRIGTMSGIGC) | S91 | F | 20's | nummular eczema | C | once/day, for 5 days | After 3 min, itches were eliminated, erythema and infiltration was remitted. |
| | | | | | | | Scratch scars were dried and epithelized. |
| | | | | | | | 5 days after application, lichenification was remitted. |
| SEQ ID NO: 52: (CFGLKMDRIGTMSGIGC) | S92 | F | 40's | contact dermatitis | A | once/day | After 2 min, itches were eliminated, erythema, scales and infiltration were remitted. |
| SEQ ID NO: 53: (CFGLKMDRIGSMTGIGC) | S93 | M | 80's | contact dermatitis | C | once/day | After 1 min, itches and pains were remitted. |
| | | | | | | | After 3 min, erythema and infiltration were remitted. |

**[Table 29-13]**

| | | | | | | | |
|---|---|---|---|---|---|---|---|
| SEQ ID NO: 53: (CFGLKMDRIGSMTGIGC) | S94 | F | 20's | atopic dermatitis | B | once/day | After 3 min, itches were eliminated, lichenification, erythema and scales were improved. |
| SEQ ID NO: 57: (CFALKMORIGTMTGLGC) | S95 | M | 80's | contact dermatitis | C | once/day | After 1 min, itches and pains were remitted. |
| | | | | | | | After 3 min, erythema and infiltration were remitted. |
| SEQ ID NO: 57: (CFALKMDRIGTMTGLGC) | S96 | F | 60's | rough skin wrinkles | A | once/day | 1 min after application, erythema, edema and itches were remitted. |
| SEQ ID NO: 58: (CFALKMDRICTMSGIGC) | S97 | M | 40's | psoriasis | B | once/day | After 3 min, scales were remitted. |
| | | | | | | | 3 min after further application, infiltration showed a tendency of being reduced. |
| SEQ ID NO: 58: (CFALKMDRIGTMSGIGC) | S98 | F | 50's | seborrheic dermatitis acne (comedo) | C | once/day | Immediately after application, seborrhea and erythema were remitted. After 3 min, acne (comedo) was reduced. |
| SEQ ID NO: 58: (CFALKMDRIGTMSGIGC) | S99 | F | 50's | rosacea | C | once/day | Immediately after application, erythema was remitted. |
| | | | | | | | After 3 min, acne (comedo) was desiccated and reduced. |
| SEQ ID NO: 58: (CFALKMDRIGTMSGIGC) | S100 | F | 20's | rough skin acne (comedo) | A | once/day | After 3 min, skin texture was improved, given resilience, ruddy face and oily skin were improved, pores were reduced. |
| SEQ ID NO: 59: (CFALKMDRIGSMTGIGC) | S101 | M | 60's | psoriasis vulgaris | C | once/day | Immediately after application, scales and infiltrative erythema were remitted. |

**[Table 29-14]**

| | | | | | | | |
|---|---|---|---|---|---|---|---|
| SEQ ID NO: 59: (CFALKMDRIGSMTGIGC) | S102 | M | 30's | atopic dermatitis | A | once/day | 2 min after application, erythema, infiltration and papules were remitted. |
| SEQ ID NO: 9: (CFGLKLDRVGSMSGLGC) | S103 | F | 30's | rough skin | A | once/day | Immediately after application, itches were remitted. |
| | | | | | | | After 1 min, scales and erythema were remitted. |
| | | | | | | | After 3 min, skin was full, skin texture was improved. |
| SEQ ID NO: 9: (CFGLKLDRVGSMSGLGC) | S104 | F | 50's | eczema-secondary erythroderma | C | once/day | After2 min, itches were eliminated. stinging pain was calmed down. |
| | | | | | | | After 3 min, erythema and infiltration were remitted, swelling lessened. |
| SEQ ID NO: 9: (CFGLKLDRVGSMSGLGC) | S105 | F | 30's | rough skin | B | once/day | Immediately after application, itches were eliminated, After 3 min, skin texture was finely improved, pore opening was improved. |
| SEQ ID NO: 10: (CFGLKLDRIGTMSGLGC) | S106 | F | 60's | contact dermatitis large winkles | C | once/day | Immediately after application, itches and stinging pain were calmed down. After 3 min, swelling and erythema were dramatically remitted, large winkles were shallowed. |
| SEQ ID NO: 10: (CFGLKLDRIGTMSGLGC) | S107 | F | 80's | eczema large winkles | B | once/day | Immediately after application, itches were eliminated |
| | | | | | | | After 3 min, erythema and edema were remitted, large winkles were shallowed. |
| SEQ ID NO: 11: (CFGLKLDRIGAMSGLGC) | S108 | F | 80's | eczema large winkles | C | once/day | Immediately after application, itches were eliminated. |
| | | | | | | | After 3 min, erythema and edema were remarkably remitted. Large winkleswere shallowed, given resilience. |
| SEQ ID NO: 11: (CFGLKLDRIGAMSGLGC) | S109 | F | 60's | rough skin wrinkles | B | once/day | After 1 min, stinging itches were eliminated. |
| | | | | | | | After 3 min, edema and erythema were remitted, large winkles were shallowed. |

**[Table 29-15]**

| | | | | | | | |
|---|---|---|---|---|---|---|---|
| SEQ ID NO: 12: (CFGLKLDRIGVMSGLGC) | S110 | F | 10's | cha pped lips | B | once/day | After 3 min, skin was moistened, desiccation and erythema was improved. |
| SEQ ID NO: 12: (CFGLKLDRIGVMSGLGC) | S111 | F | 10's | rough skin | C | once/day | After 3 min, red papules was remitted, skin texture was improved. |
| SEQ ID NO: 13: (CFGLKLDRIGIMSGLGC) | S112 | F | 20's | atopic dermatitis | C | once/day | Immediately after application, itches were eliminated. |
| | | | | | | | After 3 min, erythema was remitted, scratch scars were epithelized. lichenification were improved, and skin was softened. |
| SEQ ID NO: 13: (CFGLKLDRIGIMSGLGC) | S113 | F | 40's | rough skin | C | once/day | Immediately after application, itches were eliminated, skin texture was improved. |
| | | | | | | | After 2 min, wrinkles were shallowed, skin was full and given resilience. |
| SEQ ID NO: 14: (CFGLKLDRIGLMSGLGC) | S114 | F | 20's | atopic dermatitis | B | once/day | After 1 min, itches, scales, erythema and desiccation were remitted. |
| SEQ lD NO: 14: (CFGLKLDRIGLMSGLGC) | S115 | F | 40's | psoriasis vulgaris | C | once/day | After 3 min, scales, crusts and infiltration were remitted. |
| SEQ ID NO: 15: (CFGLKLORIGSMTGLGC) | S116 | M | 40's | psoriasis vulgaris | B | once/day, for 4 days | After 1 min, crusts, scales, erythema and infiltration were remitted. |
| | | | | | | | 4 days after application, erythema were remarkably remitted, crusts were eliminated. |
| SEQ ID NO: 52: (CFGLKMDRIGTMSGIGC) | S117 | M | 40's | psoriasis vulgaris | B | once/day, for 4 days | After 3 min, erythema, scales, crusts and infiltration were remitted. |
| | | | | | | | 1 day after application, erythema was remarkably remitted, crusts were almost eliminated. |
| | | | | | | | 4 days after application, crusts were improved such that only part of which remained. |

**[Table 29-16]**

| | | | | | | | |
|---|---|---|---|---|---|---|---|
| SEQ ID NO: 3: (CFGLKLDRIGSMSGLGC) | S118 | M | 20's | atopic dermatitis | A | Two-sided application (Right) once/day | (Right) |
| | | | | | | | After 3 min, erythema, infiltration, itches and stinging feeling were yet to be remitted. |
| SEQ ID NO: 8: (CFGLKMDRIGSMSGLGC) | | | | | | Two-sided application (Left) once/day | (Left) |
| | | | | | | | After 1 min, erythema, infiltration, itches were remitted. |
| | | | | | | | After 2 min, stinging feeling was eliminated. |
| SEQ ID NO: 3: (CFGLKLDRIGSMSGLGC) | S119 | F | 20's | atopic dermatitis | A | Two-sided application (Right) once/day | (Right) |
| | | | | | | | After 3 min, erythema, infiltration were yet to be remitted with desiccation left, and skin texture was unimproved. |
| SEQ ID NO: 8: (CFGLKMDRIGSMSGLGC) | | | | | | Two-sided application (Left) once/day | (Left) |
| | | | | | | | Immediately after application, itches were eliminated. |
| | | | | | | | After 2 min, erythema and Infiltration were remarkably improved, skin texture was improved and moistened. |
| SEQ ID NO: 3: (CFGLKLDRIGSMSGLGC) | S120 | F | 80's | eczema | A | Two-sided application (Right) once/day | (Right) |
| | | | | | | | After 5 min, itches, edema, erythema and scales were yet to be remitted. |
| SEQ ID NO: 58: (CFALKMDRIGTMSGIGC) | | | | | A | Two-sided application (Left) once/day | (Left) |
| | | | | | | | After 1 min, itches were eliminated. |
| | | | | | | | After 2 min, swelling and erythema were remarkably remitted. |
| SEQ ID NO: 3: (CFGLKLDRIGSMSGLGC) | S121 | M | 20's | chronic eczema prurigo nodularis | A | Two-sided application (Right) once/day | (Right) |
| | | | | | | | After 3 min, itches and erythema were yet to be eliminated. |
| SEQ ID NO: 58: (CFALKMDRIGTMSGIGC) | | | | | A | Two-sided application (Left) once/day | (Left) |
| | | | | | | | After 30 sec, itches were eliminated, hot feeling was also removed. |
| | | | | | | | After 3 min, hot feeling and itches were completely eliminated, erythema was remitted. prurigo nodularis also started to be reduced. |

**[Table 29-17]**

| | | | | | | | |
|---|---|---|---|---|---|---|---|
| SEQ ID NO: 3: (CFGLKLDRIGSMSGLGC) | S122 | F | 40's | rough skin | A | Two-sided application (Right) once/day | (Right) |
| | | | | | | | After 5 min, itches and erythema were yet to be remitted, skin texture was unimproved, but oily skin of side of nose and seborrheic erythema were remitted. |
| SEQ ID NO: 58: (CFALKMDRIGTMSGIGC) | | | | | | Two-sided application (Left) once/day | (Left) |
| | | | | | | | Immediately after application, redness was eliminated, and skin texture became fine. |
| | | | | | | | After 3 min, oily skin of side of nose and seborrheic erythema were remitted |
| SEQ ID NO: 9: (CFGLKLDRVGSMSGLGC) | S123 | M | 30's | atopic dermatitis | A | Two-sided application (Right) once/day | (Right) |
| | | | | | | | After 3 min, there were some remaining erythema and infiltration as compared to SEQ ID NO: 58. |
| SEQ ID NO: 58: (CFALKMDRIGTMSGIGC) | | | | | | Two-sided application (Left) once/day | (Left) |
| | | | | | | | Immediately after application, erythema started to be remitted. |
| | | | | | | | After 3 min, erythema was remarkably remitted and infiltration was remitted as compared to SEQ ID NO: 9. |
| SEQ ID NO: 3 (CFGLKLDRIGSMSGLGC) | S124 | M | 30's | chronic eczema | A | Two-sided application (Right) once/day | (Right) |
| | | | | | | | After 10 min, lichenification, erythema was yet to be remitted. |
| SEQ ID NO: 57: (CFALKMDRIGTMTGLGC) | | | | | | Two-sided application (Left) once/day | (Left) |
| | | | | | | | After 10 min, lichenification, erythema was remarkably remitted, skin was made soft. |
| SEQ ID NO: 3: (CFGLKLDRIGSMSGLGC) | S125 | M | 20's | atopic dermatitis | A | Two-sided application (Right) once/day | (Right) |
| | | | | | | | After 3 min, effect on itches was weak and without immediate effect as compared to SEQ ID NO: 59. |
| SEQ ID NO: 59: (CFALKMDRIGSMTGIGC) | | | | | | Two-sided application (Left) once/day | (Left) |
| | | | | | | | Immediately after application, effect was more immediate as compared to SEQ ID NO: 6itches were eliminated. |

**[Table 29-18]**

| | | | | | | | |
|---|---|---|---|---|---|---|---|
| SEQ ID NO: 3: (CFGLKLDRIGSMSGLGC) | S126 | F | 20's | rough skin contact dermatitis | A | Two-sided application (Right) once/day | (Right) |
| | | | | | | | After 3 min, rough desiccation remained, and erythema was not sufficiently improved. |
| SEQ ID NO: 38: (CFGLKMDRIGSMSGIGC) | | | | | | Two-sided application (Left) once/day | After 2 min, rough desiccation and erythema was remitted, skin texture was improved and moisturized. |
| SEQ ID NO: 3: (CFGLKLDRIGSMSGLGC) | S127 | F | 40's | atopic dermatitis wrinkles | A | Two-sided application (Right) once/day | (Right) |
| | | | | | | | After 4 min, desiccation, erythema, stinging feeling was left with unimoroved skin texture and wrinkles left. |
| SEQ ID NO: 34: (CFALKMDRIGTMSGIGC) | | | | | | Two-sided application (Left) once/day | (Left) |
| | | | | | | | After 1 min, desiccation, erythema was substantially remitted. |
| | | | | | | | After 4 min, no more stinging, and erythema were remarkably remitted. Moreover, skin texture was improved, substantially deep wrinkles were shallowed skin texture was impoved. |
| SEQ ID NO: 3: (CFGLKLDRIGSMSGLGC) | S128 | M | 30's | psoriasis vulgaris | A | Two-sided application (Right) once/day | (Right) |
| | | | | | | | After 3 min, crusts, erythema and infiltrative erythema aspect remained. |
| SEQ ID NO: 58: (CFALKMDRIGTMSGIGC) | | | | | | Two-sided application (Left) once/day | After 3 min, crusts, erythema were remitted, infiltrative erythema aspect having clear edges became plane, in which edges were no longer clear and skin was smooth. |
| SEQ ID NO: 3: (CFGLKLDRIGSMSGLGC) | S129 | M | 40's | chronic eczema | A | Two-sided application (Right) once/day | (Right) |
| | | | | | | | After 3 min, erythema and itches were yet to be remitted. |
| | | | | | | | After 10 min. itches and pains were vet to be removed |
| SEQ ID NO: 58: (CFALKMDRIGTMSGIGC) | | | | | | Two-sided application (Left) once/day | (Left) |
| | | | | | | | Immediately after application, erythema started to be remitted, After 1 min, itches were eliminated. After 10 min. the effect still continued. |

By the result above, it was demonstrual to or more than that of the cyclic peptide expressed by the formula II (SEQ ID NO: 3). Similar effect was obtained in a case where substituted cyclic peptide was mixed and applied if the effective amount of the cyclic peptide ated that even if part of the amino acid in the cyclic peptide was substituted, the cyclic peptide of the present invention exhibits an effect that is eqwas applied.

In specific, in subjects having atopic dermatitis, eczema, etc., one simple application from 1 *µ*g/ml to 500 *µ*g/ml to the affected site showed immediate effect of eliminating ithes, epithelized erythema, infiltration, papule and scratch scars, terminating exudate, and epithelized cracks, etc. Moreover, only one application resulted in continuous effect over 1 weeks or more. When it was applied to healthy or rough skin, it improves desiccation by exhibiting moisturizing effect immediately or within a few minutes after the application, confers/retains adequate elasticity or tenderness of skin or mucosa, softens skin, provides skin or mucosa with resilience and firmness. Furthermore, ripples and flabbiness are improved in the applied site, and dullness and spots become inconspicuous. Moreover, it prevents photosensitivity and photodermatosis, with effects of eliminating or reducing the event of light-induced inflammatory symptoms. These effects continued for over 1 week or more with one application.

In specific, in two-sided application method carried out in Examination Examples S119 to S129 (examination where, for one identical subject, CNP cyclic peptide was applied in one side, and the cyclic peptide having 4 amino acid substitutions (such as, e.g., SEQ ID NO: 58) was applied to the other side), the cyclic peptide such as SEQ ID NO: 58 had effects of suppressing inflammatory symptoms, i.e., itches, erythema, scales, infiltration, etc. in atopic dermatitis, psoriasis and eczema within a few minutes after starting its use, even at a concentration as low as 1 *µ*g/ml, and, in addition, it promotes drying and epithelialization of scratch scars. The effects are highly persistent and no itches occur even after stopping the application. In psoriasis, it was confirmed that in a few minutes after the application of the CNP cyclic peptide having amino acid substitution, an erythema with clear edges and a hard aspect that mainly consists of scales became smooth, soft and flat, the eges against normal skin started to became unclear, and erythema and scales were further remitted after 7 or 8 minutes, the skin color turns almost normal, scales became almost inconspicuous and flat. The effects are highly persistent and the smooth condition in which scales and erythema was improved was kept with no reccurence even after three weeks without more application.

The effect obtained by applying 1 *µ*M of the CNP cyclic peptide is equal to or more than the effect obtained by 10 *µ*M to 50 *µ*M of CNP. Moreover, the CNP cyclic peptide newly developed by us here in which a part of the amino acid were substituted had not only a high therapeutic effect but also an immediate affect as compared to CNP or unsubstituted CNP cyclic peptide, and it was further confirmed that it acts at a bery low concentration.

### 8.2 Confirming the effect on scalp or hair

The obtained cyclic peptide was combined with purified water at 1, 5, 30, 100, 500 *µ*g/ml and applied to the affected site. Formulations A to E in the tables refers to the cyclic peptide of 1, 5, 30, 100, 500 *µ*g/ml, respectively. No effect was observed without the peptide or by treating with purified water, comfirming that it was not placebo effect.

**[Table 30-1]**

| SEQ ID No. (amino acid sequence) of the cyclic peptide used | No. | Sex | Age | Case | Formulati on | Treatment method | Post-application diagnostic impression of hair and scalp |
|---|---|---|---|---|---|---|---|
| SEQ ID NO: 33: (CFALKLDRIGTMSGLGC) | U1 | F | 80's | alopecia pityroides | C | once/day | Immediately after application, itches were eliminated. |
| | | | | | | | After 3 min, dandruffs were remitted. |
| SEQ ID NO: 48: (CFALKMDRIGTMSGLGC) | U2 | F | 40's | multiple alopecia areata | C | once/day, for 2 days | On Day 2 of application, falling hairs were reduced and terminal hairs started to grow. |
| SEQ ID NO: 58: (CFALKMDRIGTMSGIGC) | U3 | F | 40's | multiple alopecia areata | C | once/day, for 2 days | On Day 2 of application, falling hairs were reduced, and stimulation and growth of terminal hairs were promoted. |
| SEQ ID NO: 38: (CFGLKMDRIGSMSGIGC) | U4 | F | 50's | seborrheic alopecia multiple alopecia | C | once/day, for 4 days | On Day 4 of application, hairs started to grow, and stimulation and growth of hairs were promoted, seborrhea was also improved, |
| SEQ ID NO: 50: (CFALKMDRIGSMSGIGC) | U5 | F | 50's | seborrheic alopecia multiple alopecia | C | once/day, for 4 days | On Day 4 of application, hairs started to grow, and stimulation and growth of hairs were promoted, and seborrhea was also improved. |
| SEQ ID NO: 34: (CFALKLDRIGSMTGLGC) | U6 | F | 30's | female-type AGA | C | once/day, for 2 days | After 1 day, hair became elastic, hair body was increased. |
| SEQ ID NO: 6: (CFALKLDRIGSMSGLGC) | U7 | F | 40's | female-type AGA | C | once/day, for 7 days | After 7 days, hair became elastic, stimulation and growth of dark and thick hairs were promoted, hair body was increased. |

**[Table 30-2]**

| | | | | | | | |
|---|---|---|---|---|---|---|---|
| SEQ ID NO: 7: (CFGLKADRIGSMSGLGC) | U8 | F | 50's | alopecia areata | C | once/day, for 7 days | After 7 days, hair root was regenerated, terminal hairs started to grow, and stimulation and growth of hairs were promoted. |
| SEQ ID NO: 15: (CFGLKLDRIGSMTGLGC) | U9 | F | 60's | seborrheic alopecia cystic acne | C | once/day, for 5 days | Immediately after application, itches were eliminated. |
| | | | | | | | After 3 min, seborrhea, erythema and dandruffs were remitted. |
| | | | | | | | After 2 days, the absence of itches had been carried on, scalp reddishness and cysts were remitted. |
| | | | | | | | After 5 days, cystic acne was eliminated. |
| SEQ ID NO: 57: (CFALKMDRIGTMTGLGC) | U10 | M | 70's | male-type AGA | C | once/day, for 7 days | After 7 days, stimulation and growth of downy hairs were promoted. |
| SEQ ID NO: 10: (CFGLKLDR!GTMSGLGC) | U11 | M | 70's | male-type AGA | C | once/day, for 7 days | After 7 days, stimulation and growth of downy hairs were promoted. |
| SEQ ID NO: 32: (CFALKMDRIGSMSGLGC) | U12 | F | 30's | alopecia universalis | C | once/day, for 7 days | After 7 days, dark hair root were regenerated, hair stimulation and growth were promoted. |
| SEQ ID NO: 35: (CFALKLDRIGSMSGIGC) | U13 | F | 30's | alopecia universalis | C | once/day, for 7 days | After 7 days, dark hair root were regenerated, hairs started to grow. |

**[Table 30-3]**

| | | | | | | | |
|---|---|---|---|---|---|---|---|
| SEQ ID NO: 51: (CFGLKMDRIGTMTGLGC) | U14 | M | 60's | alopecia areata | C | once/day, for 7 days | After 7 days, dark hair root was regenerated, hairs started to grow, and stimulation and growth of hairs were promoted. |
| SEQ ID NO: 40; (CFGLKLDRIGTMSGIGC) | U15 | F | 30's | ophiasis | C | twice/day, for 7 days | After 7 days, remarkably stimulated hairs along hairline, and promoted hair stimulation and growth were recognized. |
| SEQ ID NO: 49: (CF ALKMDRIGSMTGLGC) | U16 | F | 40's | multiple alopecia areata female-type AGA | C | once/day, for 7 days | After 2 days, falling hairs were reduced, stimulation and growth of hairs were promoted, the parting on forehead was made inconspicuous. |
| | | | | | | | After 7 days, hair root was regenerated 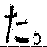 |
| SEQ ID NO: 21: (CFGLKLDRIGSMSGFGC) | U17 | F | 30's | multiple alopecia areata | C | once/day, for 7 days | After 3 days, hair stimulation and promoted hair stimulation were observed in marginal part. After 7 days, hair was entirely grown and nourished, and promotion of hair growth was observed. |
| SEQ ID NO: 49: (CFALKMDRIGSMTGLGC) | U18 | F | 30's | seborrheic alopecia | C | once/day, for 7 days | After 1 day, skin was kept in normal condition, erythema and seborrhea were remitted. |
| | | | | | | | After 7 days, stimulation and growth of hairs were promoted, thinning of hair was improved, and skin did no longer show through. |
| SEQ ID NO: 36: (CFGLKMDRIGTMSGLGC) | U19 | F | 30's | female-type AGA | C | once/day, for 6 days | After 1 day, hairs became elastic, and effects were observed of promoting stimulating and growing of, and nourishing hairs, and hair body was increased to be puffy. The effects were continuous. |
| SEQ ID NO: 58: (CFALKMDRIGTMSGIGC) | U20 | F | 30's | female-type AGA | D | once/day, for 7 days | After 1 day, hair body was increased to be puffy, and effects were observed of promoting growth of and nourishing hair. |
| | | | | | | | After 3 days, the parting became inconspicuous, skin did no longer show through. The effects were continuous. |
| SEQ ID NO: 9: (CFGLKLDRVGSMSGLGC) | U21 | F | 40's | female-type AGA | B | once/day, for 7 days | After 7 days, skin did no longer show through, hairs were nourished, hair body was increased, hair was provided with resilience and elasticity. Also, skin redness was remitted, |

**[Table 30-4]**

| | | | | | | | |
|---|---|---|---|---|---|---|---|
| SEQ ID NO: 58: (CFALKMDRIGTMSGIGC) | U22 | F | 20's | alopecia areata | A | once/day, for 7 days | After 7 days, hair root was regenerated in center, and hairs started to grow in marginal part, and stimulation and growth of hairs were promoted. |
| SEQ ID NO: 6: (CFALKLDRIGSMSGLGC) | U23 | M | 70's | male-type AGA | C | once/day, for 7 days | After 7 days, hair stimulation was promoted, and, in marginal part, in particular, remarkable hair stimulation and growth were promoted. |
| SEQ ID NO: 58: (CFALKMDRIGTMSGIGC) | U24 | M | 70's | male-type AGA | C | once/day, for 7 days | After 7 days, hair stimulation was promoted, and, in marginal part, in particular, hair stimulation and growth were promoted. |
| C SEQ ID NO: 37: (CFGLKMDRIGSMTGLGC) | U25 | F | 30's | ophiasis | C | twice/day, for 7 days | After 7 days, hair stimulation and growth were remarkably promoted. |
| SEQ ID NO: 39: (CFGLKLDRIGTMTGLGC) | U26 | M | 50's | male-type AGA | C | once/day, for 7 days | After 7 days, thick and dark hairs were observed on parietal part, and promoted stimulation and growth of hairs were observed. |
| SEQ ID NO: 53: (CFGLKMDRIGSMTGIGC) | U27 | M | 20's | male-type AGA | C | once/day, for 7 days | After 7 days, stimulating, growing and nourishing of terminal hairs were promoted. |
| SEQ ID NO: 41: (CFGLKLDRIGSMTGIGC) | U28 | M | 20's | male-type AGA | C | once/day, for 7 days | After 7 days, stimulating, growing and nourishing of terminal hairs were entirely promoted. |
| SEQ ID NO: 9: (CFGLKLDRVGSMSGLGC) | U29 | M | 20's | male-type AGA | C | once/day, for 7 days | After 7 days, regeneration of hair root, and promoted stimulating, growing and nourishing of terminal hairs were observed. |

**[Table 30-5]**

| | | | | | | | |
|---|---|---|---|---|---|---|---|
| SEQ ID NO: 51: (CFGLKMDRIGTMTGLGC) | U30 | M | 20's | male-type AGA | C | once/day, for 7 days | After 7 days, stimulation and growth of downy hairs and terminal hairs were promoted, |
| SEQ ID NO: 59: (CFALKMDRIGSMTGIGC) | U31 | M | 20's | male-type AGA | C | once/day, for 7 days | After 7 days, stimulating, growing and nourishing of hair were promoted. |
| SEQ ID NO: 52: (CFGLKMDRIGTMSGIGC) | U32 | M | 60's | alopecia areata | C | once/day, for 7 days | After 3 days, dark hair root were regenerated, stimulation and growth of white hairs were promoted. |
| SEQ ID NO: 40: (CFGLKLDRIGTMSGIGC) | U33 | M | 60's | male-type AGA | B | once/day, for 7 days | After 7 days, hair root was regenerated, stimulation and growth pf dark hairs were promoted. |
| SEQ ID NO: 40: (CFGLKLDRIGTMSGIGC) | U34 | M | 60's | alopecia areata | B | once/day, for 7 days | After 7 days, remarkable stimulation and growth of hairs were promoted. |
| SEQ ID NO: 33: (CFALKLDRIGTMSGLGC) | U35 | M | 10's | alopecia universalis | B | once/day, for 7 days | After 7 days, dark terminal hairs started to grow, and stimulation and growth of hairs were promoted. |
| SEQ ID NO: 34: (CFALKLDRIGSMTGLGC) | U36 | M | 10's | alopecia universalis | B | once/day, for 7 days | After 7 days, hairs started to grow, and stimulation and growth of hairs were promoted. |

**[Table 30-6]**

| | | | | | | | |
|---|---|---|---|---|---|---|---|
| SEQ ID NO: 49: (CFALKMDRIGSMTGLGC) | U37 | F | 40's | multiple alopecia areata | C | once/day, for 7 days | After 2 days, there was a decrease in falling hairs. After 3 days, terminal hairs started to grow, and stimulation and growth of hairs were promoted. |
| SEQ ID NO: 11: (CFGLKLDRIGAMSGLGC) | U38 | F | 40's | multiple alopecia areata | C | once/day, for 7 days | After 7 days, terminal hairs started to grow, and stimulation and growth of hairs were promoted, |
| SEQ ID NO: 11: (CFGLKLDRIGAMSGLGC) | U39 | F | 40's | multiple alopecia areata | C | once/day, for 7 days | After 7 days, dark terminal hairs started to grow, and stimulation and growth of hairs were promoted. |
| SEQ ID NO: 38: (CFGLKMDRIGSMSGIGC) | U40 | M | 50's | multiple alopecia areata seborrheic | C | once/day, for 7 days | After 4 days, hair started to grow, and stimulation and growth of hairs were and seborrhea was further improved. |
| SEQ ID NO: 50: (CFALKMDRIGSMSGIGC) | U41 | M | 50's | multiple alopecia areata seborrheic | C | once/day, for 7 days | After 4 days, hair started to grow, and stimulation and growth of hairs were and seborrhea was further improved. |
| SEQ ID NO: 12: (CFGLKLDRIGVMSGLGC) | U42 | F | 50's | alopecia areata | C | once/day, for 7 days | After 7 days, regeneration of dark hair root, hair stimulation and growth were promoted. |
| SEQ ID NO: 13: (CFGLKLDRIGIMSGLGC) | U43 | F | 40's | female-type AGA | C | once/day, for 7 days | After 7 days, hairs became elastic, stimulation and growth of dark and thick hairs were promoted. |
| SEQ ID NO: 16: (CFGLKLDRIGSMAGLGC) | U44 | M | 30's | alopecia universalis | C | once/day, for 20 days | After 3 to 4 days, hair started to grow, and stimulation and growth of hairs were promoted. |
| | | | | | | | After 7 days, hair elongation was observed. |
| | | | | | | | After 20 days, hair was further restored and the effect continued. |

**[Table 30-7]**

| | | | | | | | |
|---|---|---|---|---|---|---|---|
| SEQ ID NO: 17: (CFGLKLDRIGSMSGAGC) | U45 | M | 30's | alopecia universalis | C | once/day, for 20 days | After 3 to 4 days, hair started to grow, and stimulation and growth of hairs were promoted. |
| | | | | | | | After 7 days, hair elongation was observed. |
| | | | | | | | After 20 days, hair was further restored and the effect continued. |
| SEQ ID NO: 34: (CFALKLDRIGSMTGLGC) | U46 | M | 30's | alopecia universalis | C | once/day, for 20 days | After 3 to 4 days, hair started to grow, and stimulation and growth of hairs were promoted. |
| | | | | | | | After 7 days, hair elongation was observed. |
| | | | | | | | After 20 days, hair was further restored and the effect continued. |
| SEQ ID NO: 38: (CFGLKMDRIGSMSGIGC) | U47 | M | 30's | alopecia universalis | C | once/day, for 20 days | After 3 to 4 days, hair started to grow, and stimula.ion and growth of hairs were promoted. |
| | | | | | | | After 7 days, hair elongation was observed, |
| | | | | | | | After 20 days, hair was further restored and the effect continued. |
| SEQ ID NO: 52: (CFGLKMDRIGTMSGIGC) | U48 | M | 30's | alopecia universalis | C | once/day, for 20 days | After 3 to 4 days, hair started to grow, and stimulation and growth of hairs were promoted. |
| | | | | | | | After 7 days, hair elongation was observed. |
| | | | | | | | After 20 days, hair was further restored and the effect continued. |
| SEQ ID NO: 50: (CFALKMDRIGSMSGIGC) | U49 | M | 30's | alopecia universalis | C | once/day, for 20 days | After 3 to 4 days, hair started to grow, and stimulation and growth of hairs were promoted. |
| | | | | | | | After 7 days, hair elongation was observed, |
| | | | | | | | After 20 days, hair was further restored and the effect continued. |
| SEQ ID NO: 48: (CFALKMDRIGTMSGLGC) | U50 | F | 30's | multiple alopecia areata | C | once/day, for 7 days | After 3 days, regeneration of hair root was observed. |

**[Table 30-8]**

| | | | | | | | |
|---|---|---|---|---|---|---|---|
| SEQ ID NO: 52: (CFGLKMDRIGTMSGIGC) | U51 | F | 30's | multiple alopecia areata | C | once/day, for 7 days | After 3 days, stimulation of dark hairs, promoted hair stimulation and growth, hair restorationwere observed, as well as elongation of terminal hairs. |
| SEQ ID NO: 18: (CFGLKLDRIGSMSGVGC) | U52 | M | 10's | multiple alopecia areata | C | once/day, for 7 days | After 4 days, downy hairs started to grow, remarkably promoted stimulation and growth of hairs were observed, |
| SEQ ID NO: 34: (CFALKLDRIGSMTGLGC) | U53 | M | 10's | multiple alopecia areata | C | once/day, for 7 days | After 4 days, downy hairs started to grow, remarkably promoted stimulation and growth of hairs were observed. |
| SEQ ID NO: 38: (CFGLKMDRIGSMSGIGC) | U54 | M | 30's | multiple alopecia areata | 8 | once/day, for 7 days | After 2 days, hairs started to grow, and stimulation and growth of hairs were promoted. |
| | | | | | | | After 4 days, hair elongation was observed entirely. |
| SEQ ID NO: 8: (CFGLKMDRIGSMSGLGC) | U55 | M | 30's | multiple alopecia areata | B | oncelday, for 7 days | After 2 days, hairs started to grow, and stimulation and growth of hairs were promoted. |
| | | | | | | | After 4 days, hair elonqation was observed. |
| SEQ ID NO: 15: (CFGLKLDRIGSMTGLGC) | U56 | F | 30's | multiple alopecia areata | D | twice/day, for 7 days | After 4 days, hairs started to grow, and stimulation and growth of hairs were promoted, and scalp erythema, itches and dandruffs were eliminated. |
| SEQ ID NO: 39: CFGLKLDRIGTMTGLGC) | U57 | M | 40's | male-type AGA | C | once/day, for 7 days | After 4 days, hairs became elastic, dark and thick hairs grew, and hair body was increased. |
| SEQ ID NO: 15: (CFGLKLDRIGSMTGLGC) | U58 | F | 10's | alopecia areata | A | once/day, for 7 days | After 4 days, downy hairs started to grow, stimulation and growth of hairs were promoted, dark and thick hairs were elongated. |

**[Table 30-9]**

| | | | | | | | |
|---|---|---|---|---|---|---|---|
| SEQ ID NO: 9: (CFGLKLDRVGSMSGLGC) | U59 | M | 30's | multiple alopecia areata | B | once/day, for 7 days | After 3 days, stimulation of terminal hairs, promoted hair stimulation and growth, and hair elongation were observed. |
| SEQ ID NO: 50: (CFALKMDRlCSMSGIGC) | U60 | M | 30's | multiple alopecia areata | B | once/day, for 7 days | After 3 days, stimulation of terminal hairs, promoted hair stimulation and growth, and hair elongation were observed. |
| SEQ ID NO: 57: (CFALKMDRIGTMTGLGC) | U61 | M | 30's | multiple alopecia areata | B | once/day, for 7 days | After 3 days, stimulation of terminal hairs, promoted hair stimulation and growth, and hair elongation were observed. |
| SEQ ID NO: 48: (CFALKMDRIGTMSGLGC) | U62 | F | 30's | alopecia universalis | C | once/day, for 7 days | After 7 days, stimulation and growth of eyebrow hairs were promoted, eyebrow became thick and long, and hairs were restored, with no more falling. Also, regeneration of hair root was observed in evelashes. |
| SEQ ID NO: 41: (CFGLKLDRIGSMTGIGC) | U63 | F | 30's | ophiasis | B | once/day, for 7 days | After 6 days, hair root was regenerated, promoted stimulation and growth of hairs were observed. |

**[Table 30-10]**

| | | | | | | | |
|---|---|---|---|---|---|---|---|
| SEQ ID NO: 7: (CFGLKADRIGSMSGLGC) | U64 | F | 50's | female-type AGA | B | once/day, for 7 days | After 1 day, itches were eliminated, dandruffs were remitted, stimulation and growth of hairs were promoted, hairs became more elastic, hair body was increased. |
| | | | | | | | After 5 days, falling hairs were reduced because hair loss was prevented. |
| SEQ ID NO: 12: (CFGLKLDRIGVMSGLGC) | U65 | F | 50's | female-type AGA | B | once/day, for 7 days | After 1 day, itches were eliminated, dandruffs were remitted, stimulation and growth of hairs were promoted, hairs became more elastic, hair body was increased, |
| | | | | | | | After 5 days, falling hairs were reduced because hair loss was prevented, and |
| SEQ ID NO: 58: (CFALKMDRIGTMSGIGC) | U66 | F | 30's | female-type AGA | B | once/day, for 4 days | After 2 to 3 days, hairs became elastic, and hair body was increased, skin did no longer show through, not only stimulation and growth of hairs were promoted, but hair loss was also prevented, the parting was made |
| SEQ ID NO: 34: (CFALKLDRIGSMTGLGC) | U67 | F | 30's | female-type AGA | B | once/day, for 4 days | After 2 to 3 days, hairs became elastic, and hair body was increased, skin did no longer show through, not only stimulation and growth of hairs were promoted, but hair loss was also prevented, the parting was made |
| SEQ ID NO: 20: (CFGLKLDRIGSMSGMGC) | U68 | F | 30's | female-type AGA | C | once/day, for 7 days | After 7 days, skin did no longer show through, st mulation and growth of hairs were promoted, hairs became elastic, hair body was increased. Skin erythema, dandruffs and itches were remitted. |
| SEQ ID NO: 58: (CFALKMDRIGTMSGIGC) | U69 | F | 20's | alopecia areata | A | once/day, for 7 days | After 7 days, promoted hair stimulation and growth were observed in marginal part, and regeneration of hair root was observed in parietal part. |
| SEQ ID NO: 53: (CFGLKMDRIGSMTGIGC) | U70 | M | 70's | male-type AGA | A | once/day, for 7 days | After 7 days, promoted stimulation and growth of downy hairs, and hair restoration were observed. |
| SEQ ID NO: 19: (CFGLKLDRIGSMSGIGC) | U71 | M | 70's | male-type AGA | C | once/day, for 7 days | After 7 days, promoted stimulation and growth of downy hairs, and hair restoration were observed. |

**[Table 30-11]**

| | | | | | | | |
|---|---|---|---|---|---|---|---|
| SEQ ID NO: 21: (CFGLKLDRIGSMSGFGC) | U72 | M | 40's | male-type AGA | C | once/day, for 7 days | After 7 days, promoted hair stimulation and growth were observed, dark and thick hairs elongated, and hair body was increased. |
| SEQ ID NO: 34: (CFALKLDRIGSMTGLGC) | U73 | M | 40's | male-type AGA | B | once/day, for 7 days | After 7 days, downy hairs 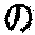 promoted hair stimulation and growth were observed. |
| SEQ ID NO: 40: (CFGLKLDRIGTMSGIGC) | U74 | M | 40's | male-type AGA | B | once/day, for 7 days | After 7 days, promoted hair stimulation and growth were observed, downy hairs became thick and dark. |
| SEQ ID NO: 33: (CFALKLDRIGTMSGLGC) | U75 | M | 40's | alopecia areata | B | once/day, for 7 days | After 7 days, downy hairs started to grow, promoted hair stimulation and growth were observed. |
| SEQ ID NO: 40: (CFGLKLDRIGTMSGIGC) | U76 | M | 40's | alopecia areata | B | once/day, for 7 days | After 7 days, terminal hairs started to grow entirely, and promoted hair stimulation and elongation were remarkably observed. |
| SEQ ID NO: 58: (CFALKMDRIGTMSGIGC) | U77 | F | 30's | multiple alopecia areata | B | once/day, for 3 days | After 2 days, terminal hairs started to grow, and promoted hair stimulation and elongation were remarkably observed. |
| SEQ ID NO: 8: (CFGLKMDRIGSMSGLGC) | U78 | F | 30's | multiple alopecia areata | B | twice/day, for 3 days | After 2 days, terminal hairs started to grow, and promoted hair stimulation and elongation were remarkably observed. |
| SEQ ID NO: 48: (CFALKMDRIGTMSGLGC) | U79 | F | 30's | multiple alopecia areata | B | twice/day, for 3 days | After 2 days, terminal hairs started to grow, and promoted hair stimulation and elongation were remarkably observed. |

**[Table 30-12]**

| | | | | | | | |
|---|---|---|---|---|---|---|---|
| SEQ ID NO: 58: (CFALKMDRIGTMSGIGC) | U80 | F | 30's | multiple alopecia areata | B | twice/day, for 3 days | After 2 days, terminal hairs started to grow, and promoted hair stimulation and elongation were remarkably observed, |
| SEQ ID NO: 15: (CFGLKLDRIGSMTGLGC) | U81 | F | 50's | seborrheic alopecia · alopecia areata | A | once/day, for 7 days | After 7 days, seborrheawas improved, dandruffs, itches were remitted, and growth terminal hairs were observed. |
| SEQ ID NO: 33: ICFALKLDRIGTMSGLGC) | U82 | F | 50's | multiple alopecia areata | A | once/day, for 7 days | After 7 days, terminal hairs and downy hairs started to grow, promoted hair stimulation, hair growth and promoted growth were observed. |
| SEQ ID NO: 16: (CFGLKLDRIGSMAGLGC) | U83 | F | 40's | female-type AGA | A | once/day, for 7 days | After 7 days, hairs along hairline became more elastic, stimulation and growth of hairs were promoted, and downy hairs became thick and dark. |
| SEQ ID NO: 14: (CFGLKLDRIGLMSGLGC) | U84 | F | 40's | female-type AGA | C | once/day, for 10 days | After 10 days, hair became more elastic, stimulation and growth of downy hairs and terminal hairs were promoted, and hairs became thick. |

**[Table 30-13]**

| | | | | | | | |
|---|---|---|---|---|---|---|---|
| SEQ ID NO: 58: (CFALKMDRIGTMSGIGC) | U85 | F | 40's | seborrheic alopecia cystic acne | A | once/day, for 7 days | After 2 days, itches were eliminated, skin erythema, seborrhea, folliculitis and dandruffs were improved, and hair stimulation and growth were promoted. |
| | | | | | | | After 7 days, normalized. |
| SEQ ID NO: 9: (CFGLKLDRVGSMSGLGC) | U86 | M | 80's | male-type AGA | C | once/day, for 7 days | After 7 days, hairs became more elastic, and hair body was increased. In addtion to promoted hair stimulation and growth, prevention of hair loss was observed, by which the area of hair loss was reduced. |
| SEQ ID NO: 3: (CFGLKMDRIGSMSGLGC) | U87 | M | 30's | ophiasis | A | Two-sided application (Left) | (Left) |
| | | | | | | once/day, for 6 days | After 6 days of application, no hair stimulation was observed. |
| SEQ ID NO: 58: (CFALKMDRIGTMSGIGC) | | | | | | Two-sided application (Right) | (Right) |
| | | | | | | once/day, for 6 days | After 6 days of application, stimulation of a number of downy hairs was observed. |
| SEQ ID NO: 3: (CFGLKLDRIGSMSGLGC) | U88 | M | 10's | multiple alopecia areata | A | Two-sided application (Left) | (Left) |
| | | | | | | once/day, for 3 days | After 3 days of application, no clear hair stimulation was observed. |
| SEQ ID NO: 50: (CFALKMDRIGSMSGIGC) | | | | | | Two-sided application (Right) | (Right) |
| | | | | | | once/day, for 3 days | After 3 days of application, stimulation and elongation of downy hairs and terminal hairs were observed, |

By the result above, it was demonstrated that even if part of the amino acid in the cyclic peptide was substituted, the cyclic peptide of the present invention exhibits an effect that is equal to or more than that of the cyclic peptide expressed by the formula II (SEQ ID NO: 3). Similar effect was obtained in a case where substituted cyclic peptide was mixed and applied if the effective amount of the cyclic peptide was applied.

In specific, in two-sided application method carried out in Examination Examples U87 to U88 (examination where, for one identical subject, CNP cyclic peptide was applied in one side, and the cyclic peptide having 4 amino acid substitutions (such as, e.g., SEQ ID NO: 58) was applied to the other side), the cyclic peptide such as SEQ ID NO: 58 had effects of suppressing itches, etc., in atopic dermatitis, psoriasis and eczema within a few minutes after starting its use, even at a concentration as low as 1 *µ*g/m.

The effect obtained by applying 1 *µ*M of the CNP cyclic peptide is equal to or more than the effect obtained by 10 *µ*M to 50 *µ*M of CNP. It had not only a high therapeutic effect but also an immediate affect as compared to CNP or unsubstituted CNP cyclic peptide.

In specific, in a case where it was applied to a site of hair loss of a site of stimulating hair growth, etc., it had effects of preventing hair loss and at the same time promoting hair growth stimulation and growing hair, providing hair with resilience and elasticity, voluming hair and reducing hair falling dramatically. Moreover, effects of nourishing hair, promoting hair growth and improvin/preventing thinning of hair. Particulaly, hairs whoes growth is stimulated tend to become terminal hairs, which are not white. These effects were not observed in CNP, for example, which were conventionally used in a therapeutic for alopecia. That is, for even intractable alopecia areata multilocularis or ophiasis, for example, the effects are expressed remarkably early; such as that, by only one application, stimulated hair growth was confirmed on the next day. Such effect continued to stimulate hair growth even after stopping the application. In AGA and healthy subject, immediate efffects are observed such as providing hair with resilience and elasticity, voluming hair and reducing hair falling dramatically, which last even after stopping the application. Moreover, in addition to the effect above, an effect of improving/preventing dermatitis on scalp was observed.

Accordingly, an inflammation of skin associated with alopecia can be improved or prevented. Such effect was useful in a case where alopecia has been exacerbated due to skin condition at the applied site such as scalp. Furthermore, when it was used as an external preparation, it exhibits an effect of moisturizing and improving skin texture at the applied site, as mentioned above. Namely, dandruff or itch can be eliminated and their occurence can be suppressed, while hair and scalp can be moisturized, desiccation can be improved or prevented, seborrheic condition can be improved, too, and scalp and hair can be kept healty.

### 8.3 Confirming the effect on nose

The obtained cyclic peptide was combined with purified water at 1, 5, 30, 100, 500 *µ*g/ml and applied to the affected site. Formulations A to E in the tables refers to the cyclic peptide of 1, 5, 30, 100, 500 *µ*g/ml, respectively. No effect was observed without the peptide or with treatment with purified water, comfirming that it was not placebo effect.

**[Table 31-1]**

| SEQ ID No. (amino acid sequence) of the cyclic peptide used | No. | Sex | Age | Diagnostic impression | Formulatio n | Treatment method | Post-treatment diagnostic skin impression |
|---|---|---|---|---|---|---|---|
| SEQ ID NO: 58: (CFALKMDRIGTMSGIGC) | T1 | F | 40's | allergic rhinitis | C | once/day | Immediately after nasal application, nasal congestion was remarkably improved, rhinorrhea stopped. |
| SEQ ID NO: 9: (CFGLKLDRVGSMSGLGC) | T2 | F | 40's | allergic rhinitis | D | once/day | Immediately after nasal application, rhinorrhea was remarkably improved. |
| SEQ ID NO: 9: (CFGLKLDRVGSMSGLGC) | T3 | F | 30's | allergic rhinitis | C | once/day | Immediately after nasal application, nasal respiration became easy, nose was clear, rhinorrhea stopped. There was no irritation upon nasal application. |
| SEQ ID NO: 32: (CFALKMDRIGSMSGLGC) | T4 | F | 30's | allergic rhinitis | C | once/day | After 1 min, nose was clear, rhinorrhea stopped. There was no irritation upon nasal application. |
| SEQ ID NO: 50: (CFALKMDRIGSMSGIGC) | T5 | F | 20's | perennial rhinitis with rhinorrhea | C | once/day | Immediately after nasal application, nose was clear through the interior of nasal cavity, and rhinorrhea stopped, There was no irritation upon nasal application. |
| SEQ ID NO: 34: (CFALKLDRIGSMTGLGC) | T6 | F | 20's | perennial rhinitis with rhinorrhea | B | once/day | After 2 min, nose was clear, rhinorrhea stopped. There was no irritation upon nasal application. |
| SEQ ID NO: 16: (CFGLKLDRIGSMAGLGC) | T7 | F | 20's | perennial rhinitis with congestion | C | once/day | After 2 min, nasal congestion was improved, and nose was clear. There was no irritation upon nasal application. |

**[Table 31-2]**

| | | | | | | | |
|---|---|---|---|---|---|---|---|
| SEQ ID NO: 52: (CFGLKMDRIGTMSGIGC) | T8 | M | 30's | allergic rhinitis with congestion | C | once/day | After 1 min, nasal congestion was improved, and nose was clear. There was no irritation upon nasal application. |
| SEQ ID NO: 57: (CF ALKMDRIGTMTGLGC) | T9 | F | 40's | allergic rhinitis with rhinorrhea | C | once/day | Immediately after nasal application, nose was refreshed and clear, itches were remitted. There was no irritation upon nasal application. |
| SEQ ID NO: 21: (CFGLKLDRIGSMSGFGC) | T10 | F | 50's | allergic rhinitis with rhinorrhea | C | once/day | After 1 min, nose was clear, no nasal drip discharged upon blowing. There was no irritation upon nasal application. |
| SEQ ID NO: 39: (CFGLKLDRIGTMTGLGC) | T11 | F | 50's | allergic rhinitis with rhinorrhea | C | once/day | After 1 min, nose was clear, no nasal drip discharged upon blowing. There was no irritation upon nasal application. |
| SEQ ID NO: 59: (CFALKMDRIGSMTGIGC) | T12 | F | 40's | allergic rhinitis with rhinorrhea | D | once/day | After 1 min, rhinorrhea stopped, no nasal drip discharged upon blowing. There was no irritation upon nasal application. |
| SEQ ID NO: 10: (CFGLKLDRIGTMSGLGC) | T13 | F | 40's | allergic rhinitis with rhinorrhea | C | once/day | After 3 min, rhinorrhea stopped. There was no irritation upon nasal application. |

**[Table 31-3]**

| | | | | | | | |
|---|---|---|---|---|---|---|---|
| SEQ ID NO: 33: (CFALKLDRIGTMSGLGC) | T14 | M | 40's | allergic rhinitis with rhinorrhea and congestion | C | once/day | After 2 min, nose was clear, rhinorrhea stopped. There was no irritation upon nasal application. |
| SEQ ID NO: 58: (CFALKMDRIGTMSGtGC) | T15 | M | 40's | allergic rhinitis with rhinorrhea and congestion | B | once/day | After 2 min, nose was clear, rhinorrhea stopped. There was no irritation upon nasal application. |
| SEQ ID NO: 20: (CFGLKLDRIGSMSGMGC) | T16 | M | 30's | allergic rhinitis with rhinorrhea | C | once/day | After 1 min, nose was clear, rhinorrhea stopped. There was no irritation upon nasal application. |
| SEQ ID NO: 40: (CFGLKLDRIGTMSGIGC) | T17 | M | 30's | allergic rhinitis with rhinorrhea | C | once/day | After 1 min, nose was clear, rhinorrhea stopped. There was no irritation upon nasal application. |
| SEQ ID NO: 19: (CFGLKLDRIGSMSGIGC) | T18 | M | 20's | allergic rhinitis with rhinorrhea | C | once/day | Immediately after nasal application, rhinorrhea stopped. There was no irritation upon nasal application. |
| SEQ ID NO: 48: (CFALKMDRIGTMSGLGC) | T19 | F | 50's | perennial rhinitis with rhinorrhea | C | once/day | Immediately after nasal application, nose was clear. After 3 min, rhinorrhea stopped. There was no irritation upon nasal application. Less irritation on nasal mucosa than commercially available steroid nasal drop drugs, causing no desiccation. |
| SEQ ID NO: 11: (CFGLKLDRIGAMSGLGC) | T20 | F | 50's | perennial rhinitis with rhinorrhea | C | once/day | After 1 min, rhinorrhea stopped. |
| | | | | | | | After 3 min, no nasal drip discharged upon blowing. There was no irritation upon nasal application. |

**[Table 31-4]**

| | | | | | | | |
|---|---|---|---|---|---|---|---|
| SEQ ID NO: 18: (CFGLKLDRIGSMSGVGC) | T21 | M | 20's | allergic rhinitis with rhinorrhea | C | once/day | Immediately after nasal application, rhinorrhea stopped. There was no irritation upon nasal application, |
| SEQ ID NO: 17: (CFGLKLDRIGSMSGAGC) | T22 | M | 30's | allergic rhinitis with rhinorrhea | C | once/day | After 1 min, nose became clear, and rhinorrhea stopped. |
| SEQ ID NO:38: (CFGLKMDRIGSMSGIGC) | T23 | M | 30's | allergic rhinitis with rhinorrhea | B | once/day | After 1 min, nose was clear, and rhinorrhea stopped. |
| SEQ ID NO: 15: (CFGLKLDRIGSMTGLGC) | T24 | M | 40's | allergic rhinitis with rhinorrhea and congestion | C | once/day | After 1 min, nose was clear, and after 2 min, rhinorrhea stopped. |
| SEQ ID NO: 51: (CFGLKMDRIGTMTGLGC) | T25 | M | 40's | allergic rhinitis with rhinorrhea and congestion | C | once/day | After 1 min, nose was clear. |
| | | | | | | | After 2 min, rhinorrhea stopped. |

**[Table 31-5]**

| | | | | | | | |
|---|---|---|---|---|---|---|---|
| SEQ ID NO: 12: (CFGLKLDRIGVMSGLGC) | T26 | M | 40's | allergic rhinitis with rhinorrhea | B | once/day | After 3 min, rhinorrhea stopped. |
| SEQ ID NO: 53: (CFGLKMDRIGSMTGIGC) | T27 | M | 40's | allergic rhinitis with rhinorrhea | C | once/day | After 1 min, rhinorrhea stopped. |
| | | | | | | | After 3 min, nose became clear, no nasal drip discharged upon blowing. There was no irritation upon nasal application. |
| SEQ ID NO: 36: (CFGLKMDRIGTMSGLGC) | T28 | F | 20's | perennial rhinitis with rhinorrhea | C | once/day | Immediately after nasal application, rhinorrhea stopped, nose was clear through the interior of nasal cavity. The effect lasted for 1 week. |
| SEQ ID NO: 6: (CFALKLDRIGSMSGLGC) | T29 | F | 20's | perennial rhinitis with rhinorrhea | B | once/day | After 2 min, rhinorrhea stopped, and nose was clear. |
| SEQ ID NO: 35: (CFALKLDRIGSMSGIGC) | T30 | F | 20's | perennial rhinitis with congestion | C | once/day | After 2 min, and nose was clear. |
| SEQ ID NO: 49: (CFALKMDRIGSMTGLGC) | T31 | F | 20's | perennial rhinitis with rhinorrhea | C | once/day | Immediately after nasal application, nose was clear. Tickling itches in nasal mucosa were also eliminated. |
| SEQ ID NO: 41: (CFGLKLDRIGSMTGIGC) | T32 | F | 20's | allergic rhinitis with congestion | C | once/day | After 1 min, nose was clear. There was no irritation upon nasal application. |

**[Table 31-6]**

| | | | | | | | |
|---|---|---|---|---|---|---|---|
| SEQ ID NO: 14: (CFGLKLDRIGLMSGLGC) | T33 | F | 50's | allergic rhinitis with rhinorrhea | C | once/day | After 1 min, nose became clear, no nasal drip discharged upon blowing. |
| SEQ ID NO: 37: (CFGLKMDRIGSMTGLGC) | T34 | F | 50's | allergic rhinitis with rhinorrhea | C | once/day | After 1 min, nose became clear, no nasal drip discharged upon blowing. |
| SEQ ID NO: 7: (CFGLKADRIGSMSGLGC) | T35 | M | 30's | perennial rhinitis with congestion | A | once/day | After 2 min, and nose was clear. There was no irritation upon nasal application. Less irritation and better efficacy upon nasal application than commercially available steroid nasal drop drugs. |
| SEQ ID NO: 13: (CFGLKLDRIGIMSGLGC) | T36 | M | 30's | perennial rhinitis with congestion | A | once/day | After 1 min, and nose was clear. There was no irritation upon nasal application. Less irritation and better efficacy upon nasal application than commercially available steroid nasal drop drugs. |
| SEQ ID NO: 50: (CFALKMDRIGSMSGIGC) | T37 | M | 60's | perennial rhinitis with rhinorrhea | A | once/day | Immediately after nasal application, and nose was clear. After 3 min, no nasal drip discharged upon blowing. The subject realized this peptide had better efficacy than commercially available steroid nasal drop drugs which were too irritative causing nasal discharge. |
| SEQ ID NO: 52: (CFGLKMDRIGTMSGIGC) | T38 | M | 60's | perennial rhinitis with rhinorrhea | A | once/day | Immediately after nasal application, and nose was clear. |
| | | | | | | | After 2 min, no nasal discharge upon blowing. The subject realized this peptide had better efficacy than commercially available steroid nasal drop drugs which were too irritative causing nasal discharge. |
| SEQ ID NO: 8: (CFGLKMDRIGSMSGLGC) | T39 | F | 40's | perennial rhinitis with congestion | A | once/day | Immediately after nasal application, nose was clear and felt ease. |

**[Table 31-7]**

| | | | | | | | |
|---|---|---|---|---|---|---|---|
| SEQ ID NO: 34: (CFALKLDRIGSMTGLGC) | T40 | F | 40's | perennial rhinitis with congestion | A | once/day | After 3 min, nose became clear. |
| SEQ ID NO: 3: (CFGLKLDRIGSMSGLGC) | T41 | F | 50's | perennial rhinitis with rhinorrhea | A | nasal application for comparison of right and left (Right) once/day | (Right) |
| | | | | | | | After 3 min, nose was not clear with remaining nasal drip, and nasal drip were discharged upon blowing. |
| | | | | | | | Nasal drip was accumulated as time went on. After 8 min, nose was not clear. |
| SEQ ID NO: 58: (CFALKMDRIGTMSGIGC) | | | | | | nasal application for comparison of right and left (Left) once/day | (Left) |
| | | | | | | | Immediately after nasal application, itches were eliminated, nose was refreshed and clear. |
| | | | | | | | After 3 min, no nasal drip discharged upon blowing. |
| | | | | | | | After 8 min, the effects described above lasted, and no nasal drip accumulated |
| SEQ ID NO: 3: (CFGLKLDRIGSMSGLGC) | T42 | F | 20's | allergic rhinitis with rhinorrhea | A | nasal application for comparison of right and left (Right) once/day | (Right) |
| | | | | | | | After 3 min, nasal discharge did not stop. |
| SEQ ID NO: 50: (CFALKMDRIGSMSGIGC) | | | | | | nasal application for comparison of right and left (Left) once/day | (Left of comparison) |
| | | | | | | | After 1 min, nose was clear. |
| | | | | | | | After 2 min, no nasal drip discharged upon blowing. |
| | | | | | | | After 3 min, the effects described above continued. |

**[Table 31-8]**

| | | | | | | | |
|---|---|---|---|---|---|---|---|
| SEQ ID NO: 3: (CFGLKLDRIGSMSGLGC) | T43 | F | 50's | perennial rhinitis with congestion | A | nasal application for comparison of right and left (Right) once/day | (Right) |
| | | | | | | | After 2 min, nose was not clear. |
| SEQ ID NO: 58: (CFALKMDRIGTMSGIGC) | | | | | | nasal application for comparison of right and left (Left) once/day | (Left) |
| | | | | | | | After 2 min, and nose was clear. |
| SEQ ID NO: 3: (CFGLKLDRIGSMSGLGC) | T44 | M | 30's | allergic rhinitis with rhinorrhea and congestion | A | nasal application for comparison of right and left (Right) once/day | (Right) |
| | | | | | | | Immediately after nasal application, nasal congestion and nasal discharge was remitted, though nose was not as clear as with SEQ ID NO: 58. |
| SEQ ID NO: 58: (CFALKMDRIGTMSGIGC) | | | | | | nasal application for comparison of right and left (Left) once/day | (Left) |
| | | | | | | | Immediately after nasal application, nose was clear and refreshed, It was fresher with SEQ ID NO: 58. |

By the result above, it was demonstrated that even if part of the amino acid in the cyclic peptide was substituted, the cyclic peptide of the present invention exhibits an effect that is equal to or more than that of the cyclic peptide expressed by the formula II (SEQ ID NO: 3). Similar effect was obtained in a case where substituted cyclic peptide was mixed and applied if the effective amount of the cyclic peptide was applied.

One 0.1m! nasal application of 1 *µ*g/ml to 500 *µ*g/ml to each nasal cavity, the affected site, immediately stopped running nose and improved nasal congestion and stopped itches in nasal mucosa or sneezing (immediate effect), and by only one nasal application the effect lasted for all day, or 1 week of more depending on the case (persistence). When being compared to CNP administration, it has not only a high therapeutic effect, but also an immediate effect, as well as an effect that act at a very low concentration.

In specific, in two-sided application method carried out in Examination Examples T41 to T44 (examination where, for one identical subject, CNP cyclic peptide was applied in one side, and the cyclic peptide having 4 amino acid substitutions (such as, e.g., SEQ ID NO: 58) was applied to the other side), the cyclic peptide such as SEQ ID NO: 58 had effects of suppressing erythema, scales, papule, edema and itches, etc., in atopic dermatitis, psoriasis, eczema and contact dermatitis, within a few minutes after starting its use, even at a concentration as low as 1 *µ*g/m.

The effect obtained by applying 1 *µ*M of the CNP cyclic peptide is equal to or more than the effect obtained by 10 *µ*M to 50 *µ*M of CNP. It was confirmed that it had not only a high therapeutic effect but also an immediate affect as compared to CNP or unsubstituted CNP cyclic peptide, as well as an effect that acts at a very low concentration. Furthermore, the newly developed CNP cyclic peptide in which part of the amino acids has been substituted had an effects at even lower concentration at which an unsubstituted CNP cyclic peptide cannot provide the effect. These effects act immediately and are capable of remarkabe improvement of rhinorrhea and nasal congestion and of stopping itches in mucosal and sneezing, too.

The present invention causes no local irritation and can be used safely in a subject who did not wish to use a nasal drop because of its strong local irritation caused by a therapeutic for rhinitis which have conventionally been used such as conventional steroid drugs.

## Claims

1. A cyclic peptide having an amino acid sequence expressed by the formula I: 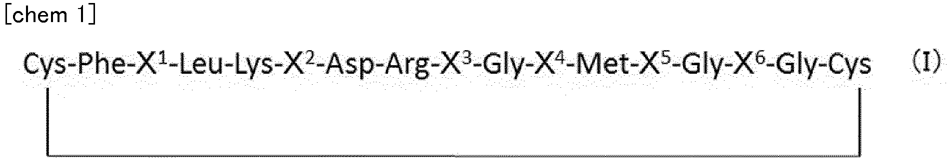 wherein,
X¹ denotes Gly or Ala,
X² denotes Leu, Ala or Met,
X³ denotes Ile or Val,
X⁴ denotes Ser, Thr, Ala, Val, Ile or Leu,
X⁵ denotes Ser, Thr or Ala,
X⁶ denotes Leu, Ala, Val, Ile, Met or Phe,
the line that links two Cys represents a disulfide bond,
and wherein the amino acid sequence has no peptide bond other than those which are formed between the amino acids constituting the amino acid sequence,
or a derivative thereof or a pharmaceutically acceptable salt thereof.

2. The cyclic peptide according to Claim 1 or a derivative thereof or a pharmaceutically acceptable salt thereof selected from SEQ ID NOs: 60 to 62.

3. The cyclic peptide according to Claim 1 or a derivative thereof or a pharmaceutically acceptable salt thereof selected from SEQ ID NOs: 22 to 31, 42 to 47 and 54 to 56.

4. The cyclic peptide according to Claim 1 or a derivative thereof or a pharmaceutically acceptable salt thereof selected from SEQ ID NOs: 6 to 21, 32 to 41, 48 to 53 and 57 to 59.

5. The cyclic peptide or a derivative thereof or a pharmaceutically acceptable salt thereof according to any one of Claims 1 to 4, wherein the derivative has been substituted with a substituent that is replaceable for the hydrogen atom, hydroxyl group, carboxyl group, amino group, imino group in the cyclic peptide.

6. A cyclic peptide or a derivative thereof or a pharmaceutically acceptable salt thereof that is formed from the cyclic peptide according to Claim 4, wherein 1 to 6 amino acid(s) in the cyclic peptide is deleted, substituted with amino acid(s), or added, and that has equal functions to the cyclic peptide expressed by each of said fomulae.

7. An external preparation comprising one or more cyclic peptides according to any one of Claims 1 to 6 and/or a derivative thereof and/or a pharmaceutically acceptable salt thereof.

8. The external preparation according to Claim 7, which is an ingredient for a therapeutic for dermatitis, a prophylactic for dermatitis, an antipruritic, an antiphlogistic, an epidermal regeneration accelerating agent or a skin-care product.

9. The external preparation according to Claim 8, wherein a skin-care product is for moisturization, and/or for preventing/improving skin roughness, and/or for preventing/improving skin sensitivity, and/or for preventing/improving dry skin, and/or for improving sebum condition/for acne care, and/or for reliefing irritation/for anti-inflammation, and/or for skin lightening, and/or for anti-aging, and/or for preventing/improving wrinkle/flabbiness and/or dullness/dark circles, and/or for preventing/improving photosensitivity, and/or for preventing/reliefing UV damages, and/or for slimming, and/or for skin-cleansing.

10. The external preparation according to Claim 8 being a bathing agent, body cleansing agent, hair cleansing agent, hair rinse/treatment, hair tonic, hair oil, hair loation, scalp care agent.

11. The external preparation according to Claim 7 being a therapeutic for alopecia, a prophylactic for alopecia, a hair growing agent and/or a hair growth stimulant and/or a prophylactic/inhibitor for hair-falling, a prophylactic cosmetic for hair-falling.

12. The external preparation according to Claim 7 being a scalp care agent for preventing/improving dandruff or itch, and/or for improving/preventing hair/scalp dryness, and/or for improving/preventing seborrheic condition of scalp.

13. The external preparation according to Claim 7 being a therapeutic for rhinitis and/or a prophylactic for rhinitis and/or a therapeutic for rhinosinusitis and/or a prophylactic for rhinosinusitis.

14. The external preparation according to Claim 7 being a cosmetic.

15. The external preparation according to any one or Claims 7 to 14, wherein the dosage form is a solid, semisolid, powder, liquid, spray, ointment, cream, emulsion, gel or patch.

16. The external preparation according to any one of Claims 7 to 15 comprising the cyclic peptide according to any one of Claims 1 to 5 and/or a derivative thereof and/or a pharmaceutically acceptable salt thereof at a concentration from 0.0001 to 1000000 *µ*g/mL.

17. The external preparation according to any one of Claims 7 to 16 for use as a quasi-drug and cosmetic product.

18. Use of cyclic peptide according to any one of Claims 1 to 5 and/or a derivative thereof and/or a pharmaceutically acceptable salt thereof for the manufacture of a topical agent.

19. A method of using the external preparation comprising applying the external preparation according to any one of Claims 7 to 17 to skin and/or mucosa of a subject.

20. The method of using the external preparation according to Claim 18, wherein the mucosa is a lip, oral cavity, nasal cavity, eye or vagina.

21. The method of using the external preparation according to Claim 18, which is a method of treating and/or preventing dermatitis, a method of alleviating and/or eliminating dermatitis, a method of treating erosion/ulcer or a skin-care method.

22. The method of using the external preparation according to Claim 18, which is a method of treating and/or preventing dermatitis selected from a group consisting of eczema, atopic dermatitis, contact dermatitis, photosensitivity, psoriasis, acne, cystic acne, seborrheic eczema and itches.

23. The method of using the external preparation according to Claim 18, which is a method of improving and preventing skin roughness, hypersensitivity of skin, dry skin, oily skin, photosensitivity, wrinkles, flabbiness, dullness, dandruffs and itches.

24. The method of using the external preparation according to Claim 18, which is a method of treating and/or preventing thinning hair/alopecia, and/or a method of stimulating hair growth and/or a method of growing hair, a method of inhibiting and/or preventing hair falling.

25. The method of using the external preparation according to Claim 18, which is a method of inhibiting and/or preventing dandruffs or itches, and/or a method of improving/preventing hair/scalp dryness, and/or a method of preventing/improving seborrheic condition of scapl.

26. The method of using the external preparation according to Claim 18, which is a method of treating/preventing rhinitis/rhinosinusitis.

27. A medicament comprising one or more cyclic peptides according to any one of Claims 1 to 5 or a derivative thereof or a pharmaceutically acceptable salt thereof.

28. The medicament according to Claim 27, which is a therapeutic for hypertension, unstable angina, acute myocardial infarction, edematous diseases, renal failure, heart failure, immune diseases, autoimmune diseases, allergic diseases, cancer, gastrointestinal diseases, Crohn's disease, ulcerative colitis, obesity, metabolic syndrome.
